(19) **Europäisches Patentamt**
**European Patent Office**
**Office européen des brevets**

(11) **EP 2 786 152 B1**

(12) **EUROPEAN PATENT SPECIFICATION**

(45) Date of publication and mention
of the grant of the patent:
**02.01.2019 Bulletin 2019/01**

(21) Application number: **12799110.7**

(22) Date of filing: **29.11.2012**

(51) Int Cl.:
***G01N 33/574*** *(2006.01)*

(86) International application number:
**PCT/EP2012/073965**

(87) International publication number:
**WO 2013/079594 (06.06.2013 Gazette 2013/23)**

(54) **DEVICE AND METHODS TO DIAGNOSE PANCREATIC CANCER**

GERÄT UND METHODEN ZUR DIAGNOSE VON BAUCHSPEICHELDRÜSENKREBS

DISPOSITIF ET METHODES POUR LE DIAGNOSTIC DU CANCER DU PANCRÉAS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2011 EP 11191365**
**30.11.2011 US 201161564891 P**

(43) Date of publication of application:
**08.10.2014 Bulletin 2014/41**

(73) Proprietor: **metanomics Health GmbH**
**10589 Berlin (DE)**

(72) Inventors:
• **RESZKA, regina**
**16341 Panketal (DE)**
• **KAMLAGE, Beate**
**12161 Berlin (DE)**

• **KALTHOFF, Holger**
**24106 Kiel (DE)**
• **SCHNIEWIND, Bodo**
**24105 Kiel (DE)**
• **MAYERLE, Julia**
**17489 Greifswald (DE)**
• **LERCH, Markus M.**
**17489 Greifswald (DE)**
• **PILARSKY, Christian**
**01309 Dresden (DE)**
• **GRÜTZMANN, Robert**
**01309 Dresden (DE)**

(74) Representative: **Herzog, Fiesser & Partner**
**Patentanwälte PartG mbB**
**Dudenstrasse 46**
**68167 Mannheim (DE)**

(56) References cited:
**WO-A1-2011/038509      WO-A2-2011/151252**

EP 2 786 152 B1

**Description**

Pancreatic cancer has the worst prognosis of all solid tumors with 5-year survival rates of less than 5% but an increasing incidence (Everhart 2009, Gastroenterology 136:1134-11449). There is a widely acknowledged demand for the establishment of innovative tools and technologies for point-of-care utilization of specific biomarkers and novel molecular imaging tools for early diagnosis, prognostic stratification and differential diagnosis of pancreatic cancer. Advances in these areas are pivotal to improve the prognosis of this malignancy, since timely surgical resection of early stage tumors is currently the only effective means of treatment of this dismal disease.

The mortality of this cancer type is the highest of any cancer type in Europe and the western world. People die soon after diagnosis due to the lack of means for early detection. Early symptoms are rare and uncharacteristic. Thus, PDACs are commonly diagnosed in an advanced stage of the disease. To date, the best imaging technologies to detect PDAC are endoscopic ultrasound (EUS), spiral computer tomography (CT), magnetic resonance cholangiopancreatography (MRCP) or endoscopic retrograde cholangiopancreatography (ERCP) (Dewitt 2006, Gastroenterol Hepatol. (4):717-25). Unfortunately, the resolution of these technologies for detecting neoplastic lesions within the pancreas is in the range of 3-10 mm. Thus, they are not able to detect pancreatic neoplasia at a curable stage. The serum concentration of conventional tumor markers such as CA19-9 is increased in a subset of pancreatic cancer patients (Fry 2008, Langenbecks Arch Surg. (393): 883-90). However, so far all available markers lack sensitivity and tumor specificity. Thus, new approaches are urgently needed to increase the diagnostic sensitivity towards the detection of very small, early stage PDAC and its precursor lesions (PanINs and IPMNs) as well as prognostic subgroups of advanced tumors.

The association between chronic inflammation and the development of malignancies has been recognized for many years. For pancreatic cancer this association was only recently confirmed and a consensus conference agreed upon a new classification for pancreatic intraepithelial neoplasia as noninvasive precursor lesions (Hruban 2004, Am J Surg Path (28): 977-987). Chronic pancreatitis is defined as recurrent bouts of a sterile inflammatory disease characterized by often progressive and irreversible morphological changes, typically causing pain and permanent impairment of pancreatic function. With an incidence of 8.2, a prevalence of 27.4 per 100 000 population and a 0.04% to 5% frequency in unselected autopsy specimens chronic pancreatitis represents a frequent disorder of the gastrointestinal tract. Various etiologies are responsible for the development of chronic pancreatitis. An increased risk of patients suffering from of chronic pancreatitis to die from pancreatic cancer was shown in an international cooperative investigation conducted by AB Lowenfels and coworkers as a multicenter historical cohort study of 2015 patients with chronic pancreatitis recruited from clinical centers in 6 countries in 1993. This study found a cumulative risk of pancreatic cancer in patients with chronic pancreatitis of 1.8% after 10 years and of 4% after 20 years with a standardized incidence ratio of 14.4. For patients with a minimum of two years follow up the risk of pancreatic cancer was 16.5 fold higher than that of the general population (Lowenfels 1993, N Engl J Med (328): 1433-1437). The search for an association between chronic pancreatitis and pancreatic cancer intensified when in 1996 a single point mutation in the third exon of the cationic trypsinogen gene on chromosome 7 (7q35) was found to be associated with hereditary pancreatitis and multiple kindreds were subsequently identified and reported. Only very recently the EUROPAC study group presented their work on clinical and genetic characteristics in hereditary pancreatitis. In a multilevel proportional hazard model employing data obtained from the European Registry of Hereditary Pancreatitis this group presented 112 families in 14 countries (418 affected individuals) (Howes 2004, Clinical Gastroenterology and Hepatology (2): 252-261). The cumulative risk (95% CI) of pancreatic cancer was 44.0% (8.0% - 80.0%) at 70 years from symptom onset with a standardized incidence ratio of 67% (50% - 82%). A previous study had also shown an estimated lifetime risk of pancreatic cancer of 40% (Lowenfels 2001, JAMA 286: 169-170, Lowenfels 1997, J Natl Cancer Inst 89: 442-44656).

In pancreatic cancer imaging studies fail to detect early pancreatic malignancies in a curable stage, however in the background of chronic pancreatitis imaging studies such as EUS, CT or MRI drop sensitivity and specificity to a degree where tossing a coin is equally reliable. Thus, the detection of pancreatic malignancy in a high risk cohort would be higly desired.

There are a few reports of metabolic changes in patients suffering pancreas-associated diseases. Schrader et al (Schrader 2009, Panceas 38: 416-421) suggests that patients with pancreatic cancer and chronic pancreatitis show significant changes in serum aminoacid levels. It has been suggested that among the ceramides sphingomyelin on the cell surface of cancer cells takes actively part in cell signalling. Ceramides are known to induce apoptosis in cancer cells. Low levels of sphingomyelin suggest less responsiveness to gemcitabine treatment (Modrak 2009, Mol Cancer Res 7:890-896).

In conclusion with a 5-year survival rate of 0.5-5%, pancreatic cancer carries the most dismal prognosis of all human tumors and represents the 4th leading cause in cancer-related deaths worldwide. It is thus a disease with a major socioeconomic impact. Accurate diagnosis and timely surgical resection of early tumors currently offer the only realistic prospect for the improvement of patient prognosis.

WO 2011/038509 discloses a method for diagnosing pancreatic cancer in a subject based on the amount of lysophosphatidylcholine.

The technical problem underlying the present invention can be seen as the provision of means and methods for complying with the aforementioned needs. The technical problem is solved by the embodiments characterized in the claims and herein below.

Thus, the present invention relates to a method for diagnosing pancreas cancer in a subject comprising the steps of:

(a) determining in a plasma, blood or serum sample of a subject suspected to suffer from pancreas cancer at least the amount of the biomarker Ceramide (d18:1,C24:0); and
(b) comparing the said amount of the at least one biomarker with a reference, whereby pancreas cancer is to be diagnosed.

The method as referred to in accordance with the present invention includes a method which essentially consists of the aforementioned steps or a method which includes further steps. However, it is to be understood that the method, in a preferred embodiment, is a method carried out ex vivo, i.e. not practised on the human or animal body. The method, preferably, can be assisted by automation.

The term "diagnosing" as used herein refers to assessing whether a subject suffers from the pancreatic cancer, or not. As will be understood by those skilled in the art, such an assessment, although preferred to be, may usually not be correct for 100% of the investigated subjects. The term, however, requires that a statistically significant portion of subjects can be correctly assessed and, thus, diagnosed. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.2, 0.1, or 0.05.

The term includes individual diagnosis of pancreatic cancer or its symptoms as well as continuous monitoring of a patient. Monitoring, i.e. diagnosing the presence or absence of pancreatic cancer or the symptoms accompanying it at various time points, includes monitoring of patients known to suffer from pancreatic cancer as well as monitoring of subjects known to be at risk of developing pancreatic cancer. Furthermore, monitoring can also be used to determine whether a patient is treated successfully or whether at least symptoms of pancreatic cancer can be ameliorated over time by a certain therapy.

The term "pancreatic cancer" or "pancreas cancer" as used herein relates to cancer which is derived from pancreatic cells. Preferably, pancreatic cancer as used herein is pancreatic adenocarcinoma. The symptoms accompanying pancreatic cancer are well known from standard text books of medicine such as Stedmen or Pschyrembl. Moreover, the terms "cancer" and "carcinoma" may, in general, also be used herein interchangeably.

The term "biomarker" as used herein refers to a molecular species which serves as an indicator for a disease or effect as referred to in this specification. Said molecular species can be a metabolite itself which is found in a sample of a subject. Moreover, the biomarker may also be a molecular species which is derived from said metabolite. In such a case, the actual metabolite will be chemically modified in the sample or during the determination process and, as a result of said modification, a chemically different molecular species, i.e. the analyte, will be the determined molecular species. It is to be understood that in such a case, the analyte represents the actual metabolite and has the same potential as an indicator for the respective medical condition. In accordance with the method or use of the present invention, the biomarker is Ceramide (d18:1,C24:0).

Moreover, a biomarker according to the present invention is not necessarily corresponding to one molecular species. Rather, the biomarker may comprise stereoisomers or enantiomeres of a compound. Further, a biomarker can also represent the sum of isomers of a biological class of isomeric molecules. Said isomers shall exhibit identical analytical characteristics in some cases and are, therefore, not distinguishable by various analytical methods including those applied in the accompanying Examples described below. However, the isomers will share at least identical sum formula parameters and, thus, in the case of, e.g., lipids an identical chain length and identical numbers of double bonds in the fatty acid and/or sphingo base moieties

Polar biomarkers can be, preferably, obtained by techniques referred to in this specification elsewhere and as described in Example 1, below. Lipid biomarkers can be obtained, preferably, as described in this specification elsewhere and, in particular, either as lipid fraction by separation of a sample after protein precipitation into an aqueous polar and an organic lipid phase by, e.g., a mixture of ethanol and dichloromethane as described in Example 1, below. Those biomarkers may be marked by "lipid fraction" herein. Alternatively or in addition, complex lipids can be obtained by liquid/liquid extraction using chloroform/methanol followed by fractionation using normal phase liquid chromatography (NPLC) as described in Example 1, below. Those biomarkers may be marked or marked in addition by the specific sub-fraction, e.g., "ceramide fraction" or "CER" or "sphingomyelins" or "SM" etc.. Further details are found below in the Tables.

In the method according to the present invention, the amount of the biomarker Ceramide (d18:1,C24:0) is to be determined. However, more preferably, a group of biomarkers will be determined in order to strengthen specificity and/or sensitivity of the assessment. Such a group, preferably, comprises at least 2, at least 3, at least 4, at least 5, at least 10 or up to all of the said biomarkers shown in the Tables 2a, 2b, 3a, 3b, 4a, or 4b.

Preferably, the at least one biomarker determined in the disclosed method is a biomarker of category 1 as shown in Table 2a or 2b. More preferably, the at least one biomarker is selected from the group consisting of the spingomyelins of the sphingomyeline fraction (SM) and the cermides of the ceramide fraction (CER) as shown in table 2b, i.e. SM_Sphingomyelin (d18:1,C19:0), SM_Sphingomyelin (d18:1,C21:0), SM_Sphingomyelin (d18:2,C22:0), SM_Sphingomyelin (d17:1,C24:1), SM_Sphingomyelin (d18:2,C20:0), SM_Sphingomyelin (d18:2,C19:0), SM_Sphingomyelin (d18:2,C21:0), CER_Ceramide (d17:1,C22:0), and CER_Ceramide (d18:2,C22:0). Preferably, the aforementioned sphingomyeline fraction (SM) and/or ceramide fraction (CER) is obtained by a method for fractionation as set forth below or in the accompanying Examples, below.

According to the disclosure, the aforementioned group of biomarkers is determined as the at least one biomarker in the disclosed method. More preferably, said at least one biomarker of category 1 is capable of diagnosing pancreatic carcinoma and accompanying liver cirrhosis or pancreatitis in a subject when compared to a reference derived from a healthy control subject or group of such subjects. More preferably, said diagnosis is achieved with significance levels set forth in Table 1, below. The category 1 biomarkers are capable of, preferably, diagnosing pancreatic cancer in a high risk group of subjects exhibiting the accompanying disease pancreatitis and/or liver cirrhosis.

Preferably, the at least one biomarker determined in the disclosed method is a biomarker of category 2 as shown in Table 3a or 3b. More preferably, the at least one biomarker is selected from the group consisting of: 1-Hydroxy-2-amino-(cis,trans)-3,5-octadecadiene (from sphingolipids), Sphingomyelin (d18:2,C18:0), Glycerol phosphate, lipid fraction, Arachidonic acid (C20:cis[5,8,11,14]4), Phosphate, lipid fraction, myo-Inositol-2-phosphate, lipid fraction (myo-Inositolphospholipids), Cholesta-2,4,6-triene, erythro-C16-Sphingosine, SM_Sphingomyelin (d18:2,C22:1), SM_Sphingomyelin (d18:2,C20:1), SM_Sphingomyelin (d18:2,C24:1), SM_Sphingomyelin (d18:1,C20:0), SM_Sphingomyelin (d18:1,C19:0), SM_Sphingomyelin (d18:2,C23:1), CER_Ceramide (d18:2,C18:0), SM_Sphingomyelin (d18:2,C18:1), SM_Sphingomyelin (d18:1,C22:0), SM_Sphingomyelin (d18:2,C16:0), SM_Sphingomyelin (d18:1,C21:0), CER_Ceramide (d18:2,C20:0), CER_Ceramide (d18:1,C22:0), CER_Ceramide (d16:1,C18:0), SM_Sphingomyelin (d16:1,C22:1), SM_Sphingomyelin (d16:1,C20:0), SM_Sphingomyelin (d16:1,C18:1), CER_Ceramide (d16:1,C20:0), SM_Sphingomyelin (d17:1,C24:1), CER_Ceramide (d18:2,C24:1), CER_Ceramide (d17:1,C24:1), Sphingosine-1-phosphate (d18:1), SM_Sphingomyelin (d16:1,C24:1) and CER_Ceramide (d16:1,C24:1).

According to the disclosure, the aforementioned group of biomarkers is determined as the at least one biomarker in the disclosed method. More preferably, said at least one biomarker of category 2 is capable of diagnosing pancreatic carcinoma and accompanying pancreatitis in a subject when compared to a reference derived from a healthy control subject or group of such subjects. More preferably, said diagnosis is achieved with significance levels set forth in Table 1, below. The category 2 biomarkers are capable of, preferably, diagnosing pancreatic cancer in a risk group of subjects exhibiting the acccompanying diesease pancreatitis.

The at least one biomarker to be determined in the method of the present invention is CER_Ceramide (d18:1,C24:0). According to the disclosure, the at least one biomarker is selected from the group consisting of: Sphingomyelin (d18:2,C18:0), O-Acetylcarnitine, 1-Hydroxy-2-amino-(cis,trans)-3,5-octadecadiene (from sphingolipids), erythro-Sphingosine, Behenic acid (C22:0), Eicosanoic acid (C20:0), 3-O-Methylsphingosine (d18:1), 5-O-Methylsphingosine (d18:1), 3-Hydroxyisobutyrate, threo-Sphingosine, Nervonic acid (C24:cis[15]1), 7-Methylguanosine, erythro-C16-Sphingosine, Tetradecanoylcarnitine, Sarcosine, Phytosphingosine (t18:0), total, Sphingomyelin (d18:1,C23:0), 5-O-Methylsphingosine (d16:1), Sphingomyelin (d18:2,C16:0), Tricosanoic acid (C23:0), Hexadecanoylcarnitine, Sphingomyelin (d18:1,C24:0), 3-Hydroxybutyrate, Octadecanoylcarnitine, SM_Sphingomyelin (d18:1,C19:0), SM_Sphingomyelin (d18:2,C18:1), SM_Sphingomyelin (d18:2,C22:1), SM_Sphingomyelin (d18:2,C20:1), SM_Sphingomyelin (d18:2,C20:0), SM_Sphingomyelin (d18:2,C19:0), SM_Sphingomyelin (d18:2,C18:0), CER_Ceramide (d18:2,C20:0), SM_Sphingomyelin (d18:1,C20:0), CER_Ceramide (d18:1,C20:0), SM_Sphingomyelin (d18:2,C22:0), SM_Sphingomyelin (d17:1,C18:0), CER_Ceramide (d18:2,C18:0), SM_Sphingomyelin (d17:1,C20:0), CER_Ceramide (d18:1,C18:0), SM_Sphingomyelin (d18:2,C21:0), SM_Sphingomyelin (d18:2,C24:2), SM_Sphingomyelin (d18:2,C23:1), SM_Sphingomyelin (d18:1,C21:0), CER_Ceramide (d18:1,C21:0), CER_Ceramide (d18:2,C22:0), SM_Sphingomyelin (d18:2,C24:1), CER_Ceramide (d16:1,C18:0), SM_Sphingomyelin (d18:1,C22:0), CER_Ceramide (d17:1,C22:0), CER_Ceramide (d18:1,C22:0), SM_Sphingomyelin (d17:1,C22:0), CER_Ceramide (d17:1,C16:0), SM_Sphingomyelin (d17:1,C24:1), CER_Ceramide (d16:1,C20:0), CER_Ceramide (d17:1,C24:1), SM_Sphingomyelin (d16:1,C20:0), CER_Ceramide (d18:1,C22:1), CER_Ceramide (d18:2,C16:0), SM_Sphingomyelin (d16:1,C18:1), SM_Sphingomyelin (d18:2,C16:0), CER_Ceramide (d18:2,C24:1), CER_Ceramide (d18:1,C23:1), CER_Ceramide (d18:1,C24:2), SM_Sphingomyelin (d18:2,C24:0), SM_Sphingomyelin (d17:1,C16:0), SM_Sphingomyelin (d16:1,C18:0), SM_Sphingomyelin (d16:1,C22:1), CER_Ceramide (d18:1,C24:1), SM_Sphingomyelin (d18:2,C23:0), CER_Ceramide (d18:2,C24:0), CE_Cholesterylester C20:4, CER_Ceramide (d18:1,C16:0), CER_Ceramide (d16:1,C22:0), Sphingosine-1-phosphate (d17:1), CER_Ceramide (d18:2,C23:0), SM_Sphingomyelin (d18:2,C14:0), SM_Sphingomyelin (d17:1,C24:0), CER_Ceramide (d18:1,C14:0), SM_Sphingomyelin (d17:1,C23:0), CER_Ceramide (d16:1,C16:0), CER_Ceramide (d18:1,C24:0), SM_Sphingomyelin (d16:1,C22:0), CER_Ceramide (d16:1,C24:1), CER_Ceramide (d17:1,C24:0), SM_Sphingomyelin (d18:1,C16:0), SM_Sphingomyelin (d18:1,C23:0), SM_Sphingomyelin (d16:1,C21:0), SM_Sphingomyelin (d18:1,C24:0), Sphingadienine-1-phosphate (d18:2), CER_Ceramide (d18:1,C23:0), SM_Sphingomyelin (d16:1,C24:1), SM_Sphingomyelin (d18:1,C14:0),and CER_Ceramide (d17:1,C23:0).

Most preferably, the aforementioned group of biomarkers is determined as the at least one biomarker in the method of the invention. More preferably, said aforementioned at least one biomarker is capable of diagnosing pancreatic carcinoma in a subject when compared to a reference derived from a group of subjects comprising healthy subjects and/or subjects suffering from pancreatitis and/or subjects suffering from liver cirrhosis (eg non-cancer group). More preferably, said diagnosis is achieved with analysis approaches set forth in Table 1, below.

Preferably, the aforementioned group of biomarkers comprises at least two ceramides and at least one sphingomyeline, 3 ceramides and at least 2 sphingomyelines, 4 ceramides and at least 3 sphingomyelines, 5 ceramides and at least 4 sphingomyelines, or 6 ceramides and at least 5 sphingomyelines,

A metabolite as used herein refers to at least one molecule of a specific metabolite up to a plurality of molecules of the said specific metabolite. It is to be understood further that a group of metabolites means a plurality of chemically different molecules wherein for each metabolite at least one molecule up to a plurality of molecules may be present. A metabolite in accordance with the present invention encompasses all classes of organic or inorganic chemical compounds including those being comprised by biological material such as organisms. Preferably, the metabolite in accordance with the present invention is a small molecule compound. More preferably, in case a plurality of metabolites is envisaged, said plurality of metabolites representing a metabolome, i.e. the collection of metabolites being comprised by an organism, an organ, a tissue, a body fluid or a cell at a specific time and under specific conditions.

In addition to the specific biomarkers recited in the specification, other biomarkers may be, preferably, determined as well in the methods of the present invention. Such biomarkers may include peptide or polypeptide biomarkers or glycosides such as the CA19.9 antigen.

The sample is a blood, plasma or serum sample. Preferably, the sample is a plasma sample.

The aforementioned samples are, preferably, pre-treated before they are used for the method of the present invention. As described in more detail below, said pre-treatment may include treatments required to release or separate the compounds or to remove excessive material or waste. Suitable techniques comprise centrifugation, extraction, fractioning, ultrafiltration, protein precipitation followed by filtration and purification and/or enrichment of compounds. Moreover, other pre-treatments are carried out in order to provide the compounds in a form or concentration suitable for compound analysis. For example, if gas-chromatography coupled mass spectrometry is used in the method of the present invention, it will be required to derivatize the compounds prior to the said gas chromatography. Suitable and necessary pre-treatments depend on the means used for carrying out the method of the invention and are well known to the person skilled in the art. Pre-treated samples as described before are also comprised by the term "sample" as used in accordance with the present invention.

The term "subject" as used herein relates to animals and, preferably, to mammals. More preferably, the subject is a primate and, most preferably, a human. The subject, preferably, is suspected to suffer from pancreatic cancer, i.e. it may already show some or all of the symptoms associated with the disease. Moreover, the subject may also preferably suffer from or shall be suspected to suffer from pancreatitis and/or liver cirrhosis in addition. Preferably, the subject, however, is besides the aforementioned diseases and disorders apparently healthy. The said subject, preferably, is at increased risk of developing pancreatic cancer (Brand RE et al, Gut. 2007;56:1460-9). More preferably, such a subject being at increased risk has one or more relatives suffering from pancreatic cancer, has a defined genetic predisposition for developing pancreatic cancer, including but not exclusive to Peutz-Jeghers Syndrome, has one or more relatives suffering from pancreatitis, and/or has a defined genetic predisposition for developing pancreatitis.

The term "determining the amount" as used herein refers to determining at least one characteristic feature of a biomarker to be determined by the method of the present invention in the sample. Characteristic features in accordance with the present invention are features which characterize the physical and/or chemical properties including biochemical properties of a biomarker. Such properties include, e.g., molecular weight, viscosity, density, electrical charge, spin, optical activity, colour, fluorescence, chemoluminescence, elementary composition, chemical structure, capability to react with other compounds, capability to elicit a response in a biological read out system (e.g., induction of a reporter gene) and the like. Values for said properties may serve as characteristic features and can be determined by techniques well known in the art. Moreover, the characteristic feature may be any feature which is derived from the values of the physical and/or chemical properties of a biomarker by standard operations, e.g., mathematical calculations such as multiplication, division or logarithmic calculus. Most preferably, the at least one characteristic feature allows the determination and/or chemical identification of the said at least one biomarker and its amount. Accordingly, the characteristic value, preferably, also comprises information relating to the abundance of the biomarker from which the characteristic value is derived. For example, a characteristic value of a biomarker may be a peak in a mass spectrum. Such a peak contains characteristic information of the biomarker, i.e. the m/z information, as well as an intensity value being related to the abundance of the said biomarker (i.e. its amount) in the sample.

As discussed before, each biomarker comprised by a sample may be, preferably, determined in accordance with the present invention quantitatively or semi-quantitatively. For quantitative determination, either the absolute or precise amount of the biomarker will be determined or the relative amount of the biomarker will be determined based on the value determined for the characteristic feature(s) referred to herein above. The relative amount may be determined in a case were the precise amount of a biomarker can or shall not be determined. In said case, it can be determined whether the amount in which the biomarker is present is enlarged or diminished with respect to a second sample comprising said biomarker in a second amount. In a preferred embodiment said second sample comprising said biomarker shall be a calculated reference as specified elsewhere herein. Quantitatively analysing a biomarker, thus, also includes what is sometimes referred to as semi-quantitative analysis of a biomarker.

Moreover, determining as used in the method of the present invention, preferably, includes using a compound separation step prior to the analysis step referred to before. Preferably, said compound separation step yields a time resolved separation of the metabolites comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be applied by the person skilled in the art without further ado. Most preferably, LC and/or GC are chromatographic techniques to be envisaged by the method of the present invention. Suitable devices for such determination of biomarkers are well known in the art. Preferably, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). Most preferably, LC-MS and/or GC-MS are used as described in detail below. Said techniques are disclosed in, e.g., Nissen 1995, Journal of Chromatography A, 703: 37-57, US 4,540,884 or US 5,397,894, the disclosure content of which is hereby incorporated by reference. As an alternative or in addition to mass spectrometry techniques, the following techniques may be used for compound determination: nuclear magnetic resonance (NMR), magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado. The method of the present invention shall be, preferably, assisted by automation. For example, sample processing or pre-treatment can be automated by robotics. Data processing and comparison is, preferably, assisted by suitable computer programs and databases. Automation as described herein before allows using the method of the present invention in high-throughput approaches.

Moreover, the at least one biomarker can also be determined by a specific chemical or biological assay. Said assay shall comprise means which allow to specifically detect the at least one biomarker in the sample. Preferably, said means are capable of specifically recognizing the chemical structure of the biomarker or are capable of specifically identifying the biomarker based on its capability to react with other compounds or its capability to elicit a response in a biological read out system (e.g., induction of a reporter gene). Means which are capable of specifically recognizing the chemical structure of a biomarker are, preferably, antibodies or other proteins which specifically interact with chemical structures, such as receptors or enzymes. Specific antibodies, for instance, may be obtained using the biomarker as antigen by methods well known in the art. Antibodies as referred to herein include both polyclonal and monoclonal antibodies, as well as fragments thereof, such as Fv, Fab and F(ab)$_2$ fragments that are capable of binding the antigen or hapten. The present invention also includes humanized hybrid antibodies wherein amino acid sequences of a non-human donor antibody exhibiting a desired antigen-specificity are combined with sequences of a human acceptor antibody. Moreover, encompassed are single chain antibodies. The donor sequences will usually include at least the antigen-binding amino acid residues of the donor but may comprise other structurally and/or functionally relevant amino acid residues of the donor antibody as well. Such hybrids can be prepared by several methods well known in the art. Suitable proteins which are capable of specifically recognizing the biomarker are, preferably, enzymes which are involved in the metabolic conversion of the said biomarker. Said enzymes may either use the biomarker as a substrate or may convert a substrate into the biomarker. Moreover, said antibodies may be used as a basis to generate oligopeptides which specifically recognize the biomarker. These oligopeptides shall, for example, comprise the enzyme's binding domains or pockets for the said biomarker. Suitable antibody and/or enzyme based assays may be RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests. Moreover, the biomarker may also be determined based on its capability to react with other compounds, i.e. by a specific chemical reaction. Further, the biomarker may be determined in a sample due to its capability to elicit a response in a biological read out system. The biological response shall be detected as read out indicating the presence and/or the amount of the biomarker comprised by the sample. The biological response may be, e.g., the induction of gene expression or a phenotypic response of a cell or an organism. In a preferred embodiment the determination of the least one biomarker is a quantitative process, e.g., allowing also the determination of the amount of the at least one biomarker in the sample

As described above, said determining of the at least one biomarker according to claim 1 can, preferably, comprise mass spectrometry (MS). Mass spectrometry as used herein encompasses all techniques which allow for the determination of the molecular weight (i.e. the mass) or a mass variable corresponding to a compound, i.e. a biomarker, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF or any combined approaches using the aforementioned techniques. How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, c) selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionisation process, whereby the quadrupole is filled with collision gas but no acceleration voltage is applied during the analysis. Details on said most preferred mass spectrometry to be used in accordance with the present invention can be found in WO 03/073464.

More preferably, said mass spectrometry is liquid chromatography (LC) MS and/or gas chromatography (GC) MS. Liquid chromatography as used herein refers to all techniques which allow for separation of compounds (i.e. metabolites) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatised prior to gas chromatography. Suitable techniques for derivatisation are well known in the art. Preferably, derivatisation in accordance with the present invention relates to methoxymation and trimethylsilylation of, preferably, polar compounds and transmethylation, methoxymation and trimethylsilylation of, preferably, non-polar (i.e. lipophilic) compounds.

The term "reference" refers to values of characteristic features of each of the biomarker which can be correlated to a medical condition, i.e. the presence or absence of the disease, diseases status or an effect referred to herein. Preferably, a reference is a threshold value (e.g., an amount or ratio of amounts) for a biomarker whereby values found in a sample to be investigated which are higher than or essentially identical to the threshold are indicative for the presence of a medical condition while those being lower are indicative for the absence of the medical condition. It will be understood that also preferably, a reference may be a threshold value for a biomarker whereby values found in a sample to be investigated which are lower or identical than the threshold are indicative for the presence of a medical condition while those being higher are indicative for the absence of the medical condition.

In accordance with the aforementioned method of the present invention, a reference is, preferably, a reference obtained from a sample from a subject or group of subjects known to suffer from pancreatic cancer. In such a case, a value for the at least one biomarker found in the test sample being essentially identical is indicative for the presence of the disease.

Moreover, the reference, also preferably, could be from a subject or group of subjects known not to suffer from pancreatic cancer, preferably, an apparently healthy subject. In such a case, a value for the at least one biomarker found in the test sample being altered with respect to the reference is indicative for the presence of the disease. The same applies mutatis mutandis for a calculated reference being, most preferably, the average or median for the relative value or the value for a degree of change of the at least one biomarker in a population of individuals (comprising the subject to be investigated). The relative values or degrees of changes of the at least one biomarker of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

The value for the at least one biomarker of the test sample and the reference values are essentially identical, if the values for the characteristic features and, in the case of quantitative determination, the intensity values are essentially identical. Essentially identical means that the difference between two values is, preferably, not significant and shall be characterized in that the values for the intensity are within at least the interval between 1st and 99th percentile, 5th and 95th percentile, 10th and 90th percentile, 20th and 80th percentile, 30th and 70th percentile, 40th and 60th percentile of the reference value, preferably, the 50th, 60th, 70th, 80th, 90th or 95th percentile of the reference value. Statistical test for determining whether two amounts are essentially identical are well known in the art and are also described elsewhere herein.

An observed difference for two values, on the other hand, shall be statistically significant. A difference in the relative or absolute value is, preferably, significant outside of the interval between 45th and 55th percentile, 40th and 60th percentile, 30th and 70th percentile, 20th and 80th percentile, 10th and 90th percentile, 5th and 95th percentile, 1st and 99th percentile of the reference value. Preferred relative changes of the medians or degrees of changes are described in the accompanying Tables as well as in the Examples. In the Tables below, a preferred relative change for the biomarkers is indicated as "up" for an increase and "down" for a decrease in column "direction of change". Values for preferred degrees of changes are indicated in the column "estimated fold change". The preferred references for the aforementioned relative changes or degrees of changes are indicated in the Tables below as well. It will be understood that these changes are, preferably, observed in comparison to the references indicated in the respective Tables, below.

Preferably, the reference, i.e. values for at least one characteristic feature of the at least one biomarker or ratios thereof, will be stored in a suitable data storage medium such as a database and are, thus, also available for future assessments.

The term "comparing" refers to determining whether the determined value of a biomarker is essentially identical to a reference or differs there from. Preferably, a value for a biomarker is deemed to differ from a reference if the observed difference is statistically significant which can be determined by statistical techniques referred to elsewhere in this description. If the difference is not statistically significant, the biomarker value and the reference are essentially identical.

Based on the comparison referred to above, a subject can be assessed to suffer from the disease, or not.

For the specific biomarkers referred to in this specification, preferred values for the changes in the relative amounts or ratios (i.e. the changes expressed as the ratios of the medians) are found in the Tables, below. Based on the ratios of the metabolites found in a subject suffering from pancreatic cancer and an apparently healthy control (blood donors) or a non-cancer group (blood donors, chronic pancreatitis and liver cirrhosis), respectively, and the calculated t-values as shown in Tables 2a, 2b, 3a, 3b, 4a, or 4b below, it can be derived whether an increase or a decrease of a given biomarker of Tables 2a, 2b, 3a, 3b, 4a, or 4b is indicative for the presence of pancreatic cancer. Negative t-values for a biomarker indicate that a decrease is indicative while positive t-values indicate that an increase of the biomarker is indicative for pancreatic cancer. It will be understood that the reference in said cases is derived from a subject or group of subjects known not to suffer from pancreatic cancer or is a calculated reference as defined elsewhere herein.

The comparison is, preferably, assisted by automation. For example, a suitable computer program comprising algorithms for the comparison of two different data sets (e.g., data sets comprising the values of the characteristic feature(s)) may be used. Such computer programs and algorithms are well known in the art. Notwithstanding the above, a comparison can also be carried out manually.

Advantageously, it has been found in the study underlying the present invention that the amount of the biomarker as referred to in claim 1 is an indicator for pancreatic cancer. Accordingly the amount of this biomarker as specified above in a sample can, in principle, be used for assessing whether a subject suffers from pancreatic cancer, or not. This is particularly helpful for an efficient diagnosis of the disease as well as for improving of the pre-clinical and clinical management of pancreatic cancer as well as an efficient monitoring of patients. Moreover, the findings underlying the present invention will also facilitate the development of efficient drug-based therapies or other interventions against pancreatic cancer as set forth in detail below.

The definitions and explanations of the terms made above apply mutatis mutandis for the following embodiments of the present invention except specified otherwise herein below.

The disclosure also relates to a method for identifying whether a subject is in need for a therapy of pancreatic cancer or a change of therapy comprising the steps of the methods of the present invention and the further step of identifying a subject in need if pancreatic cancer is diagnosed.

The phrase "in need for a therapy of pancreatic cancer" as used herein means that the disease in the subject is in a status where therapeutic intervention is necessary or beneficial in order to ameliorate or treat pancreatic cancer or the symptoms associated therewith. Accordingly, the findings of the studies underlying the present invention do not only allow diagnosing pancreatic cancer in a subject but also allow for identifying subjects which should be treated by a pancreatic cancer therapy or whose pancreatic cancer therapy needs adjustment. Once the subject has been identified, the method may further include a step of making recommendations for a therapy of pancreatic cancer.

A therapy of pancreatic cancer as according to the disclosure, preferably, comprises surgery, radiotherapy or drug treatment. Preferred surgery-based therapies include resection of the pancreas or parts thereof such as pancreaticoduodenectomy, tail pancreatectomy. total or partial pancreatoctomy, palliative bridging procedures. Drug-based therapies, preferably, include the administration of one or more drugs with antitumour properties including but not exclusive to platinum derivatives, fluoropyrimidines, pyrimidine analogues, Gemcitabine, antimetabolites, alkylating agents, anthracyclines, plant alkaloids, topoisomerase inhibitors, targeted antibodies and tryosine kinase inhibitors.

The present invention also relates to a device or system for diagnosing pancreas cancer in a sample of a subject comprising:

(a) an analyzing unit for a plasma, blood or serum sample of the subject comprising a detector for the biomarker Ceramide (d18:1, C24:0) said detector allowing for the determination of the amount of the said biomarker in the sample; and operatively linked thereto,

(b) an evaluation unit comprising a data processing unit and a data base, said data base comprising a stored reference and said data processing unit having tangibly embedded an algorithm for carrying out a comparison of the amount of the biomarker determined by the analyzing unit and the stored reference and for generating an output information based on which the diagnosis can be established, wherein the output information is at least a classifier which indicates whether the subject suffers from pancreatic cancer or not.

A device as used herein shall comprise at least the aforementioned units. The units of the device are operatively linked to each other. How to link the means in an operating manner will depend on the type of units included into the device. For example, where the detector allows for automatic qualitative or quantitative determination of the biomarker, the data obtained by said automatically operating analyzing unit can be processed by, e.g., a computer program in order to facilitate the assessment in the evaluation unit. Preferably, the units are comprised by a single device in such a case. Said device may accordingly include an analyzing unit for the biomarker and a computer or data processing device as evaluation unit for processing the resulting data for the assessment and for stabling the output information. Preferred devices are those which can be applied without the particular knowledge of a specialized clinician, e.g., electronic devices which merely require loading with a sample. The output information of the device, preferably, is a numerical value which allows drawing conclusions on the presence or absence of pancreatic cancer and, thus, is an aid for diagnosis. More preferably, the output information is a preliminary diagnosis or an aid for diagnosis based on the aforementioned numerical value, i.e. a classifier which indicates whether the subject suffers from pancreatic cancer or not. Such a preliminary diagnosis may need the evaluation of further information which can be provided in the device of the invention by including an expert knowledge database system.

A preferred reference to be used as a stored reference in accordance with the device of the present invention is an amount for the at least one biomarker to be analyzed or values derived therefrom which are derived from a subject or group of subjects known to suffer from pancreatic cancer. In such a case, the algorithm tangibly embedded, preferably, compares the determined amount for the at least one biomarker with the reference wherein an identical or essentially identical amount or value shall be indicative for the presence of pancreatic cancer in the subject.

Alternatively, another preferred reference to be used as a stored reference in accordance with the device of the present invention is an amount for the at least one biomarker to be analyzed or values derived therefrom which are derived from a subject or group of subjects known not to suffer from pancreatic cancer. In such a case, the algorithm tangibly embedded, preferably, compares the determined amount for the at least one biomarker with the reference wherein an amount or value which differs from the reference shall be indicative for the presence of pancreatic cancer in the subject. Preferred differences are those indicated as relative changes or degrees of changes for the individual biomarkers in the Tables below.

The units of the device, also preferably, can be implemented into a system comprising several devices which are operatively linked to each other. Depending on the units to be used for the system of the present invention, said means may be functionally linked by connecting each mean with the other by means which allow data transport in between said means, e.g., glass fiber cables, and other cables for high throughput data transport. Nevertheless, wireless data transfer between the means is also envisaged by the present invention, e.g., via LAN (Wireless LAN, W-LAN). A preferred system comprises means for determining biomarkers. Means for determining biomarkers as used herein encompass means for separating biomarkers, such as chromatographic devices, and means for metabolite determination, such as mass spectrometry devices. Suitable devices have been described in detail above. Preferred means for compound separation to be used in the system of the present invention include chromatographic devices, more preferably devices for liquid chromatography, HPLC, and/or gas chromatography. Preferred devices for compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other.

Most preferably, LC-MS and/or GC-MS are used in the system of the present invention as described in detail elsewhere in the specification. Further comprised shall be means for comparing and/or analyzing the results obtained from the means for determination of biomarkers. The means for comparing and/or analyzing the results may comprise at least one databases and an implemented computer program for comparison of the results. Preferred embodiments of the aforementioned systems and devices are also described in detail below.

Moreover, the present disclosure relates to a diagnostic means comprising means for the determination of at least one biomarker selected from any one of the groups referred to above.

The term "diagnostic means", preferably, relates to a diagnostic device, system or biological or chemical assay as specified elsewhere in the description in detail.

The expression "means for the determination of at least one biomarker" refers to devices or detection agents which are capable of specifically recognizing the biomarker. Suitable devices may be spectrometric devices such as mass spectrometry, NMR devices or devices for carrying out chemical or biological assays for the biomarkers. Suitable detection agents may be compounds which specifically detect the biomarkers. Detection as used herein may be a two-step process, i.e. the compound may first bind specifically to the biomarker to be detected and subsequently generate a detectable signal, e.g., fluorescent signals, chemiluminescent signals, radioactive signals and the like. For the generation of the detectable signal further compounds may be required which are all comprised by the term "means for determination of the at least one biomarker". Compounds which specifically bind to the biomarker are described elsewhere in the specification in detail and include, preferably, enzymes, antibodies, aptameres, ligands, receptors or other biological molecules or chemicals which specifically bind to the biomarkers.

In general, the present invention contemplates the use of the biomarker Ceramide (d18:1,C24:0) or a detection agent therefor in a plasma, blood or serum sample of a subject suspected to suffer from pancreatic cancer for diagnosing pancreatic cancer.

antimetabolites, alkylating agents, anthracyclines, plant alkaloids, topoisomerase inhibitors, targeted antibodies and tryosine kinase inhibitors.

The present disclosure further relates to a method for determining whether a therapy against pancreatic cancer is successful in a subject comprising the steps of the methods of the present invention and the further step of determining whether a therapy is successful if no pancreatic cancer is diagnosed.

It is to be understood that a pancreatic cancer therapy will be successful if pancreatic cancer or at least some symptoms thereof are treated or ameliorated compared to an untreated subject. Moreover, a therapy is also successful as meant herein if the disease progression can be prevented or at least slowed down compared to an untreated subject.

The present disclosure also relates to a device or system for diagnosing pancreas cancer in a sample of a subject comprising:

(a) an analyzing unit for the said sample of the subject comprising a detector for at least one biomarker of Tables 2a, 2b, 3a, 3b, 4a, or 4b or at least one biomarker of Tables 6, 7, 8, 9, 10, 11, 12, 13, 14, 15, 16, 17, 18, 19, 20, 21, 22, 23, 24, 25, 26, 27, 28, 29, 30, 31 or 32 said detector allowing for the determination of the amount of the said at least one biomarker in the sample; and operatively linked thereto,
(b) an evaluation unit comprising a data processing unit and a data base, said data base comprising a stored reference, preferably a reference as specified above in connection with the method of the invention and, more preferably, a reference derived from a subject or group of subjects known to suffer from pancreas cancer, and said data processing unit having tangibly embedded an algorithm for carrying out a comparison, preferably as specified above in connection with the method of the invention, of the amount of the at least one biomarker determined by the analyzing unit and the stored reference and for generating an output information based on which the diagnosis can be established.

A device as used herein shall comprise at least the aforementioned units. The units of the device are operatively linked to each other. How to link the means in an operating manner will depend on the type of units included into the device. For example, where the detector allows for automatic qualitative or quantitative determination of the biomarker, the data obtained by said automatically operating analyzing unit can be processed by, e.g., a computer program in order to facilitate the assessment in the evaluation unit. Preferably, the units are comprised by a single device in such a case. Said device may accordingly include an analyzing unit for the biomarker and a computer or data processing device as evaluation unit for processing the resulting data for the assessment and for stabling the output information. Preferred devices are those which can be applied without the particular knowledge of a specialized clinician, e.g., electronic devices which merely require loading with a sample. The output information of the device, preferably, is a numerical value which allows drawing conclusions on the presence or absence of pancreatic cancer and, thus, is an aid for diagnosis. More preferably, the output information is a preliminary diagnosis or an aid for diagnosis based on the aforementioned numerical value, i.e. a classifier which indicates whether the subject suffers from pancreatic cancer or not. Such a preliminary diagnosis may need the evaluation of further information which can be provided in the device of the invention by including an expert knowledge database system.

A preferred reference to be used as a stored reference in accordance with the device of the present invention is an amount for the at least one biomarker to be analyzed or values derived therefrom which are derived from a subject or group of subjects known to suffer from pancreatic cancer. In such a case, the algorithm tangibly embedded, preferably, compares the determined amount for the at least one biomarker with the reference wherein an identical or essentially identical amount or value shall be indicative for the presence of pancreatic cancer in the subject.

Alternatively, another preferred reference to be used as a stored reference in accordance with the device of the present invention is an amount for the at least one biomarker to be analyzed or values derived therefrom which are derived from a subject or group of subjects known not to suffer from pancreatic cancer. In such a case, the algorithm tangibly embedded, preferably, compares the determined amount for the at least one biomarker with the reference wherein an amount or value which differs from the reference shall be indicative for the presence of pancreatic cancer in the subject. Preferred differences are those indicated as relative changes or degrees of changes for the individual biomarkers in the Tables below.

The units of the device, also preferably, can be implemented into a system comprising several devices which are operatively linked to each other. Depending on the units to be used for the system of the present disclosure, said means may be functionally linked by connecting each mean with the other by means which allow data transport in between said means, e.g., glass fiber cables, and other cables for high throughput data transport. Nevertheless, wireless data transfer between the means is also envisaged, e.g., via LAN (Wireless LAN, W-LAN). A preferred system comprises means for determining biomarkers. Means for determining biomarkers as used herein encompass means for separating biomarkers, such as chromatographic devices, and means for metabolite determination, such as mass spectrometry devices. Suitable devices have been described in detail above. Preferred means for compound separation to be used in the system of the present disclosure include chromatographic devices, more preferably devices for liquid chromatography, HPLC, and/or gas chromatography. Preferred devices for compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other. Most preferably, LC-MS and/or GC-MS are used in the system of the present disclosure as described in detail elsewhere in the specification. Further comprised shall be means for comparing and/or analyzing the results obtained from the means for determination of biomarkers. The means for comparing and/or analyzing the results may comprise at least one databases and an implemented computer program for comparison of the results. Preferred embodiments of the aforementioned systems and devices are also described in detail below.

Furthermore, the present disclosure relates to a data collection comprising characteristic values of at least one biomarker being indicative for a medical condition or effect as set forth above (i.e. diagnosing pancreatic cancer in a subject, identifying whether a subject is in need for a therapy of pancreatic cancer or determining whether a pancreatic cancer therapy is successful). The term "data collection" refers to a collection of data which may be physically and/or logically grouped together. Accordingly, the data collection may be implemented in a single data storage medium or in physically separated data storage media being operatively linked to each other. Preferably, the data collection is implemented by means of a database. Thus, a database as used herein comprises the data collection on a suitable storage medium. Moreover, the database, preferably, further comprises a database management system. The database management system is, preferably, a network-based, hierarchical or object-oriented database management system. Furthermore, the database may be a federal or integrated database. More preferably, the database will be implemented as a distributed (federal) system, e.g. as a Client-Server-System. More preferably, the database is structured as to allow a search algorithm to compare a test data set with the data sets comprised by the data collection. Specifically, by using such an algorithm, the database can be searched for similar or identical data sets being indicative for a medical condition or effect as set forth above (e.g. a query search). Thus, if an identical or similar data set can be identified in the data collection, the test data set will be associated with the said medical condition or effect. Consequently, the information obtained from the data collection can be used, e.g., as a reference for the methods described above. More preferably, the data collection comprises characteristic values of all biomarkers comprised by any one of the groups recited above.

In light of the foregoing, the present disclosure encompasses a data storage medium comprising the aforementioned data collection.

The term "data storage medium" as used herein encompasses data storage media which are based on single physical entities such as a CD, a CD-ROM, a hard disk, optical storage media, or a diskette. Moreover, the term further includes data storage media consisting of physically separated entities which are operatively linked to each other in a manner as to provide the aforementioned data collection, preferably, in a suitable way for a query search.

The present disclosure also relates to a system comprising:

(a) means for comparing characteristic values of the at least one biomarker of a sample operatively linked to
(b) a data storage medium as described above.

The term "system" as used herein relates to different means which are operatively linked to each other. Said means may be implemented in a single device or may be physically separated devices which are operatively linked to each other. The means for comparing characteristic values of biomarkers, preferably, based on an algorithm for comparison as mentioned before. The data storage medium, preferably, comprises the aforementioned data collection or database, wherein each of the stored data sets being indicative for a medical condition or effect referred to above. Thus, the system allows identifying whether a test data set is comprised by the data collection stored in the data storage medium. Consequently, the methods of the present invention can be implemented by the system.

In a preferred aspect of the system, means for determining characteristic values of biomarkers of a sample are comprised. The term "means for determining characteristic values of biomarkers" preferably relates to the aforementioned devices for the determination of metabolites such as mass spectrometry devices, NMR devices or devices for carrying out chemical or biological assays for the biomarkers.

Moreover, the present disclosure relates to a diagnostic means comprising means for the determination of at least one biomarker selected from any one of the groups referred to above. The term "diagnostic means", preferably, relates to a diagnostic device, system or biological or chemical assay as specified elsewhere in the description in detail. The expression "means for the determination of at least one biomarker" refers to devices or detection agents which are capable of specifically recognizing the biomarker. Suitable devices may be spectrometric devices such as mass spectrometry, NMR devices or devices for carrying out chemical or biological assays for the biomarkers. Suitable detection agents may be compounds which specifically detect the biomarkers. Detection as used herein may be a two-step process, i.e. the compound may first bind specifically to the biomarker to be detected and subsequently generate a detectable signal, e.g., fluorescent signals, chemiluminescent signals, radioactive signals and the like. For the generation of the detectable signal further compounds may be required which are all comprised by the term "means for determination of the at least one biomarker". Compounds which specifically bind to the biomarker are described elsewhere in the specification in detail and include, preferably, enzymes, antibodies, aptameres, ligands, receptors or other biological molecules or chemicals which specifically bind to the biomarkers.

Further, the present disclosure relates to a diagnostic composition comprising at least one biomarker selected from any one of the groups referred to above.

The at least one biomarker selected from any of the aforementioned groups will serve as a biomarker, i.e. an indicator molecule for a medical condition or effect in the subject as set for the elsewhere herein. Thus, the biomarker molecules itself may serve as diagnostic compositions, preferably, upon visualization or detection by the means referred to in herein. Thus, a diagnostic composition which indicates the presence of a biomarker according to the present disclosure may also comprise the said biomarker physically, e.g., a complex of an antibody and the biomarker to be detected may serve as the diagnostic composition. Accordingly, the diagnostic composition may further comprise means for detection of the metabolites as specified elsewhere in this description. Alternatively, if detection means such as MS or NMR based techniques are used, the molecular species which serves as an indicator for the risk condition will be the at least one biomarker comprised by the test sample to be investigated. Thus, the at least one biomarker referred to in accordance with the present disclosure shall serve itself as a diagnostic composition due to its identification as a biomarker.

In general, the present disclosure contemplates the use of at least one biomarker of Tables 2a, 2b, 3a, 3b, 4a, or 4b or a detection agent therefor or at least one biomarker of Tables 6, 7, 8, 9, 10, 11, 12, 13, 14, 15, 16, 17, 18, 19, 20, 21, 22, 23, 24, 25, 26, 27, 28, 29, 30, 31 or 32 or a detection agent therefor in a sample of a subject for diagnosing pancreatic cancer.

Preferably, at least one biomarker of tables 12, 13, 14, 24, 25 or 26 can be used for differentiating in a subject between pancreatic cancer and a disease of the critical controls, i.e. pancreatitis and/or liver cirrhosis. Preferably, at least one biomarker from table 6, 7, 8, 15, 16, 17, 27, 28 or 29 can be used for differentiating in a subject between pancreatic cancer and pancreatitis. Preferably, at least one biomarker from table 18, 19, 20, 30, 31 or 32 be used for differentiating in a subject between pancreatic cancer and liver cirrhosis.

Moreover, the present disclosure relates also to the use of at least one biomarker of Tables 2a, 2b, 3a, 3b, 4a, or 4b or a detection agent therefor or at least one biomarker of Tables 6, 7, 8, 9, 10, 11, 12, 13, 14, 15, 16, 17, 18, 19, 20, 21, 22, 23, 24, 25, 26, 27, 28, 29, 30, 31 or 32 or a detection agent therefor for the manufacture of a diagnostic composition or a pharmaceutical composition for diagnosing pancreatic cancer based on a sample of a subject.

Preferably, at least one biomarker of tables 12, 13, 14, 24, 25 or 26 can be used for differentiating in a subject between pancreatic cancer and a disease of the critical controls, i.e. pancreatitis and/or liver cirrhosis. Preferably, at least one biomarker from table 6, 7, 8, 15, 16, 17, 27, 28 or 29 can be used for differentiating in a subject between pancreatic cancer and pancreatitis. Preferably, at least one biomarker from table 18, 19, 20, 30, 31 or 32 can be used for differentiating in a subject between pancreatic cancer and liver cirrhosis.

How diagnostic and/or pharmaceutical compositions for diagnosing pancreatic cancer can be manufactured based on the at least one biomarker or the detection agent therefor is well known to those skilled in the art. For example, antibodies or aptameres which specifically bind to the at least one biomarker can be produced. Similarly, the biomarkers itself may be used as such compositions, e.g., within complexes or in modified or derivatizsed form, e.g., when analysed by GCMS.

**Examples**

The invention will now be illustrated by the following Examples which are not intended to restrict or limit the scope of this invention.

**Example 1:** Sample preparation and MS Analysis

The study comprised 80 pancreatic adenocarcinoma patients, 80 patients with alcohol induced liver cirrhosis and 80 patients suffering from chronic pancreatitis as well as 80 healthy volunteers (blood donors). Patients and healthy volunteers were matched for age, gender and BMI. For all patients and healthy volunteers a blood sample was collected. Plasma was prepared by centrifugation, and samples were stored at -80 °C until measurements were performed.
The analysis of the plasma samples revealed biomarker candidates which where classified in two categories. The analysis of the plasma samples was carried out as follows:
Human plasma samples were prepared and subjected to LC-MS/MS and GC-MS or SPE-LC-MS/MS (hormones) analysis as described in the following:
Proteins were separated by precipitation from blood plasma. After addition of water and a mixture of ethanol and dichlormethan the remaining sample was fractioned into an aqueous, polar phase (polar fraction) and an organic, lipophilic phase (lipid fraction).

For the transmethanolysis of the lipid extracts a mixture of 140 $\mu$l of chloroform, 37 $\mu$l of hydrochloric acid (37% by weight HCl in water), 320 $\mu$l of methanol and 20 $\mu$l of toluene was added to the evaporated extract. The vessel was sealed tightly and heated for 2 hours at 100°C, with shaking. The solution was subsequently evaporated to dryness. The residue was dried completely.

The methoximation of the carbonyl groups was carried out by reaction with methoxyamine hydrochloride (20 mg/ml in pyridine, 100 l for 1.5 hours at 60°C) in a tightly sealed vessel. 20 $\mu$l of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 100 $\mu$l of N-methyl-N-(trimethylsilyl)-2,2,2-trifluoroacetamide (MSTFA) was carried out for 30 minutes at 60°C, again in the tightly sealed vessel. The final volume before injection into the GC was 220 $\mu$l.

For the polar phase the derivatization was performed in the following way: The methoximation of the carbonyl groups was carried out by reaction with methoxyamine hydrochloride (20 mg/ml in pyridine, 50 l for 1.5 hours at 60°C) in a tightly sealed vessel. 10 $\mu$l of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 50 $\mu$l of N-methyl-N-(trimethylsilyl)-2,2,2-trifluoroacetamide (MSTFA) was carried out for 30 minutes at 60°C, again in the tightly sealed vessel. The final volume before injection into the GC was 110 $\mu$l.

The GC-MS systems consist of an Agilent 6890 GC coupled to an Agilent 5973 MSD. The autosamplers are CompiPal or GCPal from CTC.

For the analysis usual commercial capillary separation columns (30 m x 0,25 mm x 0,25 $\mu$m) with different poly-methyl-siloxane stationary phases containing 0 % up to 35% of aromatic moieties, depending on the analysed sample materials and fractions from the phase separation step, were used (for example: DB-1ms, HP-5ms, DB-XLB, DB-35ms, Agilent Technologies). Up to 1 $\mu$L of the final volume was injected splitless and the oven temperature program was started at 70 °C and ended at 340 °C with different heating rates depending on the sample material and fraction from the phase separation step in order to achieve a sufficient chromatographic separation and number of scans within each analyte peak. Furthermore RTL (Retention Time Locking, Agilent Technologies) was used for the analysis and usual GC-MS standard conditions, for example constant flow with nominal 1 to 1.7 ml/min. and helium as the mobile phase gas, ionisation was done by electron impact with 70 eV, scanning within a m/z range from 15 to 600 with scan rates from 2.5 to 3 scans/sec and standard tune conditions.

The HPLC-MS systems consisted of an Agilent 1100 LC system (Agilent Technologies, Wald-bronn, Germany) coupled with an API 4000 Mass spectrometer (Applied Biosystem/MDS SCI-EX, Toronto, Canada). HPLC analysis was performed on commercially available reversed phase separation columns with C18 stationary phases (for example: GROM ODS 7 pH, Thermo Betasil C18). Up to 10 $\mu$L of the final sample volume of evaporated and reconstituted polar and lipophilic phase was injected and separation was performed with gradient elution using methanol/water/formic acid or acetonitrile/water/formic acid gradients at a flowrate of 200 $\mu$L/min.

Mass spectrometry was carried out by electrospray ionisation in positive mode for the non-polar fraction and negative or positive mode for the polar fraction using multiple-reaction-monitoring-(MRM)-mode and fullscan from 100 - 1000 amu.

Steroids and their metabolites were measured by online SPE-LC-MS (Solid phase extraction-LC-MS For steroids and related metabolites, quantification was achieved by means of stable-isotope-labelled standards, and absolute concentrations were calculated.

Analysis of complex lipids in plasma samples:

Total lipids were extracted from plasma by liquid/liquid extraction using chloroform/methanol. The lipid extracts were subsequently fractionated by normal phase liquid chromatography (NPLC) into eleven different lipid groups according to Christie (Journal of Lipid Research (26), 1985, 507-512).

The fractions were analyzed by LC-MS/MS using electrospray ionization (ESI) and atmospheric pressure chemical ionization (APCI) with detection of specific multiple reaction monitoring (MRM) transitions for cholesterol esters (CE), free sterols (FS), sphingoymelins (SM), and ceramides (CER) respectively. Sphingosines and sphingosine-1-phosphates (SP) were analyzed by LC-MS/MS using electrospray ionization (ESI) with detection of specific multiple reaction monitoring (MRM) transitions as described by Schmidt H et.al., Prostaglandins & other Lipid Mediators 81(2006), 162-170. Metabolites in the Tables below are derived from one of these fractions include the respective abbreviation in their name.

The lipid classes Monoacylglycerides (MAG), Triacylglycerides (TAG), Phosphatidylcholines (PC), Phosphatidylserines (PS), Phosphatidylinositoles (PI), Lysophosphatidylcholines (LPC), Diacylglycerols (DAG), Free fatty acids (FFA) were measured by GC-MS.

The fractions are analyzed by GC-MS after derivatization with TMSH (Trimethyl sulfonium hydroxide), yielding the fatty acid methyl esters (FAME) corresponding to the acyl moieties of the class-separated lipids. The concentrations of FAME from C14 to C24 are determined in each fraction.

Metabolites in the Tables below are derived from one of these fractions include the respective abbreviation in front of their name separated by an underscore.

**Example 2:** Data analysis and statistical evaluation

Plasma samples were analyzed in randomized analytical sequence design with pooled samples (so called "Pool") generated from aliquots of each sample. The raw peak data were normalized to the median of pool per analytical sequence to account for process variability (so called "ratios"). Ratios were log10 transformed to approach a normal distribution of data. Statistical analysis was done by a simple linear model (ANOVA) with the following fixed effects: Disease, body mass index (BMI), age, gender and storage time (storage):

$$\text{Disease + BMI + age + gender + storage}$$

The ANOVA factors "age", "BMI" and "storage" were set as numerical factors, the factor "gender" was set to the reference "male" and the factor "disease" was set to either healthy volunteers (Blood donors) as reference or, in a separate ANOVA model, to the to non-cancer group (blood donors, chronic pancreatitis and liver cirrhosis) as reference.

From the results of the ANOVA model, three different approaches for identification of biomarkers for pancreatic cancer were applied. These different approaches resulted in different biomarker categories that are explained in Table 1 and the identified biomarkers are listed in Tables 2a, 2b, 3a, 3b,4a and 4b below.

**Table 1:** Identification strategies of biomarker candidates for pancreatic cancer

| Statistical parameters of ANOVA model | Estimated fold change | Fold change ratio of pancreatic carcinoma relative to control, estimated from ANOVA on log10-transformed ratios with age, BMI, storage and gender as fixed effects. Control group consists either of healthy volunteers (blood donors) or of a non-cancer group (blood donors, chronic pancreatitis and liver cirrhosis) |
|---|---|---|
| | t-value | t-value of ANOVA on log10-transformed ratios with age, BMI, storage and gender as fixed effects, t-values give relative change in units of standard deviation. Negative t-values indicate decreases, positive indicate increases |
| | p-value | p-value of ANOVA on log10-transformed ratios with age, BMI, storage and gender as fixed effects, calculated from t-value by taking degrees of freedom and one- or two-sided test into consideration |
| | | |
| | | |

(continued)

| Potential biomarker candidate for pancreatic carcinoma category | versus healthy volunteers (blood donors), category 1 | Pankreatic carcinoma p-value <0.2 AND (Liver cirrhosis/ Pancreatitis 1-2 levels of significance weaker OR liver cirrhosis/ Pancreatitis p-value <0.2 with other direction); levels of significance being <0.05; <0.1; <0.2 |
|---|---|---|
| | versus healthy volunteers (blood donors), category 2 | Pancreatic carcinoma AND Pankreatitis p-value <0.2 AND with same direction, cirrhosis p-value >0.2 |
| | versus non-cancer group (blood donors, chronic pancreatitis and liver cirrhosis) | p-value < 0.1 |

**Table 2a:** List of identified biomarkers category 1 for pancreatic cancer in comparison relative to healthy volunteers (blood donors)

| Metabolite | Direction of change | ANOVA result of pancreatic carcinoma relative to healthy volunteers (blood donors) | | |
|---|---|---|---|---|
| | | Estimated fold change | p-value | t-value |
| TAG (C18:1 ,C18:2) | up | 1.36 | 0.02602 | 2.24 |
| 3-Indoxylsulfate | down | 0.60 | 0.09481 | -1.68 |

**Table 2b:** List of identified biomarkers category 1 from lipid analysis for pancreatic cancer in comparison relative to healthy volunteers (blood donors)

| Metabolite | Direction of change | ANOVA result of pancreatic carcinoma relative to healthy volunteers (blood donors) | | |
|---|---|---|---|---|
| | | Estimated fold change | p-value | t-value |
| SM_Sphingomyelin (d18:1,C19:0) | up | 1.51 | 0.00001 | 4.42 |
| SM_Sphingomyelin (d18:1,C21:0) | up | 1.49 | 0.00020 | 3.76 |
| SM_Sphingomyelin (d18:2,C22:0) | up | 1.51 | 0.00355 | 2.94 |
| SM_Sphingomyelin (d17:1,C24:1) | up | 1.32 | 0.00417 | 2.89 |
| SM_Sphingomyelin (d18:2,C20:0) | up | 1.35 | 0.00520 | 2.82 |
| SM_Sphingomyelin (d18:2,C19:0) | up | 1.42 | 0.00977 | 2.60 |
| SM_Sphingomyelin (d18:2,C21:0) | up | 1.30 | 0.04158 | 2.05 |
| CER_Ceramide (d17:1,C22:0) | up | 1.38 | 0.04412 | 2.02 |
| CER_Ceramide (d18:2,C22:0) | up | 1.36 | 0.04599 | 2.00 |

Abbreviation scheme for fatty acids and lipid analysis fractions (= lipid classes) from the analysis of complex lipids as described above: e.g. C24:1: Fatty acid with 24 Carbon atoms and 1 double bond in the carbon skeleton. The abbreviations schema is generally applicable to all the tables in the application and to the citation of the respective metabolites in the description of this application

| | | |
|---|---|---|
| **Lipid fractions abbreviations (= lipid classes)** | FFA | Free fatty acids |
| | TAG | Triacylglycerols |
| | DAG | Diacylglycerols |
| | MAG | Monoacylglycerols |
| | PE | Phosphatidylethanolamines |
| | PI | Phosphatidylinositols |
| | PC | Phosphatidylcholines |
| | PS | Phosphatidylserines |
| | CER | Ceramides |
| | FS | Free sterols |
| | LPC | Lysophosphatidylcholines |
| | CE | Cholesterylesters |
| | SM | Sphingomyelins |

**Table 3a:** List of identified biomarkers category 2 for pancreatic cancer in comparison relative to healthy volunteers (blood donors)

| Metabolite | Direction of change | ANOVA result of pancreatic carcinoma relative to healthy volunteers (blood donors) | | |
|---|---|---|---|---|
| | | **Estimated fold change** | **p-value** | **t-value** |
| 1-Hydroxy-2-amino-(cis,trans)-3,5-octadecadiene (from sphingolipids) | up | 1.74 | 0.00053 | 3.50 |
| Sphingomyelin (d18:2,C18:0) | up | 1.37 | 0.00118 | 3.28 |
| Glycerol phosphate, lipid fraction | up | 1.47 | 0.00876 | 2.64 |
| Arachidonic acid (C20:cis[5,8,11,14]4) | up | 1.42 | 0.01256 | 2.51 |
| Phosphate, lipid fraction | up | 1.23 | 0.01775 | 2.38 |
| myo-Inositol-2-phosphate, lipid fraction (myo-Inositolphospholipids) | up | 1.49 | 0.03785 | 2.09 |
| Cholesta-2,4,6-triene | up | 1.24 | 0.04447 | 2.02 |
| erythro-C16-Sphingosine | up | 1.44 | 0.05159 | 1.95 |
| dihomo-gamma-Linolenic acid (C20:cis [8,11,14]3) | up | 1.33 | 0.06321 | 1.86 |
| Cholestenol No 02 | up | 1.22 | 0.06781 | 1.83 |
| Phenylacetylglutamine | up | 1.65 | 0.06790 | 1.83 |
| Phosphatidylcholine (C18:0,C20:3) | up | 1.14 | 0.08110 | 1.75 |
| alpha-Tocopherol | up | 1.29 | 0.08591 | 1.72 |
| Cholesterol, total | up | 1.17 | 0.09350 | 1.68 |
| Glycerol-3-phosphate, polar fraction | up | 1.35 | 0.10166 | 1.64 |

(continued)

| Metabolite | Direction of change | ANOVA result of pancreatic carcinoma relative to healthy volunteers (blood donors) | | |
|---|---|---|---|---|
| | | Estimated fold change | p-value | t-value |
| Serine, lipid fraction | up | 1.26 | 0.11639 | 1.58 |
| conjugated Linoleic acid (C18:trans[9,11]2) | up | 1.22 | 0.17901 | 1.35 |
| DAG (C18:1,C18:2) | up | 1.19 | 0.19187 | 1.31 |
| Palmitic acid (C16:0) | up | 1.21 | 0.19455 | 1.30 |

**Table 3b:** List of identified biomarkers category 2 from lipid analysis which are altered between patients suffering from pancreatic cancer in comparison relative to healthy volunteers (blood donors). For abbreviations of lipid analysis fractions, refer to Table 2b.

| Metabolite | Direction of change | ANOVA result of pancreatic carcinoma relative to healthy volunteers (blood donors) | | |
|---|---|---|---|---|
| | | Estimated fold change | p-value | t-value |
| SM_Sphingomyelin (d18:2,C22:1) | up | 1.76 | 0.00000 | 5.54 |
| SM_Sphingomyelin (d18:2,C20:1) | up | 1.94 | 0.00000 | 5.03 |
| SM_Sphingomyelin (d18:2,C24:1) | up | 2.09 | 0.00000 | 4.93 |
| SM_Sphingomyelin (d18:1,C20:0) | up | 1.70 | 0.00000 | 4.67 |
| SM_Sphingomyelin (d18:1,C19:0) | up | 1.51 | 0.00001 | 4.42 |
| SM_Sphingomyelin (d18:2,C23:1) | up | 1.61 | 0.00002 | 4.34 |
| CER_Ceramide (d18:2,C18:0) | up | 2.14 | 0.00003 | 4.26 |
| SM_Sphingomyelin (d18:2,C18:1) | up | 1.80 | 0.00003 | 4.25 |
| SM_Sphingomyelin (d18:1,C22:0) | up | 1.73 | 0.00004 | 4.17 |
| SM_Sphingomyelin (d18:2,C16:0) | up | 1.71 | 0.00007 | 4.05 |
| SM_Sphingomyelin (d18:1,C21:0) | up | 1.49 | 0.00020 | 3.76 |
| CER_Ceramide (d18:2,C20:0) | up | 1.88 | 0.00024 | 3.72 |
| CER_Ceramide (d18:1,C22:0) | up | 1.52 | 0.00059 | 3.47 |
| CER_Ceramide (d16:1,C18:0) | up | 1.77 | 0.00127 | 3.25 |
| SM_Sphingomyelin (d16:1,C22:1) | up | 1.48 | 0.00139 | 3.23 |
| PC_Palmitic acid (C16:0) | up | 1.46 | 0.00202 | 3.12 |
| SM_Sphingomyelin (d16:1,C20:0) | up | 1.71 | 0.00233 | 3.07 |
| SM_Sphingomyelin (d16:1,C18:1) | up | 1.54 | 0.00315 | 2.98 |
| CER_Ceramide (d16:1,C20:0) | up | 1.62 | 0.00375 | 2.92 |
| SM_Sphingomyelin (d17:1,C24:1) | up | 1.32 | 0.00417 | 2.89 |
| PC_Stearic acid (C18:0) | up | 1.38 | 0.00440 | 2.87 |
| CER_Ceramide (d18:2,C24:1) | up | 1.51 | 0.00455 | 2.86 |
| CER_Ceramide (d17:1,C24:1) | up | 1.49 | 0.00503 | 2.83 |
| Sphingosine-1-phosphate (d18:1) | up | 1.63 | 0.00990 | 2.60 |

(continued)

| Metabolite | Direction of change | ANOVA result of pancreatic carcinoma relative to healthy volunteers (blood donors) | | |
|---|---|---|---|---|
| | | Estimated fold change | p-value | t-value |
| SM_Sphingomyelin (d16:1,C24:1) | up | 1.33 | 0.01326 | 2.49 |
| PC_Arachidonic acid (C20:cis [5,8,11,14]4) | up | 1.57 | 0.01416 | 2.47 |
| PC_dihomo-gamma-Linolenic acid (C20:cis[8,11,14]3) | up | 1.48 | 0.02174 | 2.31 |
| CER_Ceramide (d16:1,C24:1) | up | 1.38 | 0.02300 | 2.29 |
| SM_Sphingomyelin (d16:1,C16:0) | up | 1.34 | 0.02551 | 2.25 |
| TAG_dihomo-gamma-Linolenic acid (C20:cis[8,11,14]3) | up | 2.11 | 0.03013 | 2.18 |
| PI_Linoleic acid (C18:cis[9,12]2) | up | 3.16 | 0.03172 | 2.16 |
| PC_conjugated Linoleic acid (C18: cis[9]trans[11]2) | up | 2.21 | 0.03467 | 2.12 |
| PC_Linoleic acid (C18:cis[9,12]2) | up | 1.43 | 0.03909 | 2.07 |
| PI_Stearic acid (C18:0) | up | 1.28 | 0.04247 | 2.04 |
| PC_trans-Vaccenic acid (C18:trans [11]1) | up | 2.14 | 0.04490 | 2.01 |
| PE_Stearic acid (C18:0) | up | 1.36 | 0.04933 | 1.97 |
| TAG_Arachidonic acid (C20:cis [5,8,11,14]4) | up | 1.52 | 0.06974 | 1.82 |
| TAG_Docosatetraenoic acid (C22: cis[7,10,13,16]4) | up | 2.10 | 0.07076 | 1.81 |
| PI_Arachidonic acid (C20:cis [5,8,11,14]4) | up | 1.37 | 0.08619 | 1.72 |
| PE_Arachidonic acid (C20:cis [5,8,11,14]4) | up | 1.38 | 0.08935 | 1.70 |
| PI_dihomo-gamma-Linolenic acid (C20:cis[8,11,14]3) | up | 2.17 | 0.09658 | 1.67 |
| TAG_cis-Vaccenic acid | up | 1.37 | 0.11284 | 1.59 |
| PE_Docosahexaenoic acid (C22:cis [4,7,10,13,16,19]6) | up | 1.41 | 0.12394 | 1.54 |
| CE_Cholesterylester C18:1 | up | 1.17 | 0.15353 | 1.43 |
| CE_Cholesterylester C16:3 | up | 1.23 | 0.15666 | 1.42 |
| LPC_cis-Vaccenic acid | up | 1.60 | 0.16257 | 1.40 |
| PC_Eicosadienoic acid (C20:cis [11,14]2) | up | 1.27 | 0.16358 | 1.40 |
| FFA_Linoleic acid (C18:cis[9,12]2) | up | 1.32 | 0.16403 | 1.40 |
| TAG_Hexadecenoic acid (C16:trans [9]1) | up | 1.52 | 0.18401 | 1.33 |

**Table 4a:** List of identified biomarkers for pancreatic cancer in comparison relative to non-cancer group (blood donors, chronic pancreatitis and liver cirrhosis)

| Metabolite | Direction of change | ANOVA result of pancreatic carcinoma relative to non-cancer group (blood donors, chronic pancreatitis and liver cirrhosis) | | |
|---|---|---|---|---|
| | | Estimated fold change | p-value | t-value |
| Sphingomyelin (d18:2,C18:0) | up | 1.33 | 0.00000 | 4.88 |
| O-Acetylcarnitine | down | 0.63 | 0.00000 | -4.76 |
| 1-Hydroxy-2-amino-(cis,trans)-3,5-octadecadiene (from sphingolipids) | up | 1.51 | 0.00002 | 4.38 |
| erythro-Sphingosine | up | 1.41 | 0.00003 | 4.26 |
| Behenic acid (C22:0) | up | 1.34 | 0.00003 | 4.21 |
| Eicosanoic acid (C20:0) | up | 1.31 | 0.00008 | 4.00 |
| 3-O-Methylsphingosine (d18:1) | up | 1.52 | 0.00008 | 3.99 |
| 5-O-Methylsphingosine (d18:1) | up | 1.41 | 0.00009 | 3.96 |
| Cystine | down | 0.58 | 0.00025 | -3.70 |
| 1-Methylhistidine | down | 0.78 | 0.00045 | -3.55 |
| Phosphatidylcholine (C16:0,C20:4) | up | 1.15 | 0.00059 | 3.47 |
| 3-Hydroxyisobutyrate | down | 0.73 | 0.00061 | -3.46 |
| threo-Sphingosine | up | 1.28 | 0.00087 | 3.36 |
| Pseudouridine | down | 0.71 | 0.00114 | -3.28 |
| Nervonic acid (C24:cis[15]1) | up | 1.24 | 0.00123 | 3.26 |
| TAG (C18:2,C18:2) | up | 1.36 | 0.00141 | 3.22 |
| TAG (C16:0,C18:1,C18:3) | up | 1.41 | 0.00141 | 3.22 |
| 7-Methylguanosine | down | 0.78 | 0.00174 | -3.16 |
| Arachidonic acid (C20:cis [5,8,11,14]4) | up | 1.31 | 0.00183 | 3.14 |
| Cortisol | up | 1.35 | 0.00235 | 3.07 |
| erythro-C16-Sphingosine | up | 1.37 | 0.00571 | 2.78 |
| Erythrol | down | 0.79 | 0.00705 | -2.71 |
| Phosphate, lipid fraction | up | 1.15 | 0.00739 | 2.70 |
| N-Acetylcytidine | down | 0.76 | 0.00743 | -2.69 |
| Tetradecanoylcarnitine | down | 0.80 | 0.00751 | -2.69 |
| Heptadecanoic acid (C17:0) | up | 1.21 | 0.00806 | 2.67 |
| Phosphatidylcholine (C16:0,C16:0) | down | 0.86 | 0.00914 | -2.62 |
| Sarcosine | down | 0.82 | 0.00978 | -2.60 |
| 3-Indoxylsulfate | down | 0.63 | 0.00983 | -2.60 |
| Cholesterol, free | up | 1.16 | 0.01246 | 2.51 |
| Phytosphingosine (t18:0), total | up | 1.23 | 0.01281 | 2.50 |
| Canthaxanthin | down | 0.69 | 0.01449 | -2.46 |

(continued)

| Metabolite | Direction of change | ANOVA result of pancreatic carcinoma relative to non-cancer group (blood donors, chronic pancreatitis and liver cirrhosis) | | |
| --- | --- | --- | --- | --- |
| | | Estimated fold change | p-value | t-value |
| epsilon-Acetyllysi ne | down | 0.87 | 0.01470 | -2.45 |
| Sphingomyelin (d18:1,C23:0) | up | 1.18 | 0.01472 | 2.45 |
| 5-O-Methylsphingosine (d16:1) | up | 1.29 | 0.01489 | 2.45 |
| Ribonic acid | down | 0.80 | 0.01659 | -2.41 |
| Sphingomyelin (d18:2,C16:0) | up | 1.12 | 0.01758 | 2.39 |
| Corticosterone | up | 1.46 | 0.01803 | 2.38 |
| Tricosanoic acid (C23:0) | up | 1.21 | 0.01895 | 2.36 |
| Creatinine | down | 0.84 | 0.01931 | -2.35 |
| Glycerol, polar fraction | down | 0.79 | 0.01970 | -2.34 |
| Pantothenic acid | down | 0.80 | 0.02004 | -2.34 |
| Hexadecanoylcarnitine | down | 0.82 | 0.02184 | -2.30 |
| Isocitrate | down | 0.86 | 0.02311 | -2.28 |
| myo-Inositol | down | 0.83 | 0.02791 | -2.21 |
| gamma-Linolenic acid (C18:cis [6,9,12]3) | up | 1.32 | 0.02976 | 2.18 |
| Docosapentaenoic acid (C22:cis [4,7,10,13,16]5) | up | 1.25 | 0.03076 | 2.17 |
| dihomo-gamma-Linolenic acid (C20:cis[8,11,14]3) | up | 1.22 | 0.03162 | 2.16 |
| Linoleic acid (C18:cis[9,12]2) | up | 1.20 | 0.03269 | 2.15 |
| TAG (C18:2,C18:3) | up | 1.25 | 0.03558 | 2.11 |
| Uridine | down | 0.85 | 0.03662 | -2.10 |
| Carnitine | down | 0.87 | 0.03667 | -2.10 |
| Cholestenol No 02 | up | 1.14 | 0.03972 | 2.07 |
| Methionine | down | 0.85 | 0.04546 | -2.01 |
| Cholesta-1,3,5-triene | up | 1.18 | 0.04598 | 2.01 |
| TAG (C18:1 ,C18:2) | up | 1.17 | 0.04661 | 2.00 |
| N,N-Dimethylarginine (ADMA) | down | 0.89 | 0.04892 | -1.98 |
| Cholesterol, total | up | 1.12 | 0.05248 | 1.95 |
| Citrate | down | 0.84 | 0.05345 | -1.94 |
| Sphingomyelin (d18:1,C24:0) | up | 1.10 | 0.05405 | 1.93 |
| Cholesta-2,4,6-triene | up | 1.13 | 0.05513 | 1.93 |
| Phosphatidylcholine (C18:0,C20:4) | up | 1.09 | 0.05565 | 1.92 |
| Lignoceric acid (C24:0) | up | 1.15 | 0.05632 | 1.92 |
| Glycerol phosphate, lipid fraction | up | 1.18 | 0.05662 | 1.91 |

(continued)

| Metabolite | Direction of change | ANOVA result of pancreatic carcinoma relative to non-cancer group (blood donors, chronic pancreatitis and liver cirrhosis) | | |
|---|---|---|---|---|
| | | Estimated fold change | p-value | t-value |
| Palmitoleic acid (C16:cis[9]1) | down | 0.82 | 0.05742 | -1.91 |
| Taurine | up | 1.17 | 0.06588 | 1.85 |
| 3-Hydroxybutyrate | down | 0.66 | 0.06755 | -1.83 |
| TAG (C16:0,C18:1,C18:2) | up | 1.22 | 0.06941 | 1.82 |
| Quinic acid | down | 0.71 | 0.06943 | -1.82 |
| Methionine sulfoxide | up | 1.41 | 0.07795 | 1.77 |
| Octadecanoylcarnitine | down | 0.89 | 0.08186 | -1.75 |
| Arginine | up | 1.14 | 0.08205 | 1.74 |
| Dehydroepiandrosterone sulfate | up | 1.29 | 0.08210 | 1.74 |
| Glycochenodeoxycholic acid | down | 0.67 | 0.08647 | -1.72 |
| Isopalmitic acid (C16:0) | up | 1.16 | 0.09486 | 1.68 |
| Isoleucine | up | 1.12 | 0.09611 | 1.67 |
| Furoylglycine | down | 0.63 | 0.09655 | -1.67 |
| Mannosamine | up | 1.30 | 0.09902 | 1.65 |
| alpha-Tocopherol | up | 1.16 | 0.09961 | 1.65 |

**Table 4b:** List of identified biomarkers from lipid analysis for pancreatic cancer in comparison relative to non-cancer group (blood donors, chronic pancreatitis and liver cirrhosis). For abbreviations of lipid analysis fractions, refer to Table 2b.

| Metabolite | Direction of change | ANOVA result of pancreatic carcinoma relative to non-cancer group (blood donors, chronic pancreatitis and liver cirrhosis) | | |
|---|---|---|---|---|
| | | Estimated fold change | p-value | t-value |
| SM_Sphingomyelin (d18:1,C19:0) | up | 1.42 | 0.00000 | 6.47 |
| SM_Sphingomyelin (d18:2,C18:1) | up | 1.70 | 0.00000 | 6.40 |
| SM_Sphingomyelin (d18:2,C22:1) | up | 1.46 | 0.00000 | 6.27 |
| SM_Sphingomyelin (d18:2,C20:1) | up | 1.63 | 0.00000 | 6.20 |
| SM_Sphingomyelin (d18:2,C20:0) | up | 1.49 | 0.00000 | 6.14 |
| SM_Sphingomyelin (d18:2,C19:0) | up | 1.62 | 0.00000 | 6.03 |
| SM_Sphingomyelin (d18:2,C18:0) | up | 1.76 | 0.00000 | 5.96 |

(continued)

| Metabolite | Direction of change | ANOVA result of pancreatic carcinoma relative to non-cancer group (blood donors, chronic pancreatitis and liver cirrhosis) | | |
| --- | --- | --- | --- | --- |
| | | Estimated fold change | p-value | t-value |
| CER_Ceramide (d18:2,C20:0) | up | 1.82 | 0.00000 | 5.96 |
| SM_Sphingomyelin (d18:1,C20:0) | up | 1.48 | 0.00000 | 5.80 |
| CER_Ceramide (d18:1,C20:0) | up | 1.73 | 0.00000 | 5.72 |
| SM_Sphingomyelin (d18:2,C22:0) | up | 1.60 | 0.00000 | 5.61 |
| SM_Sphingomyelin (d17:1,C18:0) | up | 1.57 | 0.00000 | 5.52 |
| CER_Ceramide (d18:2,C18:0) | up | 1.80 | 0.00000 | 5.48 |
| SM_Sphingomyelin (d17:1,C20:0) | up | 1.53 | 0.00000 | 5.42 |
| CER_Ceramide (d18:1,C18:0) | up | 1.77 | 0.00000 | 5.35 |
| SM_Sphingomyelin (d18:2,C21:0) | up | 1.50 | 0.00000 | 5.29 |
| SM_Sphingomyelin (d18:2,C24:2) | up | 1.33 | 0.00000 | 5.26 |
| SM_Sphingomyelin (d18:2,C23:1) | up | 1.41 | 0.00000 | 5.25 |
| SM_Sphingomyelin (d18:1,C21:0) | up | 1.37 | 0.00000 | 5.18 |
| CER_Ceramide (d18:1,C21:0) | up | 1.61 | 0.00000 | 5.08 |
| CER_Ceramide (d18:2,C22:0) | up | 1.58 | 0.00000 | 4.92 |
| SM_Sphingomyelin (d18:2,C24:1) | up | 1.52 | 0.00000 | 4.68 |
| CER_Ceramide (d16:1,C18:0) | up | 1.64 | 0.00000 | 4.65 |
| SM_Sphingomyelin (d18:1,C22:0) | up | 1.43 | 0.00001 | 4.55 |
| CER_Ceramide (d17:1,C22:0) | up | 1.54 | 0.00001 | 4.53 |
| CER_Ceramide (d18:1,C22:0) | up | 1.39 | 0.00001 | 4.50 |
| SM_Sphingomyelin (d17:1,C22:0) | up | 1.39 | 0.00001 | 4.48 |
| CER_Ceramide (d17:1,C16:0) | up | 1.45 | 0.00001 | 4.43 |

(continued)

| Metabolite | Direction of change | ANOVA result of pancreatic carcinoma relative to non-cancer group (blood donors, chronic pancreatitis and liver cirrhosis) | | |
| --- | --- | --- | --- | --- |
| | | Estimated fold change | p-value | t-value |
| SM_Sphingomyelin (d17: 1,C24:1) | up | 1.28 | 0.00002 | 4.36 |
| CER_Ceramide (d16:1,C20: 0) | up | 1.53 | 0.00002 | 4.34 |
| CER_Ceramide (d17:1,C24: 1) | up | 1.43 | 0.00002 | 4.30 |
| SM_Sphingomyelin (d16: 1,C20:0) | up | 1.57 | 0.00003 | 4.24 |
| CER_Ceramide (d18:1,C22: 1) | up | 1.53 | 0.00007 | 4.03 |
| CER_Ceramide (d18:2,C16: 0) | up | 1.37 | 0.00009 | 3.99 |
| SM_Sphingomyelin (d16: 1,C18:1) | up | 1.41 | 0.00009 | 3.98 |
| SM_Sphingomyelin (d18: 2,C16:0) | up | 1.36 | 0.00013 | 3.88 |
| FFA_Palmitoleic acid (C16: cis[9]1) | down | 0.58 | 0.00015 | -3.84 |
| CER_Ceramide (d18:2,C24: 1) | up | 1.39 | 0.00016 | 3.82 |
| CER_Ceramide (d18:1,C23: 1) | up | 1.40 | 0.00027 | 3.68 |
| CER_Ceramide (d18:1,C24: 2) | up | 1.37 | 0.00036 | 3.61 |
| SM_Sphingomyelin (d18: 2,C24:0) | up | 1.23 | 0.00038 | 3.60 |
| SM_Sphingomyelin (d17: 1,C16:0) | up | 1.32 | 0.00038 | 3.59 |
| SM_Sphingomyelin (d16: 1,C18:0) | up | 1.30 | 0.00087 | 3.36 |
| SM_Sphingomyelin (d16: 1,C22:1) | up | 1.27 | 0.00090 | 3.35 |
| CER_Ceramide (d18:1,C24: 1) | up | 1.26 | 0.00094 | 3.34 |
| SM_Sphingomyelin (d18: 2,C23:0) | up | 1.26 | 0.00095 | 3.34 |
| CER_Ceramide (d18:2,C24: 0) | up | 1.38 | 0.00141 | 3.22 |
| CE_Cholesterylester C20:4 | up | 1.35 | 0.00271 | 3.02 |
| CER_Ceramide (d18:1,C16: 0) | up | 1.28 | 0.00273 | 3.02 |

(continued)

| Metabolite | Direction of change | ANOVA result of pancreatic carcinoma relative to non-cancer group (blood donors, chronic pancreatitis and liver cirrhosis) | | |
|---|---|---|---|---|
| | | Estimated fold change | p-value | t-value |
| TAG_gamma-Linolenic acid (C18:cis[6,9,12]3) | up | 1.82 | 0.00314 | 2.98 |
| CER_Ceramide (d16:1,C22:0) | up | 1.35 | 0.00316 | 2.98 |
| Sphingosine-1-phosphate (d17:1) | up | 1.40 | 0.00330 | 2.97 |
| TAG_Arachidonic acid (C20:cis[5,8,11,14]4) | up | 1.49 | 0.00389 | 2.91 |
| CER_Ceramide (d18:2,C23:0) | up | 1.36 | 0.00390 | 2.91 |
| SM_Sphingomyelin (d18:2,C14:0) | up | 1.23 | 0.00461 | 2.86 |
| SM_Sphingomyelin (d17:1,C24:0) | up | 1.23 | 0.00502 | 2.83 |
| CER_Ceramide (d18:1,C14:0) | up | 1.24 | 0.00560 | 2.79 |
| TAG_Linoleic acid (C18:cis[9,12]2) | up | 1.43 | 0.00613 | 2.76 |
| SM_Sphingomyelin (d17:1,C23:0) | up | 1.35 | 0.00686 | 2.72 |
| CER_Ceramide (d16:1,C16:0) | up | 1.23 | 0.00816 | 2.66 |
| CE_Cholesterylester C22:6 | up | 1.31 | 0.00974 | 2.60 |
| FS_Cholesterol, free | up | 1.19 | 0.01104 | 2.56 |
| PC_Arachidonic acid (C20:cis[5,8,11,14]4) | up | 1.31 | 0.01279 | 2.51 |
| PE_Docosahexaenoic acid (C22:cis[4,7,10,13,16,19]6) | up | 1.38 | 0.01427 | 2.47 |
| PC_Palmitic acid (C16:0) | up | 1.19 | 0.01488 | 2.45 |
| CER_Ceramide (d18:1,C24:0) | up | 1.18 | 0.01503 | 2.45 |
| SM_Sphingomyelin (d16:1,C22:0) | up | 1.25 | 0.01518 | 2.44 |
| CER_Ceramide (d16:1,C24:1) | up | 1.22 | 0.01811 | 2.38 |
| FFA_cis-Vaccenic acid | down | 0.72 | 0.01903 | -2.36 |
| CER_Ceramide (d17:1,C24:0) | up | 1.28 | 0.01929 | 2.35 |
| SM_Sphingomyelin (d18:1,C16:0) | up | 1.06 | 0.02097 | 2.32 |

(continued)

| Metabolite | Direction of change | ANOVA result of pancreatic carcinoma relative to non-cancer group (blood donors, chronic pancreatitis and liver cirrhosis) | | |
|---|---|---|---|---|
| | | Estimated fold change | p-value | t-value |
| PE_Arachidonic acid (C20: cis[5,8,11,14]4) | up | 1.29 | 0.02242 | 2.30 |
| PC_conjugated Linoleic acid (C18:cis[9]trans[11]2) | up | 1.66 | 0.02257 | 2.29 |
| SM_Sphingomyelin (d18: 1,C23:0) | up | 1.12 | 0.02326 | 2.28 |
| SM_Sphingomyelin (d16: 1,C21:0) | up | 1.24 | 0.02336 | 2.28 |
| SM_Sphingomyelin (d18: 1,C24:0) | up | 1.11 | 0.02410 | 2.27 |
| TAG_Hexadecenoic acid (C16:trans[9]1) | up | 1.51 | 0.02746 | 2.22 |
| PC_dihomo-gamma-Linolenic acid (C20:cis [8,11,14]3) | up | 1.25 | 0.03002 | 2.18 |
| PC_Stearic acid (C18:0) | up | 1.15 | 0.03337 | 2.14 |
| Sphingadienine-1-phosphate (d18:2) | up | 1.27 | 0.03389 | 2.13 |
| PI_Arachidonic acid (C20: cis[5,8,11,14]4) | up | 1.26 | 0.03429 | 2.13 |
| CER_Ceramide (d18:1,C23: 0) | up | 1.18 | 0.03517 | 2.12 |
| TAG_dihomo-gamma-Linolenic acid (C20:cis [8,11,14]3) | up | 1.52 | 0.03869 | 2.08 |
| FFA_Palmitic acid (C16:0) | down | 0.83 | 0.03943 | -2.07 |
| SM_Sphingomyelin (d16: 1,C24:1) | up | 1.15 | 0.03947 | 2.07 |
| PE_Stearic acid (C18:0) | up | 1.21 | 0.04116 | 2.05 |
| SM_Sphingomyelin (d18: 1,C14:0) | up | 1.16 | 0.04187 | 2.04 |
| DAG_Palmitic acid (C16:0) | down | 0.61 | 0.04809 | -1.99 |
| CE_Cholesterylester C16:1 | down | 0.83 | 0.04869 | -1.98 |
| LPC_Arachidonic acid (C20: cis[5,8,11,14]4) | up | 1.56 | 0.05164 | 1.95 |
| CER_Ceramide (d17:1,C23: 0) | up | 1.23 | 0.05520 | 1.93 |
| CE_Cholesterylester C14:1 | down | 0.82 | 0.08528 | -1.73 |
| PC_Linoleic acid (C18:cis [9,12]2) | up | 1.18 | 0.09492 | 1.68 |

(continued)

| Metabolite | Direction of change | ANOVA result of pancreatic carcinoma relative to non-cancer group (blood donors, chronic pancreatitis and liver cirrhosis) | | |
| --- | --- | --- | --- | --- |
| | | Estimated fold change | p-value | t-value |
| FFA_Myristoleic acid (C14: cis[9]1) | down | 0.66 | 0.09529 | -1.67 |
| FFA_Oleic acid (C18:cis[9]1) | down | 0.82 | 0.09792 | -1.66 |

Example 3: Analysis of serum metabolites

Serum samples were obtained from the patients described in the aforementioned Examples 1. The samples were pre-treated, analyzed by MS as described in Example 1 and statistically evaluated essentially as described Examples 1 and 2, above.

The estimated fold changes, t- and p-values found for the metabolite biomarkers in the serum samples were essentially as described in any one of Tables 2a, 2b, 3a, 3b, 4a or 4b and the estimated fold change, direction of change (i.e. up or down regulation) and the significance values could be, therefore, essentially confirmed for another sample type.

Example 4: Analysis and evaluation of further plasma samples

Additionally plasma samples from a second centre were collected from 80 pancreatic adenocarcinoma patients and 80 patients suffering from chronic pancreatitis. Patients were matched for age, gender and BMI. Plasma was prepared and analysed by MS as described in example 1 and statistically evaluated as described in Table 5. Abbreviations of lipid classes are as described in Table 2b.

**Table 5:** Additional identification strategies of biomarker candidates from the matrix plasma or the mixed matrix plasma-serum

| Statistical parameters of ANOVA model | | |
| --- | --- | --- |
| Estimated fold change | Fold change ratio of pancreatic carcinoma relative to blood donors (PAC 1), critical controls (liver cirrhosis and pancreatitis, PAC 2), chronic pancreatitis (PAC 2A) or liver cirrhosis (PAC 2B), estimated from ANOVA on log 10-transformed ratios with age, BMI, storage and gender as fixed effects ANOVA models applied on data to generate results shown in Tables 21-32 included additionally "center" as fixed effect. | |
| t-value | t-value of ANOVA as described for the "estimated fold change", t-values give relative change in units of standard deviation. Negative t-values indicate decreases, positive indicate increases | |
| p-value | p-value of ANOVA as described for the "estimated fold change", calculated from t-value by taking degrees of freedom and two-sided test into consideration | |

**Table 6:** List of all identified biomarkers in plasma of center 2 for pancreatic cancer relative to pancreatitis (PAC 2A)

| Metabolite | Direction of change | ANOVA result of pancreatic cancer relative to pancreatitis (plasma) | | |
| --- | --- | --- | --- | --- |
| | | Estimated fold change | p-value | t-value |
| Cholesterylester C18:4 | down | 0.41 | 0.00002 | -4.40 |
| alpha-Ketoglutarate | up | 1.82 | 0.00003 | 4.32 |
| Lysophosphatidylcholine (C18:2) | down | 0.66 | 0.00007 | -4.09 |

(continued)

| Metabolite | Direction of change | ANOVA result of pancreatic cancer relative to pancreatitis (plasma) | | |
|---|---|---|---|---|
| | | Estimated fold change | p-value | t-value |
| Alanine | down | 0.76 | 0.00019 | -3.83 |
| Lysophosphatidylethanolamine (C22:5) | down | 0.77 | 0.00028 | -3.72 |
| Glycocholic acid | up | 6.15 | 0.00035 | 3.66 |
| Ceramide (d16:1,C24:0) | down | 0.61 | 0.00039 | -3.63 |
| Sorbitol | down | 0.55 | 0.00040 | -3.63 |
| Cholesterylester C18:3 | down | 0.72 | 0.00042 | -3.60 |
| Propionylcarnitine | down | 0.69 | 0.00053 | -3.54 |
| Cholesterylester C14:0 | down | 0.74 | 0.00064 | -3.49 |
| Taurochenodeoxycholic acid | up | 5.08 | 0.00098 | 3.36 |
| Pipecolic acid | down | 0.74 | 0.00109 | -3.33 |
| Cholesterylester C20:5 | down | 0.70 | 0.00154 | -3.22 |
| Glutamine | down | 0.74 | 0.00245 | -3.08 |
| Phosphatidylcholine (C16:0,C22:6) | up | 1.07 | 0.00255 | 3.07 |
| LPC_Linoleic acid (C18:cis[9,12]2) | down | 0.59 | 0.00358 | -2.96 |
| TAG_Eicosanoic acid (C20:0) | down | 0.46 | 0.00425 | -2.90 |
| Cholesterylester C12:0 | down | 0.57 | 0.00533 | -2.83 |
| Cholesterylester C16:2 | down | 0.74 | 0.00561 | -2.81 |
| Eicosapentaenoic acid (C20:cis [5,8,11,14,17]5) | down | 0.68 | 0.00648 | -2.76 |
| Cholesterylester C20:3 | down | 0.79 | 0.00803 | -2.69 |
| Biliverdin | up | 1.69 | 0.00815 | 2.68 |
| Lysophosphatidylcholine (C18:1) | down | 0.88 | 0.00925 | -2.64 |
| LPC_Oleic acid (C18:cis[9]1) | down | 0.44 | 0.01217 | -2.54 |
| Cholesterylester C18:0 | down | 0.76 | 0.01493 | -2.46 |
| Lysophosphatidylcholine (C20:4) | down | 0.84 | 0.01637 | -2.43 |
| 5-Oxoproline | down | 0.90 | 0.01660 | -2.42 |
| Ceramide (d16:1,C23:0) | down | 0.72 | 0.02356 | -2.29 |
| Sphingomyelin (d18:0,C16:0) | up | 1.23 | 0.02497 | 2.26 |
| Sphinganine-1-phosphate (d18:0) | down | 0.79 | 0.02587 | -2.25 |
| LPC_Palmitic acid (C16:0) | down | 0.72 | 0.03048 | -2.18 |
| Phosphatidylcholine (C16:0,C20:5) | down | 0.90 | 0.03238 | -2.16 |
| PE_Docosapentaenoic acid (C22:cis [7,10,13,16,19]5) | down | 0.50 | 0.03359 | -2.14 |
| PC_gamma-Linolenic acid (C18:cis[6,9,12] 3) | down | 0.52 | 0.03828 | -2.09 |
| Cholesterylester C22:5 | down | 0.87 | 0.03897 | -2.08 |

(continued)

| Metabolite | Direction of change | ANOVA result of pancreatic cancer relative to pancreatitis (plasma) | | |
|---|---|---|---|---|
| | | Estimated fold change | p-value | t-value |
| LPC_Stearic acid (C18:0) | down | 0.80 | 0.04535 | -2.02 |
| Glycine | down | 0.88 | 0.04614 | -2.01 |
| Glycerate | down | 0.86 | 0.05974 | -1.90 |
| PC_Eicosapentaenoic acid (C20:cis [5,8,11,14,17]5) | down | 0.78 | 0.06030 | -1.89 |
| Valine | down | 0.89 | 0.07659 | -1.78 |
| Erucic acid (C22:cis[13]1) | up | 1.10 | 0.09018 | 1.71 |
| Indole-3-lactic acid | down | 0.85 | 0.09824 | -1.66 |
| Leucine | down | 0.87 | 0.09946 | -1.66 |
| Choline | down | 0.89 | 0.10621 | -1.63 |
| Glycolate | down | 0.90 | 0.10882 | -1.61 |
| Lysophosphatidylcholine (C18:0) | down | 0.88 | 0.11067 | -1.60 |
| Kynurenine | up | 1.16 | 0.11598 | 1.58 |
| Sphingomyelin (d18:1,C16:0) | up | 1.06 | 0.11812 | 1.57 |
| Tryptophan | down | 0.86 | 0.12938 | -1.52 |
| Sphingomyelin (d16:1,C24:0) | down | 0.84 | 0.13019 | -1.52 |
| Carnosine | down | 0.61 | 0.14021 | -1.49 |
| TAG_Myristoleic acid (C14:cis[9]1) | down | 0.62 | 0.14311 | -1.47 |
| Kynurenic acid | up | 1.29 | 0.14554 | 1.46 |
| Ketoleucine | down | 0.83 | 0.14665 | -1.46 |
| 2-Methylserine | down | 0.89 | 0.14788 | -1.45 |
| PE_Linoleic acid (C18:cis[9,12]2) | down | 0.86 | 0.14813 | -1.45 |
| Lysine | down | 0.92 | 0.18402 | -1.33 |
| 5-Hydroxy-3-indoleacetic acid (5-HIAA) | up | 1.17 | 0.19486 | 1.30 |

**Table 7:** List of identified up regulated biomarkers in plasma of center 2 for pancreatic cancer relative to pancreatitis

| Metabolite | Direction of change | ANOVA result of pancreatic cancer relative to pancreatitis (plasma) | | |
|---|---|---|---|---|
| | | Estimated fold change | p-value | t-value |
| alpha-Ketoglutarate | up | 1.82 | 0.00003 | 4.32 |
| Glycocholic acid | up | 6.15 | 0.00035 | 3.66 |
| Taurochenodeoxycholic acid | up | 5.08 | 0.00098 | 3.36 |
| Phosphatidylcholine (C16:0,C22:6) | up | 1.07 | 0.00255 | 3.07 |
| Biliverdin | up | 1.69 | 0.00815 | 2.68 |
| Sphingomyelin (d18:0,C16:0) | up | 1.23 | 0.02497 | 2.26 |

(continued)

| Metabolite | Direction of change | ANOVA result of pancreatic cancer relative to pancreatitis (plasma) | | |
| --- | --- | --- | --- | --- |
| | | Estimated fold change | p-value | t-value |
| Erucic acid (C22:cis[13]1) | up | 1.10 | 0.09018 | 1.71 |
| Kynurenine | up | 1.16 | 0.11598 | 1.58 |
| Sphingomyelin (d18:1,C16:0) | up | 1.06 | 0.11812 | 1.57 |
| Kynurenic acid | up | 1.29 | 0.14554 | 1.46 |
| 5-Hydroxy-3-indoleacetic acid (5-HIAA) | up | 1.17 | 0.19486 | 1.30 |

**Table 8:** List of identified down regulated biomarkers in plasma of center 2 for pancreatic cancer relative to pancreatitis

| Metabolite | Direction of change | ANOVA result of pancreatic cancer relative to pancreatitis (plasma) | | |
| --- | --- | --- | --- | --- |
| | | Estimated fold change | p-value | t-value |
| Cholesterylester C18:4 | down | 0.41 | 0.00002 | -4.40 |
| Lysophosphatidylcholine (C18:2) | down | 0.66 | 0.00007 | -4.09 |
| Alanine | down | 0.76 | 0.00019 | -3.83 |
| Lysophosphatidylethanolamine (C22:5) | down | 0.77 | 0.00028 | -3.72 |
| Ceramide (d16:1,C24:0) | down | 0.61 | 0.00039 | -3.63 |
| Sorbitol | down | 0.55 | 0.00040 | -3.63 |
| Cholesterylester C18:3 | down | 0.72 | 0.00042 | -3.60 |
| Propionylcarnitine | down | 0.69 | 0.00053 | -3.54 |
| Cholesterylester C14:0 | down | 0.74 | 0.00064 | -3.49 |
| Pipecolic acid | down | 0.74 | 0.00109 | -3.33 |
| Cholesterylester C20:5 | down | 0.70 | 0.00154 | -3.22 |
| Glutamine | down | 0.74 | 0.00245 | -3.08 |
| LPC_Linoleic acid (C18:cis[9,12]2) | down | 0.59 | 0.00358 | -2.96 |
| TAG_Eicosanoic acid (C20:0) | down | 0.46 | 0.00425 | -2.90 |
| Cholesterylester C12:0 | down | 0.57 | 0.00533 | -2.83 |
| Cholesterylester C16:2 | down | 0.74 | 0.00561 | -2.81 |
| Eicosapentaenoic acid (C20:cis [5,8,11,14,17]5) | down | 0.68 | 0.00648 | -2.76 |
| Cholesterylester C20:3 | down | 0.79 | 0.00803 | -2.69 |
| Lysophosphatidylcholine (C18:1) | down | 0.88 | 0.00925 | -2.64 |
| LPC_Oleic acid (C18:cis[9]1) | down | 0.44 | 0.01217 | -2.54 |
| Cholesterylester C18:0 | down | 0.76 | 0.01493 | -2.46 |
| Lysophosphatidylcholine (C20:4) | down | 0.84 | 0.01637 | -2.43 |
| 5-Oxoproline | down | 0.90 | 0.01660 | -2.42 |

(continued)

| Metabolite | Direction of change | ANOVA result of pancreatic cancer relative to pancreatitis (plasma) | | |
| | | Estimated fold change | p-value | t-value |
|---|---|---|---|---|
| Ceramide (d16:1,C23:0) | down | 0.72 | 0.02356 | -2.29 |
| Sphinganine-1-phosphate (d18:0) | down | 0.79 | 0.02587 | -2.25 |
| LPC_Palmitic acid (C16:0) | down | 0.72 | 0.03048 | -2.18 |
| Phosphatidylcholine (C16:0,C20:5) | down | 0.90 | 0.03238 | -2.16 |
| PE_Docosapentaenoic acid (C22:cis [7,10,13,16,19]5) | down | 0.50 | 0.03359 | -2.14 |
| PC_gamma-Linolenic acid (C18:cis[6,9,12] 3) | down | 0.52 | 0.03828 | -2.09 |
| Cholesterylester C22:5 | down | 0.87 | 0.03897 | -2.08 |
| LPC_Stearic acid (C18:0) | down | 0.80 | 0.04535 | -2.02 |
| Glycine | down | 0.88 | 0.04614 | -2.01 |
| Glycerate | down | 0.86 | 0.05974 | -1.90 |
| PC_Eicosapentaenoic acid (C20:cis [5,8,11,14,17]5) | down | 0.78 | 0.06030 | -1.89 |
| Valine | down | 0.89 | 0.07659 | -1.78 |
| Indole-3-lactic acid | down | 0.85 | 0.09824 | -1.66 |
| Leucine | down | 0.87 | 0.09946 | -1.66 |
| Choline | down | 0.89 | 0.10621 | -1.63 |
| Glycolate | down | 0.90 | 0.10882 | -1.61 |
| Lysophosphatidylcholine (C18:0) | down | 0.88 | 0.11067 | -1.60 |
| Tryptophan | down | 0.86 | 0.12938 | -1.52 |
| Sphingomyelin (d16:1,C24:0) | down | 0.84 | 0.13019 | -1.52 |
| Carnosine | down | 0.61 | 0.14021 | -1.49 |
| TAG_Myristoleic acid (C14:cis[9]1) | down | 0.62 | 0.14311 | -1.47 |
| Ketoleucine | down | 0.83 | 0.14665 | -1.46 |
| 2-Methylserine | down | 0.89 | 0.14788 | -1.45 |
| PE_Linoleic acid (C18:cis[9,12]2) | down | 0.86 | 0.14813 | -1.45 |
| Lysine | down | 0.92 | 0.18402 | -1.33 |

**Table 9:** List of all identified biomarkers in serum for pancreatic cancer relative to healthy volunteers (blood donors (PAC 1)

| Metabolite | Direction of change | ANOVA result of pancreatic cancer relative to blood donors (serum) | | |
| | | Estimated fold change | p-value | t-value |
|---|---|---|---|---|
| Cholesterylester C22:4 | up | 2.28 | 0.000000007 | 6.17 |
| Cholesterylester C22:5 | up | 1.77 | 0.00002 | 4.41 |

(continued)

| Metabolite | Direction of change | ANOVA result of pancreatic cancer relative to blood donors (serum) | | |
|---|---|---|---|---|
| | | Estimated fold change | p-value | t-value |
| PC_Docosapentaenoic acid (C22:cis [4,7,10,13,16]5) | up | 5.46 | 0.00003 | 4.30 |
| Cholesterylester C20:3 | up | 1.85 | 0.00007 | 4.09 |
| Fructose | up | 3.37 | 0.00015 | 3.89 |
| 2-Methylserine | up | 1.57 | 0.00016 | 3.87 |
| Hippuric acid | down | 0.28 | 0.00096 | -3.37 |
| TAG_Docosapentaenoic acid (C22:cis [4,7,10,13,16]5) | up | 5.06 | 0.00103 | 3.35 |
| PC_Oleic acid (C18:cis[9]1) | up | 1.80 | 0.00105 | 3.35 |
| Tryptophan | up | 1.55 | 0.00117 | 3.31 |
| Pregnenolone sulfate | up | 2.51 | 0.00128 | 3.28 |
| Sphingosine-1-phosphate (d16:1) | up | 2.97 | 0.00138 | 3.29 |
| Docosapentaenoic acid (C22:cis [7,10,13,16,19]5) | up | 1.65 | 0.00161 | 3.21 |
| Galactonic acid | up | 2.17 | 0.00193 | 3.17 |
| Cholesterylester C20:2 | up | 1.44 | 0.00213 | 3.13 |
| Sphingomyelin (d18:0,C16:0) | up | 1.54 | 0.00244 | 3.09 |
| FFA_Docosapentaenoic acid (C22:cis [7,10,13,16,19]5) | up | 4.45 | 0.00274 | 3.05 |
| LPC_Palmitic acid (C16:0) | up | 1.53 | 0.00330 | 2.99 |
| Cholic acid | up | 6.70 | 0.00344 | 2.98 |
| PC_Palmitoleic acid (C16:cis[9]1) | up | 2.30 | 0.00432 | 2.90 |
| Cholesterylester C16:0 | up | 1.41 | 0.00501 | 2.85 |
| Cholesterylester C16:2 | up | 1.50 | 0.01098 | 2.58 |
| Succinate | up | 1.40 | 0.01116 | 2.57 |
| TAG_Docosapentaenoic acid (C22:cis [7,10,13,16,19]5) | up | 3.30 | 0.01147 | 2.56 |
| Glycocholic acid | up | 5.12 | 0.01313 | 2.51 |
| PI_Oleic acid (C18:cis[9]1) | up | 2.99 | 0.01416 | 2.49 |
| Ceramide (d18:1,C24:0) | up | 1.56 | 0.01460 | 2.47 |
| Sphingomyelin (d18:1,C16:0) | up | 1.18 | 0.01483 | 2.46 |
| LPC_Stearic acid (C18:0) | up | 1.43 | 0.01678 | 2.42 |
| FFA_Docosahexaenoic acid (C22:cis [4,7,10,13,16,19]6) | up | 2.26 | 0.01731 | 2.41 |
| Cholesterylester C18:3 | up | 1.46 | 0.02156 | 2.33 |
| FFA_Eicosenoic acid (C20:cis[11]1) | up | 2.93 | 0.02789 | 2.22 |
| Kynurenic acid | up | 1.97 | 0.02902 | 2.21 |

(continued)

| Metabolite | Direction of change | ANOVA result of pancreatic cancer relative to blood donors (serum) | | |
| --- | --- | --- | --- | --- |
| | | Estimated fold change | p-value | t-value |
| Lysophosphatidylcholine (C18:0) | up | 1.28 | 0.03372 | 2.14 |
| Lysophosphatidylcholine (C20:4) | up | 1.24 | 0.03800 | 2.09 |
| Biliverdin | up | 1.63 | 0.04156 | 2.06 |
| TAG_Docosahexaenoic acid (C22:cis [4,7,10,13,16,19]6) | up | 2.85 | 0.04507 | 2.02 |
| PC_Eicosenoic acid (C20:cis[11]1) | up | 2.19 | 0.04666 | 2.01 |
| Lysophosphatidylcholine (C18:2) | down | 0.75 | 0.04847 | -1.99 |
| Stearic acid (C18:0) | up | 1.40 | 0.04893 | 1.99 |
| TAG_Palmitoleic acid (C16:cis[9]1) | up | 1.67 | 0.05722 | 1.92 |
| Taurochenodeoxycholic acid | up | 2.93 | 0.06746 | 1.84 |
| Kynurenine | up | 1.25 | 0.07061 | 1.82 |
| Heptadecenoic acid (C17:cis[10]1) | up | 1.38 | 0.07317 | 1.80 |
| Glucose-1-phosphate | down | 0.58 | 0.07424 | -1.80 |
| LPC_Oleic acid (C18:cis[9]1) | up | 1.59 | 0.08184 | 1.75 |
| 5-Hydroxy-3-indoleacetic acid (5-HIAA) | up | 1.39 | 0.08376 | 1.74 |
| Cholesterylester C18:2 | up | 1.26 | 0.08377 | 1.74 |
| Lysophosphatidylcholine (C18:1) | up | 1.20 | 0.08784 | 1.72 |
| TAG_Eicosadienoic acid (C20:cis[11,14]2) | up | 2.08 | 0.09012 | 1.71 |
| Lysophosphatidylcholine (C16:0) | up | 1.13 | 0.09367 | 1.69 |
| Proline betaine | up | 2.01 | 0.10504 | 1.63 |
| PC_gamma-Linolenic acid (C18:cis[6,9,12]3) | up | 2.17 | 0.11216 | 1.60 |
| Glucosamine | up | 1.64 | 0.12655 | 1.54 |
| TAG_Eicosapentaenoic acid (C20:cis [5,8,11,14,17]5) | up | 2.21 | 0.13027 | 1.52 |
| LPC_Linoleic acid (C18:cis[9,12]2) | down | 0.56 | 0.14564 | -1.46 |
| Cholesterylester C15:0 | up | 1.27 | 0.14612 | 1.46 |
| PE_Oleic acid (C18:cis[9]1) | up | 1.77 | 0.16835 | 1.39 |
| Phosphatidylcholine (C16:0,C22:6) | up | 1.14 | 0.16876 | 1.38 |
| Erucic acid (C22:cis[13]1) | up | 1.11 | 0.18298 | 1.34 |
| DAG_Oleic acid (C18:cis[9]1) | up | 2.55 | 0.19062 | 1.32 |
| Lysophosphatidylcholine (C17:0) | up | 1.24 | 0.19156 | 1.31 |
| Phosphatidylcholine (C18:1,C18:2) | up | 1.08 | 0.19555 | 1.30 |
| Sphingomyelin (d16:1,C24:0) | up | 1.29 | 0.19802 | 1.29 |
| Trimethylamine-N-oxide (TMAO) | down | 0.57 | 0.19802 | -1.29 |

**Table 10:** List of identified up regulated biomarkers in serum for pancreatic cancer relative to healthy volunteers (blood donors)

| Metabolite | Direction of change | ANOVA result of pancreatic cancer relative to blood donors (serum) | | |
|---|---|---|---|---|
| | | Estimated fold change | p-value | t-value |
| Cholesterylester C22:4 | up | 2.28 | 0.000000007 | 6.17 |
| Cholesterylester C22:5 | up | 1.77 | 0.00002 | 4.41 |
| PC_Docosapentaenoic acid (C22:cis [4,7,10,13,16]5) | up | 5.46 | 0.00003 | 4.30 |
| Cholesterylester C20:3 | up | 1.85 | 0.00007 | 4.09 |
| Fructose | up | 3.37 | 0.00015 | 3.89 |
| 2-Methylserine | up | 1.57 | 0.00016 | 3.87 |
| TAG_Docosapentaenoic acid (C22:cis [4,7,10,13,16]5) | up | 5.06 | 0.00103 | 3.35 |
| PC_Oleic acid (C18:cis[9]1) | up | 1.80 | 0.00105 | 3.35 |
| Tryptophan | up | 1.55 | 0.00117 | 3.31 |
| Pregnenolone sulfate | up | 2.51 | 0.00128 | 3.28 |
| Sphingosine-1-phosphate (d16:1) | up | 2.97 | 0.00138 | 3.29 |
| Docosapentaenoic acid (C22:cis [7,10,13,16,19]5) | up | 1.65 | 0.00161 | 3.21 |
| Galactonic acid | up | 2.17 | 0.00193 | 3.17 |
| Cholesterylester C20:2 | up | 1.44 | 0.00213 | 3.13 |
| Sphingomyelin (d18:0,C16:0) | up | 1.54 | 0.00244 | 3.09 |
| FFA_Docosapentaenoic acid (C22:cis [7,10,13,16,19]5) | up | 4.45 | 0.00274 | 3.05 |
| LPC_Palmitic acid (C16:0) | up | 1.53 | 0.00330 | 2.99 |
| Cholic acid | up | 6.70 | 0.00344 | 2.98 |
| PC_Palmitoleic acid (C16:cis[9]1) | up | 2.30 | 0.00432 | 2.90 |
| Cholesterylester C16:0 | up | 1.41 | 0.00501 | 2.85 |
| Cholesterylester C16:2 | up | 1.50 | 0.01098 | 2.58 |
| Succinate | up | 1.40 | 0.01116 | 2.57 |
| TAG_Docosapentaenoic acid (C22:cis [7,10,13,16,19]5) | up | 3.30 | 0.01147 | 2.56 |
| Glycocholic acid | up | 5.12 | 0.01313 | 2.51 |
| PI_Oleic acid (C18:cis[9]1) | up | 2.99 | 0.01416 | 2.49 |
| Ceramide (d18:1,C24:0) | up | 1.56 | 0.01460 | 2.47 |
| Sphingomyelin (d18:1,C16:0) | up | 1.18 | 0.01483 | 2.46 |
| LPC_Stearic acid (C18:0) | up | 1.43 | 0.01678 | 2.42 |
| FFA_Docosahexaenoic acid (C22:cis [4,7,10,13,16,19]6) | up | 2.26 | 0.01731 | 2.41 |
| Cholesterylester C18:3 | up | 1.46 | 0.02156 | 2.33 |

(continued)

| Metabolite | Direction of change | ANOVA result of pancreatic cancer relative to blood donors (serum) | | |
|---|---|---|---|---|
| | | Estimated fold change | p-value | t-value |
| FFA_Eicosenoic acid (C20:cis[11]1) | up | 2.93 | 0.02789 | 2.22 |
| Kynurenic acid | up | 1.97 | 0.02902 | 2.21 |
| Lysophosphatidylcholine (C18:0) | up | 1.28 | 0.03372 | 2.14 |
| Lysophosphatidylcholine (C20:4) | up | 1.24 | 0.03800 | 2.09 |
| Biliverdin | up | 1.63 | 0.04156 | 2.06 |
| TAG_Docosahexaenoic acid (C22:cis [4,7,10,13,16,19]6) | up | 2.85 | 0.04507 | 2.02 |
| PC_Eicosenoic acid (C20:cis[11]1) | up | 2.19 | 0.04666 | 2.01 |
| Stearic acid (C18:0) | up | 1.40 | 0.04893 | 1.99 |
| TAG_Palmitoleic acid (C16:cis[9]1) | up | 1.67 | 0.05722 | 1.92 |
| Taurochenodeoxycholic acid | up | 2.93 | 0.06746 | 1.84 |
| Kynurenine | up | 1.25 | 0.07061 | 1.82 |
| Heptadecenoic acid (C17:cis[10]1) | up | 1.38 | 0.07317 | 1.80 |
| LPC_Oleic acid (C18:cis[9]1) | up | 1.59 | 0.08184 | 1.75 |
| 5-Hydroxy-3-indoleacetic acid (5-HIAA) | up | 1.39 | 0.08376 | 1.74 |
| Cholesterylester C18:2 | up | 1.26 | 0.08377 | 1.74 |
| Lysophosphatidylcholine (C18:1) | up | 1.20 | 0.08784 | 1.72 |
| TAG_Eicosadienoic acid (C20:cis[11,14]2) | up | 2.08 | 0.09012 | 1.71 |
| Lysophosphatidylcholine (C16:0) | up | 1.13 | 0.09367 | 1.69 |
| Proline betaine | up | 2.01 | 0.10504 | 1.63 |
| PC_gamma-Linolenic acid (C18:cis[6,9,12]3) | up | 2.17 | 0.11216 | 1.60 |
| Glucosamine | up | 1.64 | 0.12655 | 1.54 |
| TAG_Eicosapentaenoic acid (C20:cis [5,8,11,14,17]5) | up | 2.21 | 0.13027 | 1.52 |
| Cholesterylester C15:0 | up | 1.27 | 0.14612 | 1.46 |
| PE_Oleic acid (C18:cis[9]1) | up | 1.77 | 0.16835 | 1.39 |
| Phosphatidylcholine (C16:0,C22:6) | up | 1.14 | 0.16876 | 1.38 |
| Erucic acid (C22:cis[13]1) | up | 1.11 | 0.18298 | 1.34 |
| DAG_Oleic acid (C18:cis[9]1) | up | 2.55 | 0.19062 | 1.32 |
| Lysophosphatidylcholine (C17:0) | up | 1.24 | 0.19156 | 1.31 |
| Phosphatidylcholine (C18:1,C18:2) | up | 1.08 | 0.19555 | 1.30 |
| Sphingomyelin (d16:1,C24:0) | up | 1.29 | 0.19802 | 1.29 |

**Table 11:** List of identified down regulated biomarkers in serum for pancreatic cancer relative to healthy volunteers (blood donors)

| Metabolite | Direction of change | ANOVA result of pancreatic cancer relative to blood donors (serum) | | |
| --- | --- | --- | --- | --- |
| | | Estimated fold change | p-value | t-value |
| Hippuric acid | down | 0.28 | 0.00096 | -3.37 |
| Lysophosphatidylcholine (C18:2) | down | 0.75 | 0.04847 | -1.99 |
| Glucose-1-phosphate | down | 0.58 | 0.07424 | -1.80 |
| LPC_Linoleic acid (C18:cis[9,12]2) | down | 0.56 | 0.14564 | -1.46 |
| Trimethylamine-N-oxide (TMAO) | down | 0.57 | 0.19802 | -1.29 |

**Table 12:** List of all identified biomarkers in serum for pancreatic cancer relative to critical controls (PAC 2)

| Metabolite | Direction of change | ANOVA result of pancreatic cancer relative to critical controls (serum) | | |
| --- | --- | --- | --- | --- |
| | | Estimated fold change | p-value | t-value |
| PC_Docosapentaenoic acid (C22:cis [4,7,10,13,16]5) | up | 1.41 | 0.05554 | 1.92 |
| Phosphatidylcholine (C16:0,C22:6) | up | 1.13 | 0.05693 | 1.91 |
| PC_gamma-Linolenic acid (C18:cis[6,9,12]3) | up | 1.53 | 0.05875 | 1.90 |
| TAG_Eicosadienoic acid (C20:cis[11,14]2) | up | 1.46 | 0.08470 | 1.73 |
| Stearic acid (C18:0) | up | 1.16 | 0.08750 | 1.72 |
| TAG_Linolenic acid (C18:cis[9,12,15]3) | up | 1.28 | 0.12187 | 1.55 |
| PC_Oleic acid (C18:cis[9]1) | up | 1.14 | 0.12474 | 1.54 |
| PC_Myristic acid (C14:0) | up | 1.44 | 0.12855 | 1.53 |
| Ribose | down | 0.71 | 0.13184 | -1.51 |
| Fumarate | down | 0.86 | 0.13883 | -1.49 |
| FFA_Docosahexaenoic acid (C22:cis [4,7,10,13,16,19]6) | up | 1.35 | 0.14935 | 1.45 |
| Lactate | down | 0.83 | 0.15111 | -1.44 |
| Sphingomyelin (d18:1,C16:0) | down | 0.95 | 0.15276 | -1.43 |
| Malate | down | 0.83 | 0.15532 | -1.43 |
| 2-Methylserine | down | 0.92 | 0.16129 | -1.41 |
| Ceramide (d16:1,C23:0) | up | 1.21 | 0.16215 | 1.40 |
| TAG_Docosapentaenoic acid (C22:cis [4,7,10,13,16]5) | up | 1.38 | 0.17157 | 1.37 |
| Hypoxanthine | down | 0.75 | 0.17685 | -1.35 |
| Glucosamine | up | 1.26 | 0.18317 | 1.34 |

(continued)

| Metabolite | Direction of change | ANOVA result of pancreatic cancer relative to critical controls (serum) | | |
| --- | --- | --- | --- | --- |
| | | Estimated fold change | p-value | t-value |
| Glycolate | down | 0.93 | 0.18443 | -1.33 |
| Cholesterylester C20:3 | up | 1.15 | 0.18688 | 1.32 |
| Glycine | down | 0.92 | 0.18849 | -1.32 |
| Sphingomyelin (d16:1,C24:0) | up | 1.17 | 0.18883 | 1.32 |
| Glutamine | down | 0.76 | 0.19760 | -1.29 |

**Table 13:** List of identified up regulated biomarkers in serum for pancreatic cancer relative to critical controls

| Metabolite | Direction of change | ANOVA result of pancreatic cancer relative to critical controls (serum) | | |
| --- | --- | --- | --- | --- |
| | | Estimated fold change | p-value | t-value |
| PC_Docosapentaenoic acid (C22:cis [4,7,10,13,16]5) | up | 1.41 | 0.05554 | 1.92 |
| Phosphatidylcholine (C16:0,C22:6) | up | 1.13 | 0.05693 | 1.91 |
| PC_gamma-Linolenic acid (C18:cis[6,9,12]3) | up | 1.53 | 0.05875 | 1.90 |
| TAG_Eicosadienoic acid (C20:cis[11,14]2) | up | 1.46 | 0.08470 | 1.73 |
| Stearic acid (C18:0) | up | 1.16 | 0.08750 | 1.72 |
| TAG_Linolenic acid (C18:cis[9,12,15]3) | up | 1.28 | 0.12187 | 1.55 |
| PC_Oleic acid (C18:cis[9]1) | up | 1.14 | 0.12474 | 1.54 |
| PC_Myristic acid (C14:0) | up | 1.44 | 0.12855 | 1.53 |
| FFA_Docosahexaenoic acid (C22:cis [4,7,10,13,16,19]6) | up | 1.35 | 0.14935 | 1.45 |
| Ceramide (d16:1,C23:0) | up | 1.21 | 0.16215 | 1.40 |
| TAG_Docosapentaenoic acid (C22:cis [4,7,10,13,16]5) | up | 1.38 | 0.17157 | 1.37 |
| Glucosamine | up | 1.26 | 0.18317 | 1.34 |
| Cholesterylester C20:3 | up | 1.15 | 0.18688 | 1.32 |
| Sphingomyelin (d16:1,C24:0) | up | 1.17 | 0.18883 | 1.32 |

**Table 14:** List of identified down regulated biomarkers in serum for pancreatic cancer relative to critical controls

| Metabolite | Direction of change | ANOVA result of pancreatic cancer relative to critical controls (serum) | | |
| --- | --- | --- | --- | --- |
| | | Estimated fold change | p-value | t-value |
| Ribose | down | 0.71 | 0.13184 | -1.51 |
| Fumarate | down | 0.86 | 0.13883 | -1.49 |

(continued)

| Metabolite | Direction of change | ANOVA result of pancreatic cancer relative to critical controls (serum) | | |
| --- | --- | --- | --- | --- |
| | | Estimated fold change | p-value | t-value |
| Lactate | down | 0.83 | 0.15111 | -1.44 |
| Sphingomyelin (d18:1,C16:0) | down | 0.95 | 0.15276 | -1.43 |
| Malate | down | 0.83 | 0.15532 | -1.43 |
| 2-Methylserine | down | 0.92 | 0.16129 | -1.41 |
| Hypoxanthine | down | 0.75 | 0.17685 | -1.35 |
| Glycolate | down | 0.93 | 0.18443 | -1.33 |
| Glycine | down | 0.92 | 0.18849 | -1.32 |
| Glutamine | down | 0.76 | 0.19760 | -1.29 |

**Table 15:** List of all identified biomarkers in serum for pancreatic cancer relative to pancreatitis (PAC 2A)

| Metabolite | Direction of change | ANOVA result of pancreatic cancer relative to pancreatitis (serum) | | |
| --- | --- | --- | --- | --- |
| | | Estimated fold change | p-value | t-value |
| LPC_Oleic acid (C18:cis[9]1) | down | 0.70 | 0.01238 | -2.53 |
| TAG_Myristoleic acid (C14:cis[9]1) | down | 0.46 | 0.01477 | -2.47 |
| Lysophosphatidylcholine (C18:1) | down | 0.83 | 0.01824 | -2.39 |
| Glycine | down | 0.81 | 0.02851 | -2.21 |
| Sphinganine-1-phosphate (d18:0) | down | 0.74 | 0.03116 | -2.18 |
| Cholesterylester C12:0 | down | 0.68 | 0.03163 | -2.17 |
| Pregnenolone sulfate | down | 0.69 | 0.03192 | -2.17 |
| LPC_Stearic acid (C18:0) | down | 0.78 | 0.03646 | -2.11 |
| Cholesterylester C14:0 | down | 0.82 | 0.04501 | -2.02 |
| 5-Oxoproline | down | 0.81 | 0.04726 | -2.00 |
| PC_Eicosapentaenoic acid (C20:cis [5,8,11,14,17]5) | down | 0.74 | 0.04742 | -2.00 |
| Eicosapentaenoic acid (C20:cis [5,8,11,14,17]5) | down | 0.73 | 0.04857 | -1.99 |
| LPC_Linoleic acid (C18:cis[9,12]2) | down | 0.57 | 0.05111 | -1.97 |
| TAG_Palmitoleic acid (C16:cis[9]1) | down | 0.69 | 0.05277 | -1.95 |
| Carnosine | down | 0.67 | 0.05400 | -1.94 |
| LPC_Palmitic acid (C16:0) | down | 0.80 | 0.05501 | -1.93 |
| Phosphatidylcholine (C16:0,C20:5) | down | 0.85 | 0.05529 | -1.93 |
| Cresol sulfate | down | 0.46 | 0.05564 | -1.93 |
| Ceramide (d16:1,C24:0) | down | 0.78 | 0.06239 | -1.88 |
| Cholesterylester C18:4 | down | 0.70 | 0.07185 | -1.81 |

(continued)

| Metabolite | Direction of change | ANOVA result of pancreatic cancer relative to pancreatitis (serum) | | |
|---|---|---|---|---|
| | | Estimated fold change | p-value | t-value |
| Alanine | down | 0.83 | 0.07558 | -1.79 |
| Lysophosphatidylethanolamine (C22:5) | down | 0.89 | 0.08150 | -1.75 |
| Serine | down | 0.86 | 0.08156 | -1.75 |
| Cholesterylester C16:2 | down | 0.81 | 0.08514 | -1.73 |
| Cryptoxanthin | up | 1.50 | 0.08678 | 1.72 |
| Lysophosphatidylcholine (C18:0) | down | 0.87 | 0.09267 | -1.69 |
| Cholesterylester C20:5 | down | 0.79 | 0.09787 | -1.67 |
| Cholesterylester C18:0 | down | 0.84 | 0.12381 | -1.55 |
| Fumarate | down | 0.80 | 0.13082 | -1.52 |
| Lysophosphatidylcholine (C20:4) | down | 0.89 | 0.13517 | -1.50 |
| Sphingomyelin (d16:1,C24:0) | down | 0.82 | 0.13698 | -1.50 |
| beta-Carotene | up | 1.41 | 0.13710 | 1.49 |
| Lactate | down | 0.77 | 0.14211 | -1.48 |
| Leucine | down | 0.86 | 0.14553 | -1.46 |
| Glucose-1-phosphate | up | 1.47 | 0.15784 | 1.42 |
| Docosapentaenoic acid (C22:cis [7,10,13,16,19]5) | down | 0.83 | 0.16804 | -1.39 |
| PE_Docosapentaenoic acid (C22:cis [7,10,13,16,19]5) | down | 0.69 | 0.17591 | -1.36 |
| Cholesterylester C16:0 | down | 0.89 | 0.18249 | -1.34 |

**Table 16:** List of identified up regulated biomarkers in serum for pancreatic cancer relative to pancreatitis

| Metabolite | Direction of change | ANOVA result of pancreatic cancer relative to pancreatitis (serum) | | |
|---|---|---|---|---|
| | | Estimated fold change | p-value | t-value |
| Cryptoxanthin | up | 1.50 | 0.08678 | 1.72 |
| beta-Carotene | up | 1.41 | 0.13710 | 1.49 |
| Glucose-1-phosphate | up | 1.47 | 0.15784 | 1.42 |

**Table 17:** List of identified down regulated biomarkers in serum for pancreatic cancer relative to pancreatitis

| Metabolite | Direction of change | ANOVA result of pancreatic cancer relative to pancreatitis (serum) | | |
|---|---|---|---|---|
| | | Estimated fold change | p-value | t-value |
| LPC_Oleic acid (C18:cis[9]1) | down | 0.70 | 0.01238 | -2.53 |

(continued)

| Metabolite | Direction of change | ANOVA result of pancreatic cancer relative to pancreatitis (serum) | | |
| --- | --- | --- | --- | --- |
| | | Estimated fold change | p-value | t-value |
| TAG_Myristoleic acid (C14:cis[9]1) | down | 0.46 | 0.01477 | -2.47 |
| Lysophosphatidylcholine (C18:1) | down | 0.83 | 0.01824 | -2.39 |
| Glycine | down | 0.81 | 0.02851 | -2.21 |
| Sphinganine-1-phosphate (d18:0) | down | 0.74 | 0.03116 | -2.18 |
| Cholesterylester C12:0 | down | 0.68 | 0.03163 | -2.17 |
| Pregnenolone sulfate | down | 0.69 | 0.03192 | -2.17 |
| LPC_Stearic acid (C18:0) | down | 0.78 | 0.03646 | -2.11 |
| Cholesterylester C14:0 | down | 0.82 | 0.04501 | -2.02 |
| 5-Oxoproline | down | 0.81 | 0.04726 | -2.00 |
| PC_Eicosapentaenoic acid (C20:cis [5,8,11,14,17]5) | down | 0.74 | 0.04742 | -2.00 |
| Eicosapentaenoic acid (C20:cis [5,8,11,14,17]5) | down | 0.73 | 0.04857 | -1.99 |
| LPC_Linoleic acid (C18:cis[9,12]2) | down | 0.57 | 0.05111 | -1.97 |
| TAG_Palmitoleic acid (C16:cis[9]1) | down | 0.69 | 0.05277 | -1.95 |
| Carnosine | down | 0.67 | 0.05400 | -1.94 |
| LPC_Palmitic acid (C16:0) | down | 0.80 | 0.05501 | -1.93 |
| Phosphatidylcholine (C16:0,C20:5) | down | 0.85 | 0.05529 | -1.93 |
| Cresol sulfate | down | 0.46 | 0.05564 | -1.93 |
| Ceramide (d16:1,C24:0) | down | 0.78 | 0.06239 | -1.88 |
| Cholesterylester C18:4 | down | 0.70 | 0.07185 | -1.81 |
| Alanine | down | 0.83 | 0.07558 | -1.79 |
| Lysophosphatidylethanolamine (C22:5) | down | 0.89 | 0.08150 | -1.75 |
| Serine | down | 0.86 | 0.08156 | -1.75 |
| Cholesterylester C16:2 | down | 0.81 | 0.08514 | -1.73 |
| Lysophosphatidylcholine (C18:0) | down | 0.87 | 0.09267 | -1.69 |
| Cholesterylester C20:5 | down | 0.79 | 0.09787 | -1.67 |
| Cholesterylester C18:0 | down | 0.84 | 0.12381 | -1.55 |
| Fumarate | down | 0.80 | 0.13082 | -1.52 |
| Lysophosphatidylcholine (C20:4) | down | 0.89 | 0.13517 | -1.50 |
| Sphingomyelin (d16:1,C24:0) | down | 0.82 | 0.13698 | -1.50 |
| Lactate | down | 0.77 | 0.14211 | -1.48 |
| Leucine | down | 0.86 | 0.14553 | -1.46 |
| Docosapentaenoic acid (C22:cis [7,10,13,16,19]5) | down | 0.83 | 0.16804 | -1.39 |

(continued)

| Metabolite | Direction of change | ANOVA result of pancreatic cancer relative to pancreatitis (serum) | | |
| --- | --- | --- | --- | --- |
| | | Estimated fold change | p-value | t-value |
| PE_Docosapentaenoic acid (C22:cis [7,10,13,16,19]5) | down | 0.69 | 0.17591 | -1.36 |
| Cholesterylester C16:0 | down | 0.89 | 0.18249 | -1.34 |

**Table 18:** List of all identified biomarkers in serum for pancreatic cancer relative to liver cirrhosis (PAC 2B)

| Metabolite | Direction of change | ANOVA result of pancreatic cancer relative to liver cirrhosis (serum) | | |
| --- | --- | --- | --- | --- |
| | | Estimated fold change | p-value | t-value |
| Stearic acid (C18:0) | up | 1.38 | 0.00067 | 3.47 |
| PC_gamma-Linolenic acid (C18:cis[6,9,12]3) | up | 2.16 | 0.00164 | 3.20 |
| Cholesterylester C20:5 | up | 1.62 | 0.00175 | 3.19 |
| PC_Eicosapentaenoic acid (C20:cis [5,8,11,14,17]5) | up | 1.74 | 0.00186 | 3.17 |
| PC_Docosapentaenoic acid (C22:cis [4,7,10,13,16]5) | up | 1.91 | 0.00226 | 3.11 |
| Ceramide (d18:1,C24:0) | up | 1.43 | 0.00236 | 3.09 |
| Cholesterylester C20:3 | up | 1.46 | 0.00330 | 2.99 |
| Sphingomyelin (d16:1,C24:0) | up | 1.49 | 0.00334 | 2.98 |
| Eicosapentaenoic acid (C20:cis[5,8,11,14,17]5) | up | 1.51 | 0.00384 | 2.94 |
| Ceramide (d16:1,C24:0) | up | 1.49 | 0.00392 | 2.93 |
| TAG_Linolenic acid (C18:cis[9,12,15]3) | up | 1.63 | 0.00446 | 2.89 |
| Cholesterylester C18:3 | up | 1.42 | 0.00482 | 2.86 |
| Ceramide (d16:1,C23:0) | up | 1.53 | 0.00632 | 2.77 |
| Phosphatidylcholine (C16:0,C22:6) | up | 1.24 | 0.00636 | 2.77 |
| LPC_Linoleic acid (C18:cis[9,12]2) | up | 2.37 | 0.00700 | 2.73 |
| PC_Oleic acid (C18:cis[9]1) | up | 1.28 | 0.00773 | 2.70 |
| FFA_Docosahexaenoic acid (C22:cis [4,7,10,13,16,19]6) | up | 1.81 | 0.00792 | 2.69 |
| PC_Linolenic acid (C18:cis[9,12,15]3) | up | 1.88 | 0.00812 | 2.68 |
| Docosapentaenoic acid (C22:cis [7,10,13,16,19]5) | up | 1.33 | 0.00913 | 2.64 |
| TAG_Eicosadienoic acid (C20:cis[11,14]2) | up | 1.86 | 0.01270 | 2.52 |
| Lysophosphatidylcholine (C20:4) | up | 1.19 | 0.01474 | 2.47 |
| TAG_Eicosapentaenoic acid (C20:cis [5,8,11,14,17]5) | up | 2.07 | 0.01495 | 2.46 |
| Lysophosphatidylcholine (C18:2) | up | 1.28 | 0.01580 | 2.44 |

(continued)

| Metabolite | Direction of change | ANOVA result of pancreatic cancer relative to liver cirrhosis (serum) | | |
| --- | --- | --- | --- | --- |
| | | Estimated fold change | p-value | t-value |
| Carnosine | up | 1.54 | 0.01592 | 2.44 |
| Heptadecenoic acid (C17:cis[10]1) | up | 1.24 | 0.01689 | 2.42 |
| FFA_Eicosapentaenoic acid (C20:cis [5,8,11,14,17]5) | up | 1.99 | 0.01705 | 2.41 |
| PC_Myristic acid (C14:0) | up | 1.96 | 0.01840 | 2.38 |
| 14-Methylhexadecanoic acid | up | 1.27 | 0.01954 | 2.36 |
| TAG_Docosapentaenoic acid (C22:cis [4,7,10,13,16]5) | up | 1.86 | 0.02131 | 2.33 |
| Kynurenine | down | 0.82 | 0.02257 | -2.30 |
| Phosphatidylcholine (C16:0,C20:5) | up | 1.17 | 0.02428 | 2.28 |
| Lysophosphatidylcholine (C18:0) | up | 1.22 | 0.02431 | 2.27 |
| TAG_conjugated Linoleic acid (C18:cis[9] trans[11]2) | up | 1.72 | 0.02560 | 2.25 |
| Ribose | down | 0.57 | 0.02577 | -2.25 |
| Cholesterylester C18:2 | up | 1.29 | 0.02623 | 2.24 |
| Testosterone | up | 1.54 | 0.02680 | 2.24 |
| TAG_Docosapentaenoic acid (C22:cis [7,10,13,16,19]5) | up | 1.86 | 0.02948 | 2.20 |
| Lysophosphatidylcholine (C16:0) | up | 1.11 | 0.03088 | 2.18 |
| Cholesterylester C16:2 | up | 1.25 | 0.03189 | 2.17 |
| LPC_Palmitic acid (C16:0) | up | 1.28 | 0.03333 | 2.15 |
| Cholesterylester C18:2 | up | 1.19 | 0.03457 | 2.13 |
| Myristic acid (C14:0) | up | 1.29 | 0.03653 | 2.11 |
| Docosatetraenoic acid (C22:cis[7,10,13,16]4) | up | 1.19 | 0.04034 | 2.07 |
| TAG_Myristic acid (C14:0) | up | 1.51 | 0.04361 | 2.03 |
| Taurochenodeoxycholic acid | down | 0.47 | 0.04388 | -2.03 |
| Cholesterylester C22:5 | up | 1.21 | 0.04727 | 2.00 |
| PC_Myristoleic acid (C14:cis[9]1) | up | 1.66 | 0.05242 | 1.95 |
| TAG_Eicosanoic acid (C20:0) | up | 1.61 | 0.05258 | 1.95 |
| LPC_Stearic acid (C18:0) | up | 1.23 | 0.05580 | 1.93 |
| Cholesterylester C18:4 | up | 1.37 | 0.05787 | 1.91 |
| Cholesterylester C16:0 | up | 1.19 | 0.06247 | 1.88 |
| FFA_Docosapentaenoic acid (C22:cis [7,10,13,16,19]5) | up | 1.69 | 0.06500 | 1.86 |
| TAG_Docosahexaenoic acid (C22:cis [4,7,10,13,16,19]6) | up | 1.76 | 0.06865 | 1.83 |
| PE_Oleic acid (C18:cis[9]1) | up | 1.43 | 0.07002 | 1.82 |

(continued)

| Metabolite | Direction of change | ANOVA result of pancreatic cancer relative to liver cirrhosis (serum) | | |
| --- | --- | --- | --- | --- |
| | | Estimated fold change | p-value | t-value |
| Sphingomyelin (d16:1,C23:0) | up | 1.24 | 0.07046 | 1.82 |
| Sphingosine-1-phosphate (d16:1) | up | 1.28 | 0.07368 | 1.80 |
| Lauric acid (C12:0) | up | 1.30 | 0.07606 | 1.79 |
| myo-Inositol, lipid fraction | up | 1.17 | 0.08053 | 1.76 |
| 5-Hydroxy-3-indoleacetic acid (5-HIAA) | down | 0.84 | 0.08180 | -1.75 |
| Phosphatidylcholine (C18:1,C18:2) | up | 1.06 | 0.09267 | 1.69 |
| Pipecolic acid | down | 0.88 | 0.11401 | -1.59 |
| Lysophosphatidylethanolamine (C22:5) | up | 1.11 | 0.11498 | 1.59 |
| Lysophosphatidylcholine (C17:0) | up | 1.18 | 0.11517 | 1.58 |
| DAG_Oleic acid (C18:cis[9]1) | up | 1.79 | 0.12216 | 1.55 |
| Cholesterylester C15:0 | up | 1.16 | 0.12227 | 1.55 |
| Lysophosphatidylcholine (C18:1) | up | 1.11 | 0.12679 | 1.54 |
| PC_Eicosatrienoic acid (C20:[11,14,17]3) | up | 1.58 | 0.13618 | 1.50 |
| Leucine | up | 1.11 | 0.14514 | 1.46 |
| 1-Methyladenosine | down | 0.91 | 0.14557 | -1.46 |
| Cholesterylester C20:1 | down | 0.85 | 0.15720 | -1.42 |
| Erucic acid (C22:cis[13]1) | up | 1.06 | 0.16005 | 1.41 |
| Valine | up | 1.10 | 0.16236 | 1.40 |
| Sphinganine-1-phosphate (d18:0) | up | 1.19 | 0.16256 | 1.40 |
| PI_Myristic acid (C14:0) | up | 1.75 | 0.17323 | 1.37 |
| Phosphatidylcholine (C16:1,C18:2) | up | 1.11 | 0.17360 | 1.37 |
| PI_Myristoleic acid (C14:cis[9]1) | up | 1.57 | 0.18259 | 1.34 |
| PS_Myristoleic acid (C14:cis[9]1) | up | 1.83 | 0.18320 | 1.34 |
| Biliverdin | down | 0.82 | 0.19005 | -1.32 |
| 2-Methylserine | down | 0.92 | 0.19235 | -1.31 |
| Sphingomyelin (d18:0,C16:0) | up | 1.11 | 0.19448 | 1.30 |
| Sphingomyelin (d18:1,C16:0) | down | 0.95 | 0.19464 | -1.30 |
| Eicosenoic acid (C20:cis[11]1) | up | 1.11 | 0.19801 | 1.29 |
| Cholic acid | up | 1.52 | 0.19824 | 1.29 |

**Table 19:** List of identified up regulated biomarkers in serum for pancreatic cancer relative to liver cirrhosis

| Metabolite | Direction of change | ANOVA result of pancreatic cancer relative to liver cirrhosis (serum) | | |
| --- | --- | --- | --- | --- |
| | | Estimated fold change | p-value | t-value |
| Stearic acid (C18:0) | up | 1.38 | 0.00067 | 3.47 |

(continued)

| Metabolite | Direction of change | ANOVA result of pancreatic cancer relative to liver cirrhosis (serum) | | |
| --- | --- | --- | --- | --- |
| | | Estimated fold change | p-value | t-value |
| PC_gamma-Linolenic acid (C18:cis[6,9,12]3) | up | 2.16 | 0.00164 | 3.20 |
| Cholesterylester C20:5 | up | 1.62 | 0.00175 | 3.19 |
| PC_Eicosapentaenoic acid (C20:cis [5,8,11,14,17]5) | up | 1.74 | 0.00186 | 3.17 |
| PC_Docosapentaenoic acid (C22:cis [4,7,10,13,16]5) | up | 1.91 | 0.00226 | 3.11 |
| Ceramide (d18:1,C24:0) | up | 1.43 | 0.00236 | 3.09 |
| Cholesterylester C20:3 | up | 1.46 | 0.00330 | 2.99 |
| Sphingomyelin (d16:1,C24:0) | up | 1.49 | 0.00334 | 2.98 |
| Eicosapentaenoic acid (C20:cis[5,8,11,14,17]5) | up | 1.51 | 0.00384 | 2.94 |
| Ceramide (d16:1,C24:0) | up | 1.49 | 0.00392 | 2.93 |
| TAG_Linolenic acid (C18:cis[9,12,15]3) | up | 1.63 | 0.00446 | 2.89 |
| Cholesterylester C18:3 | up | 1.42 | 0.00482 | 2.86 |
| Ceramide (d16:1,C23:0) | up | 1.53 | 0.00632 | 2.77 |
| Phosphatidylcholine (C16:0,C22:6) | up | 1.24 | 0.00636 | 2.77 |
| LPC_Linoleic acid (C18:cis[9,12]2) | up | 2.37 | 0.00700 | 2.73 |
| PC_Oleic acid (C18:cis[9]1) | up | 1.28 | 0.00773 | 2.70 |
| FFA_Docosahexaenoic acid (C22:cis [4,7,10,13,16,19]6) | up | 1.81 | 0.00792 | 2.69 |
| PC_Linolenic acid (C18:cis[9,12,15]3) | up | 1.88 | 0.00812 | 2.68 |
| Docosapentaenoic acid (C22:cis [7,10,13,16,19]5) | up | 1.33 | 0.00913 | 2.64 |
| TAG_Eicosadienoic acid (C20:cis[11,14]2) | up | 1.86 | 0.01270 | 2.52 |
| Lysophosphatidylcholine (C20:4) | up | 1.19 | 0.01474 | 2.47 |
| TAG_Eicosapentaenoic acid (C20:cis [5,8,11,14,17]5) | up | 2.07 | 0.01495 | 2.46 |
| Lysophosphatidylcholine (C18:2) | up | 1.28 | 0.01580 | 2.44 |
| Carnosine | up | 1.54 | 0.01592 | 2.44 |
| Heptadecenoic acid (C17:cis[10]1) | up | 1.24 | 0.01689 | 2.42 |
| FFA_Eicosapentaenoic acid (C20:cis [5,8,11,14,17]5) | up | 1.99 | 0.01705 | 2.41 |
| PC_Myristic acid (C14:0) | up | 1.96 | 0.01840 | 2.38 |
| 14-Methylhexadecanoic acid | up | 1.27 | 0.01954 | 2.36 |
| TAG_Docosapentaenoic acid (C22:cis [4,7,10,13,16]5) | up | 1.86 | 0.02131 | 2.33 |
| Phosphatidylcholine (C16:0,C20:5) | up | 1.17 | 0.02428 | 2.28 |
| Lysophosphatidylcholine (C18:0) | up | 1.22 | 0.02431 | 2.27 |

(continued)

| Metabolite | Direction of change | ANOVA result of pancreatic cancer relative to liver cirrhosis (serum) | | |
| --- | --- | --- | --- | --- |
| | | Estimated fold change | p-value | t-value |
| TAG_conjugated Linoleic acid (C18:cis[9] trans[11]2) | up | 1.72 | 0.02560 | 2.25 |
| Cholesterylester C18:2 | up | 1.29 | 0.02623 | 2.24 |
| Testosterone | up | 1.54 | 0.02680 | 2.24 |
| TAG_Docosapentaenoic acid (C22:cis [7,10,13,16,19]5) | up | 1.86 | 0.02948 | 2.20 |
| Lysophosphatidylcholine (C16:0) | up | 1.11 | 0.03088 | 2.18 |
| Cholesterylester C16:2 | up | 1.25 | 0.03189 | 2.17 |
| LPC_Palmitic acid (C16:0) | up | 1.28 | 0.03333 | 2.15 |
| Cholesterylester C18:2 | up | 1.19 | 0.03457 | 2.13 |
| Myristic acid (C14:0) | up | 1.29 | 0.03653 | 2.11 |
| Docosatetraenoic acid (C22:cis[7,10,13,16]4) | up | 1.19 | 0.04034 | 2.07 |
| TAG_Myristic acid (C14:0) | up | 1.51 | 0.04361 | 2.03 |
| Cholesterylester C22:5 | up | 1.21 | 0.04727 | 2.00 |
| PC_Myristoleic acid (C14:cis[9]1) | up | 1.66 | 0.05242 | 1.95 |
| TAG_Eicosanoic acid (C20:0) | up | 1.61 | 0.05258 | 1.95 |
| LPC_Stearic acid (C18:0) | up | 1.23 | 0.05580 | 1.93 |
| Cholesterylester C18:4 | up | 1.37 | 0.05787 | 1.91 |
| Cholesterylester C16:0 | up | 1.19 | 0.06247 | 1.88 |
| FFA_Docosapentaenoic acid (C22:cis [7,10,13,16,19]5) | up | 1.69 | 0.06500 | 1.86 |
| TAG_Docosahexaenoic acid (C22:cis [4,7,10,13,16,19]6) | up | 1.76 | 0.06865 | 1.83 |
| PE_Oleic acid (C18:cis[9]1) | up | 1.43 | 0.07002 | 1.82 |
| Sphingomyelin (d16:1,C23:0) | up | 1.24 | 0.07046 | 1.82 |
| Sphingosine-1-phosphate (d16:1) | up | 1.28 | 0.07368 | 1.80 |
| Lauric acid (C12:0) | up | 1.30 | 0.07606 | 1.79 |
| myo-Inositol, lipid fraction | up | 1.17 | 0.08053 | 1.76 |
| Phosphatidylcholine (C18:1,C18:2) | up | 1.06 | 0.09267 | 1.69 |
| Lysophosphatidylethanolamine (C22:5) | up | 1.11 | 0.11498 | 1.59 |
| Lysophosphatidylcholine (C17:0) | up | 1.18 | 0.11517 | 1.58 |
| DAG_Oleic acid (C18:cis[9]1) | up | 1.79 | 0.12216 | 1.55 |
| Cholesterylester C15:0 | up | 1.16 | 0.12227 | 1.55 |
| Lysophosphatidylcholine (C18:1) | up | 1.11 | 0.12679 | 1.54 |
| PC_Eicosatrienoic acid (C20:[11,14,17]3) | up | 1.58 | 0.13618 | 1.50 |
| Leucine | up | 1.11 | 0.14514 | 1.46 |

(continued)

| Metabolite | Direction of change | ANOVA result of pancreatic cancer relative to liver cirrhosis (serum) | | |
| --- | --- | --- | --- | --- |
| | | Estimated fold change | p-value | t-value |
| Erucic acid (C22:cis[13]1) | up | 1.06 | 0.16005 | 1.41 |
| Valine | up | 1.10 | 0.16236 | 1.40 |
| Sphinganine-1-phosphate (d18:0) | up | 1.19 | 0.16256 | 1.40 |
| PI_Myristic acid (C14:0) | up | 1.75 | 0.17323 | 1.37 |
| Phosphatidylcholine (C16:1,C18:2) | up | 1.11 | 0.17360 | 1.37 |
| PI_Myristoleic acid (C14:cis[9]1) | up | 1.57 | 0.18259 | 1.34 |
| PS_Myristoleic acid (C14:cis[9]1) | up | 1.83 | 0.18320 | 1.34 |
| Sphingomyelin (d18:0,C16:0) | up | 1.11 | 0.19448 | 1.30 |
| Eicosenoic acid (C20:cis[11]1) | up | 1.11 | 0.19801 | 1.29 |
| Cholic acid | up | 1.52 | 0.19824 | 1.29 |

**Table 20:** List of identified down regulated biomarkers in serum for pancreatic cancer relative to liver cirrhosis

| Metabolite | Direction of change | ANOVA result of pancreatic cancer relative to liver cirrhosis (serum) | | |
| --- | --- | --- | --- | --- |
| | | Estimated fold change | p-value | t-value |
| Kynurenine | down | 0.82 | 0.02257 | -2.30 |
| Ribose | down | 0.57 | 0.02577 | -2.25 |
| Taurochenodeoxycholic acid | down | 0.47 | 0.04388 | -2.03 |
| 5-Hydroxy-3-indoleacetic acid (5-HIAA) | down | 0.84 | 0.08180 | -1.75 |
| Pipecolic acid | down | 0.88 | 0.11401 | -1.59 |
| 1-Methyladenosine | down | 0.91 | 0.14557 | -1.46 |
| Cholesterylester C20:1 | down | 0.85 | 0.15720 | -1.42 |
| Biliverdin | down | 0.82 | 0.19005 | -1.32 |
| 2-Methylserine | down | 0.92 | 0.19235 | -1.31 |
| Sphingomyelin (d18:1,C16:0) | down | 0.95 | 0.19464 | -1.30 |

**Table 21:** List of all identified biomarkers in plasma combined with serum for pancreatic cancer relative to healthy volunteers (blood donors) (PAC 1)

| Metabolite | Direction of change | ANOVA result of pancreatic cancer relative to blood donors (plasma combined with serum) | | |
| --- | --- | --- | --- | --- |
| | | Estimated fold change | p-value | t-value |
| alpha-Ketoglutarate | up | 9.34 | 0.00000000000002 | 8.25 |
| Cholesterylester C22:4 | up | 2.22 | 0.000000000035 | 6.99 |

(continued)

| Metabolite | Direction of change | ANOVA result of pancreatic cancer relative to blood donors (plasma combined with serum) | | |
|---|---|---|---|---|
| | | Estimated fold change | p-value | t-value |
| Leucine | up | 2.05 | 0.0000000002 | 6.70 |
| Valine | up | 1.72 | 0.00000001 | 6.07 |
| Ketoleucine | up | 2.35 | 0.00000001 | 5.91 |
| Pipecolic acid | up | 2.04 | 0.00000003 | 5.72 |
| Asparagine | up | 1.72 | 0.00000014 | 5.43 |
| Docosatetraenoic acid (C22:cis [7,10,13,16]4) | up | 1.98 | 0.00000130 | 4.98 |
| 2-Methylserine | up | 1.64 | 0.00000386 | 4.73 |
| Cholesterylester C22:5 | up | 1.67 | 0.00000440 | 4.71 |
| Fructose | up | 2.98 | 0.00000441 | 4.71 |
| Sphingosine-1-phosphate (d16:1) | up | 3.13 | 0.00001 | 4.62 |
| PC_Docosapentaenoic acid (C22:cis [4,7,10,13,16]5) | up | 4.57 | 0.00002 | 4.32 |
| PC_Oleic acid (C18:cis[9]1) | up | 1.94 | 0.00004 | 4.23 |
| Succinate | up | 1.56 | 0.00005 | 4.15 |
| Sphingomyelin (d18:0,C16:0) | up | 1.70 | 0.00006 | 4.10 |
| Hippuric acid | down | 0.27 | 0.00006 | -4.08 |
| Cholesterylester C20:3 | up | 1.67 | 0.00011 | 3.93 |
| TAG_Docosapentaenoic acid (C22:cis [4,7,10,13,16]5) | up | 4.73 | 0.00031 | 3.66 |
| Galactonic acid | up | 2.30 | 0.00036 | 3.65 |
| PC_Palmitoleic acid (C16:cis[9]1) | up | 2.37 | 0.00037 | 3.62 |
| Sphingomyelin (d18:1,C16:0) | up | 1.22 | 0.00045 | 3.56 |
| Docosapentaenoic acid (C22:cis [7,10,13,16,19]5) | up | 1.60 | 0.00050 | 3.53 |
| Pregnenolone sulfate | up | 2.21 | 0.00070 | 3.44 |
| Cholesterylester C20:2 | up | 1.42 | 0.00073 | 3.43 |
| Tryptophan | up | 1.51 | 0.00083 | 3.39 |
| Cholesterylester C16:0 | up | 1.41 | 0.00088 | 3.38 |
| FFA_Docosapentaenoic acid (C22:cis [7,10,13,16,19]5) | up | 4.68 | 0.00100 | 3.34 |
| Cholic acid | up | 6.33 | 0.00126 | 3.28 |
| FFA_Eicosenoic acid (C20:cis[11]1) | up | 4.00 | 0.00129 | 3.26 |
| Ceramide (d18:1,C24:0) | up | 1.59 | 0.00144 | 3.23 |
| Kynurenic acid | up | 2.27 | 0.00148 | 3.23 |
| PI_Oleic acid (C18:cis[9]1) | up | 2.45 | 0.00523 | 2.82 |
| Lysophosphatidylcholine (C18:2) | down | 0.69 | 0.00595 | -2.78 |

(continued)

| Metabolite | Direction of change | ANOVA result of pancreatic cancer relative to blood donors (plasma combined with serum) | | |
|---|---|---|---|---|
| | | Estimated fold change | p-value | t-value |
| FFA_Docosahexaenoic acid (C22:cis [4,7,10,13,16,19]6) | up | 2.48 | 0.00622 | 2.76 |
| LPC_Stearic acid (C18:0) | up | 1.48 | 0.00707 | 2.72 |
| PC_Eicosenoic acid (C20:cis[11]1) | up | 2.22 | 0.00717 | 2.71 |
| Cholesterylester C16:2 | up | 1.43 | 0.00932 | 2.62 |
| Glycocholic acid | up | 5.42 | 0.00951 | 2.62 |
| Lysophosphatidylcholine (C18:0) | up | 1.31 | 0.01256 | 2.52 |
| Heptadecenoic acid (C17:cis[10]1) | up | 1.44 | 0.01500 | 2.45 |
| Biliverdin | up | 1.79 | 0.01527 | 2.45 |
| LPC_Palmitic acid (C16:0) | up | 1.53 | 0.01921 | 2.36 |
| Stearic acid (C18:0) | up | 1.38 | 0.02091 | 2.33 |
| 5-Hydroxy-3-indoleacetic acid (5-HIAA) | up | 1.45 | 0.02204 | 2.31 |
| TAG_Docosapentaenoic acid (C22:cis [7,10,13,16,19]5) | up | 2.53 | 0.02330 | 2.29 |
| Lysophosphatidylcholine (C18:1) | up | 1.22 | 0.02485 | 2.26 |
| Lysophosphatidylcholine (C20:4) | up | 1.23 | 0.02684 | 2.23 |
| Kynurenine | up | 1.28 | 0.02892 | 2.20 |
| Glucosamine | up | 1.78 | 0.03443 | 2.13 |
| TAG_Palmitoleic acid (C16:cis[9]1) | up | 1.60 | 0.03648 | 2.10 |
| Taurochenodeoxycholic acid | up | 3.43 | 0.04332 | 2.03 |
| Lysophosphatidylcholine (C17:0) | up | 1.32 | 0.04382 | 2.03 |
| DAG_Oleic acid (C18:cis[9]1) | up | 2.77 | 0.04832 | 1.99 |
| TAG_Docosahexaenoic acid (C22:cis [4,7,10,13,16,19]6) | up | 2.39 | 0.04997 | 1.97 |
| Cholesterylester C15:0 | up | 1.29 | 0.05953 | 1.89 |
| TAG_conjugated Linoleic acid (C18:cis [9]trans[11]2) | up | 1.84 | 0.08111 | 1.75 |
| Cholesterylester C18:2 | up | 1.22 | 0.08351 | 1.74 |
| Phosphatidylcholine (C16:0,C20:5) | down | 0.84 | 0.08774 | -1.71 |
| TAG_Eicosapentaenoic acid (C20:cis [5,8,11,14,17]5) | up | 2.09 | 0.08949 | 1.71 |
| Erucic acid (C22:cis[13]1) | up | 1.12 | 0.09898 | 1.66 |
| Cholesterylester C18:3 | up | 1.25 | 0.09957 | 1.65 |
| LPC_Linoleic acid (C18:cis[9,12]2) | down | 0.59 | 0.10137 | -1.65 |
| Lysophosphatidylcholine (C16:0) | up | 1.11 | 0.10256 | 1.64 |

(continued)

| Metabolite | Direction of change | ANOVA result of pancreatic cancer relative to blood donors (plasma combined with serum) | | |
| --- | --- | --- | --- | --- |
| | | Estimated fold change | p-value | t-value |
| TAG_Eicosadienoic acid (C20:cis [11,14]2) | up | 1.72 | 0.10731 | 1.62 |
| 14-Methylhexadecanoic acid | up | 1.31 | 0.10958 | 1.61 |
| Proline betaine | up | 1.77 | 0.11250 | 1.59 |
| Sphinganine-1-phosphate (d18:0) | up | 1.32 | 0.13098 | 1.52 |
| Phosphatidylcholine (C16:0,C22:6) | up | 1.11 | 0.14949 | 1.45 |
| Ceramide (d16:1,C24:0) | down | 0.79 | 0.15785 | -1.42 |
| Cholesterylester C12:0 | down | 0.72 | 0.16157 | -1.40 |
| Glucose-1-phosphate | down | 0.72 | 0.16783 | -1.38 |
| Indole-3-lactic acid | up | 1.16 | 0.18190 | 1.34 |
| PE_Oleic acid (C18:cis[9]1) | up | 1.75 | 0.18899 | 1.32 |
| Indole-3-acetic acid | up | 1.34 | 0.19449 | 1.30 |
| Betaine | up | 1.42 | 0.19599 | 1.30 |
| PI_Palmitic acid (C16:0) | up | 1.80 | 0.19993 | 1.29 |

**Table 22:** List of identified up regulated biomarkers in plasma combined with serum for pancreatic cancer relative to healthy volunteers (blood donors)

| Metabolite | Direction of change | ANOVA result of pancreatic cancer relative to blood donors (plasma combined with serum) | | |
| --- | --- | --- | --- | --- |
| | | Estimated fold change | p-value | t-value |
| alpha-Ketoglutarate | up | 9.34 | 0.00000000000002 | 8.25 |
| Cholesterylester C22:4 | up | 2.22 | 0.000000000035 | 6.99 |
| Leucine | up | 2.05 | 0.0000000002 | 6.70 |
| Valine | up | 1.72 | 0.00000001 | 6.07 |
| Ketoleucine | up | 2.35 | 0.00000001 | 5.91 |
| Pipecolic acid | up | 2.04 | 0.00000003 | 5.72 |
| Asparagine | up | 1.72 | 0.00000014 | 5.43 |
| Docosatetraenoic acid (C22:cis [7,10,13,16]4) | up | 1.98 | 0.00000130 | 4.98 |
| 2-Methylserine | up | 1.64 | 0.00000386 | 4.73 |
| Cholesterylester C22:5 | up | 1.67 | 0.00000440 | 4.71 |
| Fructose | up | 2.98 | 0.00000441 | 4.71 |
| Sphingosine-1-phosphate (d16:1) | up | 3.13 | 0.00001 | 4.62 |
| PC_Docosapentaenoic acid (C22:cis [4,7,10,13,16]5) | up | 4.57 | 0.00002 | 4.32 |
| PC_Oleic acid (C18:cis[9]1) | up | 1.94 | 0.00004 | 4.23 |

(continued)

| Metabolite | Direction of change | ANOVA result of pancreatic cancer relative to blood donors (plasma combined with serum) | | |
|---|---|---|---|---|
| | | Estimated fold change | p-value | t-value |
| Succinate | up | 1.56 | 0.00005 | 4.15 |
| Sphingomyelin (d18:0,C16:0) | up | 1.70 | 0.00006 | 4.10 |
| Cholesterylester C20:3 | up | 1.67 | 0.00011 | 3.93 |
| TAG_Docosapentaenoic acid (C22:cis[4,7,10,13,16]5) | up | 4.73 | 0.00031 | 3.66 |
| Galactonic acid | up | 2.30 | 0.00036 | 3.65 |
| PC_Palmitoleic acid (C16:cis[9]1) | up | 2.37 | 0.00037 | 3.62 |
| Sphingomyelin (d18:1,C16:0) | up | 1.22 | 0.00045 | 3.56 |
| Docosapentaenoic acid (C22:cis[7,10,13,16,19]5) | up | 1.60 | 0.00050 | 3.53 |
| Pregnenolone sulfate | up | 2.21 | 0.00070 | 3.44 |
| Cholesterylester C20:2 | up | 1.42 | 0.00073 | 3.43 |
| Tryptophan | up | 1.51 | 0.00083 | 3.39 |
| Cholesterylester C16:0 | up | 1.41 | 0.00088 | 3.38 |
| FFA_Docosapentaenoic acid (C22:cis[7,10,13,16,19]5) | up | 4.68 | 0.00100 | 3.34 |
| Cholic acid | up | 6.33 | 0.00126 | 3.28 |
| FFA_Eicosenoic acid (C20:cis[11]1) | up | 4.00 | 0.00129 | 3.26 |
| Ceramide (d18:1,C24:0) | up | 1.59 | 0.00144 | 3.23 |
| Kynurenic acid | up | 2.27 | 0.00148 | 3.23 |
| PI_Oleic acid (C18:cis[9]1) | up | 2.45 | 0.00523 | 2.82 |
| FFA_Docosahexaenoic acid (C22:cis[4,7,10,13,16,19]6) | up | 2.48 | 0.00622 | 2.76 |
| LPC_Stearic acid (C18:0) | up | 1.48 | 0.00707 | 2.72 |
| PC_Eicosenoic acid (C20:cis[11]1) | up | 2.22 | 0.00717 | 2.71 |
| Cholesterylester C16:2 | up | 1.43 | 0.00932 | 2.62 |
| Glycocholic acid | up | 5.42 | 0.00951 | 2.62 |
| Lysophosphatidylcholine (C18:0) | up | 1.31 | 0.01256 | 2.52 |
| Heptadecenoic acid (C17:cis[10]1) | up | 1.44 | 0.01500 | 2.45 |
| Biliverdin | up | 1.79 | 0.01527 | 2.45 |
| LPC_Palmitic acid (C16:0) | up | 1.53 | 0.01921 | 2.36 |
| Stearic acid (C18:0) | up | 1.38 | 0.02091 | 2.33 |
| 5-Hydroxy-3-indoleacetic acid (5-HIAA) | up | 1.45 | 0.02204 | 2.31 |
| TAG_Docosapentaenoic acid (C22:cis[7,10,13,16,19]5) | up | 2.53 | 0.02330 | 2.29 |
| Lysophosphatidylcholine (C18:1) | up | 1.22 | 0.02485 | 2.26 |

(continued)

| Metabolite | Direction of change | ANOVA result of pancreatic cancer relative to blood donors (plasma combined with serum) | | |
| --- | --- | --- | --- | --- |
| | | Estimated fold change | p-value | t-value |
| Lysophosphatidylcholine (C20:4) | up | 1.23 | 0.02684 | 2.23 |
| Kynurenine | up | 1.28 | 0.02892 | 2.20 |
| Glucosamine | up | 1.78 | 0.03443 | 2.13 |
| TAG_Palmitoleic acid (C16:cis[9]1) | up | 1.60 | 0.03648 | 2.10 |
| Taurochenodeoxycholic acid | up | 3.43 | 0.04332 | 2.03 |
| Lysophosphatidylcholine (C17:0) | up | 1.32 | 0.04382 | 2.03 |
| DAG_Oleic acid (C18:cis[9]1) | up | 2.77 | 0.04832 | 1.99 |
| TAG_Docosahexaenoic acid (C22:cis [4,7,10,13,16,19]6) | up | 2.39 | 0.04997 | 1.97 |
| Cholesterylester C15:0 | up | 1.29 | 0.05953 | 1.89 |
| TAG_conjugated Linoleic acid (C18:cis [9]trans[11]2) | up | 1.84 | 0.08111 | 1.75 |
| Cholesterylester C18:2 | up | 1.22 | 0.08351 | 1.74 |
| TAG_Eicosapentaenoic acid (C20:cis [5,8,11,14,17]5) | up | 2.09 | 0.08949 | 1.71 |
| Erucic acid (C22:cis[13]1) | up | 1.12 | 0.09898 | 1.66 |
| Cholesterylester C18:3 | up | 1.25 | 0.09957 | 1.65 |
| Lysophosphatidylcholine (C16:0) | up | 1.11 | 0.10256 | 1.64 |
| TAG_Eicosadienoic acid (C20:cis [11,14]2) | up | 1.72 | 0.10731 | 1.62 |
| 14-Methylhexadecanoic acid | up | 1.31 | 0.10958 | 1.61 |
| Proline betaine | up | 1.77 | 0.11250 | 1.59 |
| Sphinganine-1-phosphate (d18:0) | up | 1.32 | 0.13098 | 1.52 |
| Phosphatidylcholine (C16:0,C22:6) | up | 1.11 | 0.14949 | 1.45 |
| Indole-3-lactic acid | up | 1.16 | 0.18190 | 1.34 |
| PE_Oleic acid (C18:cis[9]1) | up | 1.75 | 0.18899 | 1.32 |
| Indole-3-acetic acid | up | 1.34 | 0.19449 | 1.30 |
| Betaine | up | 1.42 | 0.19599 | 1.30 |
| PI_Palmitic acid (C16:0) | up | 1.80 | 0.19993 | 1.29 |

**Table 23:** List of identified down regulated biomarkers in plasma combined with serum for pancreatic cancer relative to healthy volunteers (blood donors)

| Metabolite | Direction of change | ANOVA result of pancreatic cancer relative to blood donors (plasma combined with serum) | | |
| --- | --- | --- | --- | --- |
| | | Estimated fold change | p-value | t-value |
| Hippuric acid | down | 0.27 | 0.00006 | -4.08 |

(continued)

| Metabolite | Direction of change | ANOVA result of pancreatic cancer relative to blood donors (plasma combined with serum) | | |
| --- | --- | --- | --- | --- |
| | | Estimated fold change | p-value | t-value |
| Lysophosphatidylcholine (C18:2) | down | 0.69 | 0.00595 | -2.78 |
| Phosphatidylcholine (C16:0,C20:5) | down | 0.84 | 0.08774 | -1.71 |
| LPC_Linoleic acid (C18:cis [9,12]2) | down | 0.59 | 0.10137 | -1.65 |
| Ceramide (d16:1,C24:0) | down | 0.79 | 0.15785 | -1.42 |
| Cholesterylester C12:0 | down | 0.72 | 0.16157 | -1.40 |
| Glucose-1-phosphate | down | 0.72 | 0.16783 | -1.38 |

**Table 24:** List of all identified biomarkers in plasma combined with serum for pancreatic cancer relative to critical controls (PAC 2)

| Metabolite | Direction of change | ANOVA result of pancreatic cancer relative to critical controls (plasma combined with serum) | | |
| --- | --- | --- | --- | --- |
| | | Estimated fold change | p-value | t-value |
| LPC_Oleic acid (C18:cis[9]1) | down | 0.69 | 0.00546 | -2.79 |
| Cholesterylester C12:0 | down | 0.75 | 0.00748 | -2.69 |
| Cholesterylester C14:0 | down | 0.86 | 0.00881 | -2.63 |
| Alanine | down | 0.88 | 0.01017 | -2.58 |
| Cholesterylester C18:4 | down | 0.74 | 0.01394 | -2.47 |
| Glycocholic acid | up | 1.82 | 0.02287 | 2.28 |
| Glycine | down | 0.90 | 0.02482 | -2.25 |
| Stearic acid (C18:0) | up | 1.14 | 0.02804 | 2.21 |
| 2-Methylserine | down | 0.91 | 0.02992 | -2.18 |
| TAG_Eicosadienoic acid (C20:cis[11,14]2) | up | 1.35 | 0.04018 | 2.06 |
| Phosphatidylcholine (C16:0,C22:6) | up | 1.09 | 0.04165 | 2.04 |
| Asparagine | down | 0.91 | 0.04736 | -1.99 |
| Pipecolic acid | down | 0.90 | 0.05013 | -1.96 |
| Glutamine | down | 0.77 | 0.05148 | -1.95 |
| Lysophosphatidylethanolamine (C22:5) | down | 0.92 | 0.05198 | -1.95 |
| PC_Oleic acid (C18:cis[9]1) | up | 1.12 | 0.06220 | 1.87 |
| DAG_Stearic acid (C18:0) | down | 0.79 | 0.08217 | -1.74 |
| Glycerate | down | 0.88 | 0.09088 | -1.69 |
| TAG_Myristoleic acid (C14:cis[9]1) | down | 0.74 | 0.09560 | -1.67 |
| 5-Oxoproline | down | 0.92 | 0.10028 | -1.65 |

(continued)

| Metabolite | Direction of change | ANOVA result of pancreatic cancer relative to critical controls (plasma combined with serum) | | |
| --- | --- | --- | --- | --- |
| | | Estimated fold change | p-value | t-value |
| Choline | down | 0.91 | 0.10048 | -1.65 |
| Salicylic acid | down | 0.80 | 0.10848 | -1.61 |
| Cholesterylester C16:2 | down | 0.90 | 0.11472 | -1.58 |
| Sphingomyelin (d18:0,C16:0) | up | 1.08 | 0.11808 | 1.57 |
| Biliverdin | up | 1.18 | 0.12151 | 1.55 |
| TAG_Linolenic acid (C18:cis[9,12,15]3) | up | 1.17 | 0.14699 | 1.45 |
| Cholesterylester C18:0 | down | 0.91 | 0.16196 | -1.40 |
| PE_Docosapentaenoic acid (C22:cis [7,10,13,16,19]5) | down | 0.79 | 0.16219 | -1.40 |
| Ribose | down | 0.79 | 0.16452 | -1.39 |
| Fumarate | down | 0.91 | 0.16548 | -1.39 |
| Glycolate | down | 0.95 | 0.16991 | -1.38 |
| TAG_conjugated Linoleic acid (C18:cis[9] trans[11]2) | up | 1.21 | 0.17916 | 1.35 |
| TAG_Docosapentaenoic acid (C22:cis [4,7,10,13,16]5) | up | 1.26 | 0.18170 | 1.34 |
| Taurochenodeoxycholic acid | up | 1.42 | 0.18203 | 1.34 |
| PC_Eicosatrienoic acid (C20:[11,14,17]3) | up | 1.33 | 0.18356 | 1.33 |
| TAG (C16:0,C18:2) | up | 1.06 | 0.19207 | 1.31 |
| LPC_Stearic acid (C18:0) | down | 0.91 | 0.19809 | -1.29 |

**Table 25:** List of identified up regulated biomarkers in plasma combined with serum for pancreatic cancer relative to critical controls

| Metabolite | Direction of change | ANOVA result of pancreatic cancer relative to critical controls (plasma combined with serum) | | |
| --- | --- | --- | --- | --- |
| | | Estimated fold change | p-value | t-value |
| Glycocholic acid | up | 1.82 | 0.02287 | 2.28 |
| Stearic acid (C18:0) | up | 1.14 | 0.02804 | 2.21 |
| TAG_Eicosadienoic acid (C20:cis[11,14] 2) | up | 1.35 | 0.04018 | 2.06 |
| Phosphatidylcholine (C16:0,C22:6) | up | 1.09 | 0.04165 | 2.04 |
| PC_Oleic acid (C18:cis[9]1) | up | 1.12 | 0.06220 | 1.87 |
| Sphingomyelin (d18:0,C16:0) | up | 1.08 | 0.11808 | 1.57 |
| Biliverdin | up | 1.18 | 0.12151 | 1.55 |
| TAG_Linolenic acid (C18:cis[9,12,15]3) | up | 1.17 | 0.14699 | 1.45 |

(continued)

| Metabolite | Direction of change | ANOVA result of pancreatic cancer relative to critical controls (plasma combined with serum) | | |
| --- | --- | --- | --- | --- |
| | | Estimated fold change | p-value | t-value |
| TAG_conjugated Linoleic acid (C18:cis[9] trans[11]2) | up | 1.21 | 0.17916 | 1.35 |
| TAG_Docosapentaenoic acid (C22:cis [4,7,10,13,16]5) | up | 1.26 | 0.18170 | 1.34 |
| Taurochenodeoxycholic acid | up | 1.42 | 0.18203 | 1.34 |
| PC_Eicosatrienoic acid (C20:[11,14,17]3) | up | 1.33 | 0.18356 | 1.33 |
| TAG (C16:0,C18:2) | up | 1.06 | 0.19207 | 1.31 |

**Table 26:** List of identified down regulated biomarkers in plasma combined with serum for pancreatic cancer relative to critical controls

| Metabolite | Direction of change | ANOVA result of pancreatic cancer relative to critical controls (plasma combined with serum) | | |
| --- | --- | --- | --- | --- |
| | | Estimated fold change | p-value | t-value |
| LPC_Oleic acid (C18:cis[9]1) | down | 0.69 | 0.00546 | -2.79 |
| Cholesterylester C12:0 | down | 0.75 | 0.00748 | -2.69 |
| Cholesterylester C14:0 | down | 0.86 | 0.00881 | -2.63 |
| Alanine | down | 0.88 | 0.01017 | -2.58 |
| Cholesterylester C18:4 | down | 0.74 | 0.01394 | -2.47 |
| Glycine | down | 0.90 | 0.02482 | -2.25 |
| 2-Methylserine | down | 0.91 | 0.02992 | -2.18 |
| Asparagine | down | 0.91 | 0.04736 | -1.99 |
| Pipecolic acid | down | 0.90 | 0.05013 | -1.96 |
| Glutamine | down | 0.77 | 0.05148 | -1.95 |
| Lysophosphatidylethanolamine (C22:5) | down | 0.92 | 0.05198 | -1.95 |
| DAG_Stearic acid (C18:0) | down | 0.79 | 0.08217 | -1.74 |
| Glycerate | down | 0.88 | 0.09088 | -1.69 |
| TAG_Myristoleic acid (C14:cis[9]1) | down | 0.74 | 0.09560 | -1.67 |
| 5-Oxoproline | down | 0.92 | 0.10028 | -1.65 |
| Choline | down | 0.91 | 0.10048 | -1.65 |
| Salicylic acid | down | 0.80 | 0.10848 | -1.61 |
| Cholesterylester C16:2 | down | 0.90 | 0.11472 | -1.58 |
| Cholesterylester C18:0 | down | 0.91 | 0.16196 | -1.40 |
| PE_Docosapentaenoic acid (C22:cis [7,10,13,16,19]5) | down | 0.79 | 0.16219 | -1.40 |
| Ribose | down | 0.79 | 0.16452 | -1.39 |
| Fumarate | down | 0.91 | 0.16548 | -1.39 |

(continued)

| Metabolite | Direction of change | ANOVA result of pancreatic cancer relative to critical controls (plasma combined with serum) | | |
| | | Estimated fold change | p-value | t-value |
|---|---|---|---|---|
| Glycolate | down | 0.95 | 0.16991 | -1.38 |
| LPC_Stearic acid (C18:0) | down | 0.91 | 0.19809 | -1.29 |

**Table 27:** List of all identified biomarkers in plasma combined with serum for pancreatic cancer relative to pancreatitis (PAC 2A)

| Metabolite | Direction of change | ANOVA result of pancreatic cancer relative to pancreatitis (plasma combined with serum) | | |
| | | Estimated fold change | p-value | t-value |
|---|---|---|---|---|
| Cholesterylester C14:0 | down | 0.77 | 0.00005 | -4.09 |
| Cholesterylester C18:4 | down | 0.58 | 0.00010 | -3.95 |
| Lysophosphatidylethanolamine (C22:5) | down | 0.84 | 0.00012 | -3.90 |
| Cholesterylester C12:0 | down | 0.61 | 0.00015 | -3.84 |
| LPC_Oleic acid (C18:cis[9]1) | down | 0.55 | 0.00022 | -3.73 |
| Eicosapentaenoic acid (C20:cis [5,8,11,14,17]5) | down | 0.68 | 0.00027 | -3.69 |
| Alanine | down | 0.80 | 0.00029 | -3.66 |
| Ceramide (d16:1,C24:0) | down | 0.72 | 0.00034 | -3.63 |
| Cholesterylester C20:5 | down | 0.74 | 0.00057 | -3.48 |
| Lysophosphatidylcholine (C18:1) | down | 0.86 | 0.00091 | -3.35 |
| Cholesterylester C16:2 | down | 0.77 | 0.00109 | -3.30 |
| LPC_Stearic acid (C18:0) | down | 0.78 | 0.00130 | -3.25 |
| Phosphatidylcholine (C16:0,C20:5) | down | 0.86 | 0.00163 | -3.18 |
| PC_Eicosapentaenoic acid (C20:cis [5,8,11,14,17]5) | down | 0.74 | 0.00167 | -3.17 |
| LPC_Palmitic acid (C16:0) | down | 0.76 | 0.00170 | -3.17 |
| Glycine | down | 0.84 | 0.00188 | -3.13 |
| Glycocholic acid | up | 2.46 | 0.00404 | 2.90 |
| Lysophosphatidylcholine (C18:2) | down | 0.81 | 0.00404 | -2.90 |
| 5-Oxoproline | down | 0.85 | 0.00410 | -2.89 |
| Sphinganine-1-phosphate (d18:0) | down | 0.78 | 0.00425 | -2.88 |
| TAG_Myristoleic acid (C14:cis[9]1) | down | 0.55 | 0.00488 | -2.84 |
| LPC_Linoleic acid (C18:cis[9,12]2) | down | 0.63 | 0.00547 | -2.80 |
| Cholesterylester C18:0 | down | 0.81 | 0.00670 | -2.73 |
| Lysophosphatidylcholine (C18:0) | down | 0.86 | 0.00685 | -2.72 |
| Cholesterylester C18:3 | down | 0.82 | 0.00736 | -2.70 |
| Lysophosphatidylcholine (C20:4) | down | 0.87 | 0.00739 | -2.70 |

(continued)

| Metabolite | Direction of change | ANOVA result of pancreatic cancer relative to pancreatitis (plasma combined with serum) | | |
| --- | --- | --- | --- | --- |
| | | Estimated fold change | p-value | t-value |
| Taurochenodeoxycholic acid | up | 2.17 | 0.01179 | 2.53 |
| Leucine | down | 0.86 | 0.01568 | -2.43 |
| Cholesterylester C20:3 | down | 0.85 | 0.01867 | -2.36 |
| Pregnenolone sulfate | down | 0.78 | 0.01873 | -2.36 |
| Serine | down | 0.89 | 0.01943 | -2.35 |
| Carnosine | down | 0.68 | 0.02021 | -2.34 |
| Sphingomyelin (d16:1,C24:0) | down | 0.83 | 0.02233 | -2.30 |
| DAG_Stearic acid (C18:0) | down | 0.72 | 0.02884 | -2.20 |
| Ceramide (d16:1,C23:0) | down | 0.80 | 0.03191 | -2.16 |
| TAG_Palmitoleic acid (C16:cis[9]1) | down | 0.77 | 0.03262 | -2.15 |
| Asparagine | down | 0.89 | 0.03707 | -2.09 |
| Cholesterylester C16:0 | down | 0.90 | 0.03905 | -2.07 |
| PE_Docosapentaenoic acid (C22:cis [7,10,13,16,19]5) | down | 0.67 | 0.04939 | -1.97 |
| Tryptophan | down | 0.87 | 0.05941 | -1.89 |
| Choline | down | 0.88 | 0.06011 | -1.89 |
| Biliverdin | up | 1.26 | 0.06655 | 1.84 |
| Valine | down | 0.91 | 0.07986 | -1.76 |
| Glycerate | down | 0.86 | 0.08079 | -1.75 |
| Ceramide (d18:1,C24:0) | down | 0.90 | 0.09344 | -1.68 |
| Lysine | down | 0.91 | 0.10007 | -1.65 |
| Lauric acid (C12:0) | down | 0.81 | 0.10060 | -1.65 |
| Myristic acid (C14:0) | down | 0.83 | 0.10275 | -1.64 |
| Pipecolic acid | down | 0.90 | 0.11358 | -1.59 |
| Indole-3-lactic acid | down | 0.89 | 0.11582 | -1.58 |
| Cholesterylester C22:5 | down | 0.91 | 0.11967 | -1.56 |
| 2-Methylserine | down | 0.91 | 0.12497 | -1.54 |
| Cysteine | down | 0.86 | 0.13689 | -1.49 |
| Fumarate | down | 0.89 | 0.14401 | -1.46 |
| Glucose-1-phosphate | up | 1.25 | 0.14459 | 1.46 |
| Glucose | up | 1.37 | 0.16252 | 1.40 |
| Lysophosphatidylcholine (C17:0) | down | 0.91 | 0.18118 | -1.34 |
| Glutamine | down | 0.81 | 0.18744 | -1.32 |
| Lactate | down | 0.87 | 0.18771 | -1.32 |
| Sorbitol | down | 0.84 | 0.19282 | -1.31 |
| PC_Myristoleic acid (C14:cis[9]1) | down | 0.76 | 0.19825 | -1.29 |

(continued)

| Metabolite | Direction of change | ANOVA result of pancreatic cancer relative to pancreatitis (plasma combined with serum) | | |
| --- | --- | --- | --- | --- |
| | | Estimated fold change | p-value | t-value |
| Ketoleucine | down | 0.89 | 0.19870 | -1.29 |
| Pyruvate | down | 0.84 | 0.19911 | -1.29 |

**Table 28:** List of identified up regulated biomarkers in plasma combined with serum for pancreatic cancer relative to pancreatitis

| Metabolite | Direction of change | ANOVA result of pancreatic cancer relative to pancreatitis (plasma combined with serum) | | |
| --- | --- | --- | --- | --- |
| | | Estimated fold change | p-value | t-value |
| Glycocholic acid | up | 2.46 | 0.00404 | 2.90 |
| Taurochenodeoxycholic acid | up | 2.17 | 0.01179 | 2.53 |
| Biliverdin | up | 1.26 | 0.06655 | 1.84 |
| Glucose-1-phosphate | up | 1.25 | 0.14459 | 1.46 |
| Glucose | up | 1.37 | 0.16252 | 1.40 |

**Table 29:** List of identified down regulated biomarkers in plasma combined with serum for pancreatic cancer relative to pancreatitis

| Metabolite | Direction of change | ANOVA result of pancreatic cancer relative to pancreatitis (plasma combined with serum) | | |
| --- | --- | --- | --- | --- |
| | | Estimated fold change | p-value | t-value |
| Cholesterylester C14:0 | down | 0.77 | 0.00005 | -4.09 |
| Cholesterylester C18:4 | down | 0.58 | 0.00010 | -3.95 |
| Lysophosphatidylethanolamine (C22:5) | down | 0.84 | 0.00012 | -3.90 |
| Cholesterylester C12:0 | down | 0.61 | 0.00015 | -3.84 |
| LPC_Oleic acid (C18:cis[9]1) | down | 0.55 | 0.00022 | -3.73 |
| Eicosapentaenoic acid (C20:cis [5,8,11,14,17]5) | down | 0.68 | 0.00027 | -3.69 |
| Alanine | down | 0.80 | 0.00029 | -3.66 |
| Ceramide (d16:1,C24:0) | down | 0.72 | 0.00034 | -3.63 |
| Cholesterylester C20:5 | down | 0.74 | 0.00057 | -3.48 |
| Lysophosphatidylcholine (C18:1) | down | 0.86 | 0.00091 | -3.35 |
| Cholesterylester C16:2 | down | 0.77 | 0.00109 | -3.30 |
| LPC_Stearic acid (C18:0) | down | 0.78 | 0.00130 | -3.25 |
| Phosphatidylcholine (C16:0,C20:5) | down | 0.86 | 0.00163 | -3.18 |
| PC_Eicosapentaenoic acid (C20:cis [5,8,11,14,17]5) | down | 0.74 | 0.00167 | -3.17 |

(continued)

| Metabolite | Direction of change | ANOVA result of pancreatic cancer relative to pancreatitis (plasma combined with serum) | | |
| --- | --- | --- | --- | --- |
| | | Estimated fold change | p-value | t-value |
| LPC_Palmitic acid (C16:0) | down | 0.76 | 0.00170 | -3.17 |
| Glycine | down | 0.84 | 0.00188 | -3.13 |
| Lysophosphatidylcholine (C18:2) | down | 0.81 | 0.00404 | -2.90 |
| 5-Oxoproline | down | 0.85 | 0.00410 | -2.89 |
| Sphinganine-1-phosphate (d18:0) | down | 0.78 | 0.00425 | -2.88 |
| TAG_Myristoleic acid (C14:cis[9]1) | down | 0.55 | 0.00488 | -2.84 |
| LPC_Linoleic acid (C18:cis[9,12]2) | down | 0.63 | 0.00547 | -2.80 |
| Cholesterylester C18:0 | down | 0.81 | 0.00670 | -2.73 |
| Lysophosphatidylcholine (C18:0) | down | 0.86 | 0.00685 | -2.72 |
| Cholesterylester C18:3 | down | 0.82 | 0.00736 | -2.70 |
| Lysophosphatidylcholine (C20:4) | down | 0.87 | 0.00739 | -2.70 |
| Leucine | down | 0.86 | 0.01568 | -2.43 |
| Cholesterylester C20:3 | down | 0.85 | 0.01867 | -2.36 |
| Pregnenolone sulfate | down | 0.78 | 0.01873 | -2.36 |
| Serine | down | 0.89 | 0.01943 | -2.35 |
| Carnosine | down | 0.68 | 0.02021 | -2.34 |
| Sphingomyelin (d16:1,C24:0) | down | 0.83 | 0.02233 | -2.30 |
| DAG_Stearic acid (C18:0) | down | 0.72 | 0.02884 | -2.20 |
| Ceramide (d16:1,C23:0) | down | 0.80 | 0.03191 | -2.16 |
| TAG_Palmitoleic acid (C16:cis[9]1) | down | 0.77 | 0.03262 | -2.15 |
| Asparagine | down | 0.89 | 0.03707 | -2.09 |
| Cholesterylester C16:0 | down | 0.90 | 0.03905 | -2.07 |
| PE_Docosapentaenoic acid (C22:cis[7,10,13,16,19]5) | down | 0.67 | 0.04939 | -1.97 |
| Tryptophan | down | 0.87 | 0.05941 | -1.89 |
| Choline | down | 0.88 | 0.06011 | -1.89 |
| Valine | down | 0.91 | 0.07986 | -1.76 |
| Glycerate | down | 0.86 | 0.08079 | -1.75 |
| Ceramide (d18:1,C24:0) | down | 0.90 | 0.09344 | -1.68 |
| Lysine | down | 0.91 | 0.10007 | -1.65 |
| Lauric acid (C12:0) | down | 0.81 | 0.10060 | -1.65 |
| Myristic acid (C14:0) | down | 0.83 | 0.10275 | -1.64 |
| Pipecolic acid | down | 0.90 | 0.11358 | -1.59 |
| Indole-3-lactic acid | down | 0.89 | 0.11582 | -1.58 |
| Cholesterylester C22:5 | down | 0.91 | 0.11967 | -1.56 |
| 2-Methylserine | down | 0.91 | 0.12497 | -1.54 |

(continued)

| Metabolite | Direction of change | ANOVA result of pancreatic cancer relative to pancreatitis (plasma combined with serum) | | |
|---|---|---|---|---|
| | | Estimated fold change | p-value | t-value |
| Cysteine | down | 0.86 | 0.13689 | -1.49 |
| Fumarate | down | 0.89 | 0.14401 | -1.46 |
| Lysophosphatidylcholine (C17:0) | down | 0.91 | 0.18118 | -1.34 |
| Glutamine | down | 0.81 | 0.18744 | -1.32 |
| Lactate | down | 0.87 | 0.18771 | -1.32 |
| Sorbitol | down | 0.84 | 0.19282 | -1.31 |
| PC_Myristoleic acid (C14:cis[9]1) | down | 0.76 | 0.19825 | -1.29 |
| Ketoleucine | down | 0.89 | 0.19870 | -1.29 |
| Pyruvate | down | 0.84 | 0.19911 | -1.29 |

**Table 30:** List of all identified biomarkers in plasma combined with serum for pancreatic cancer relative to liver cirrhosis (PAC 2B)

| Metabolite | Direction of change | ANOVA result of pancreatic cancer relative to liver cirrhosis (plasma combined with serum) | | |
|---|---|---|---|---|
| | | Estimated fold change | p-value | t-value |
| Stearic acid (C18:0) | up | 1.40 | 0.00005 | 4.14 |
| Ceramide (d18:1,C24:0) | up | 1.48 | 0.00008 | 4.01 |
| Cholesterylester C20:5 | up | 1.65 | 0.00013 | 3.89 |
| PC_Eicosapentaenoic acid (C20:cis [5,8,11,14,17]5) | up | 1.76 | 0.00014 | 3.87 |
| Cholesterylester C20:3 | up | 1.46 | 0.00040 | 3.59 |
| Phosphatidylcholine (C16:0,C22:6) | up | 1.25 | 0.00041 | 3.58 |
| Sphingomyelin (d16:1,C24:0) | up | 1.48 | 0.00069 | 3.44 |
| Eicosapentaenoic acid (C20:cis [5,8,11,14,17]5) | up | 1.51 | 0.00085 | 3.38 |
| TAG_Linolenic acid (C18:cis[9,12,15]3) | up | 1.62 | 0.00099 | 3.34 |
| PC_Oleic acid (C18:cis[9]1) | up | 1.33 | 0.00119 | 3.28 |
| LPC_Linoleic acid (C18:cis[9,12]2) | up | 2.33 | 0.00128 | 3.26 |
| PC_gamma-Linolenic acid (C18:cis [6,9,12]3) | up | 2.07 | 0.00134 | 3.25 |
| Ceramide (d16:1,C24:0) | up | 1.48 | 0.00138 | 3.24 |
| Cholesterylester C18:3 | up | 1.40 | 0.00139 | 3.24 |
| Docosapentaenoic acid (C22:cis [7,10,13,16,19]5) | up | 1.35 | 0.00180 | 3.16 |
| Ceramide (d16:1,C23:0) | up | 1.52 | 0.00193 | 3.14 |

(continued)

| Metabolite | Direction of change | ANOVA result of pancreatic cancer relative to liver cirrhosis (plasma combined with serum) | | |
| --- | --- | --- | --- | --- |
| | | Estimated fold change | p-value | t-value |
| PC_Docosapentaenoic acid (C22:cis [4,7,10,13,16]5) | up | 1.85 | 0.00220 | 3.10 |
| Carnosine | up | 1.74 | 0.00273 | 3.04 |
| FFA_Docosahexaenoic acid (C22:cis [4,7,10,13,16,19]6) | up | 1.89 | 0.00275 | 3.03 |
| TAG_Eicosadienoic acid (C20:cis[11,14]2) | up | 1.87 | 0.00290 | 3.01 |
| TAG_Eicosapentaenoic acid (C20:cis [5,8,11,14,17]5) | up | 2.19 | 0.00302 | 3.00 |
| Heptadecenoic acid (C17:cis[10]1) | up | 1.28 | 0.00325 | 2.97 |
| TAG_conjugated Linoleic acid (C18:cis[9] trans[11]2) | up | 1.77 | 0.00470 | 2.85 |
| 14-Methylhexadecanoic acid | up | 1.29 | 0.00545 | 2.81 |
| Cholesterylester C18:2 | up | 1.30 | 0.00572 | 2.79 |
| Lysophosphatidylcholine (C18:0) | up | 1.25 | 0.00580 | 2.78 |
| TAG_Docosapentaenoic acid (C22:cis [4,7,10,13,16]5) | up | 1.96 | 0.00704 | 2.72 |
| PC_Linolenic acid (C18:cis[9,12,15]3) | up | 1.77 | 0.00818 | 2.67 |
| Lysophosphatidylcholine (C20:4) | up | 1.19 | 0.00833 | 2.66 |
| Lysophosphatidylcholine (C16:0) | up | 1.13 | 0.00885 | 2.64 |
| Phosphatidylcholine (C16:0,C20:5) | up | 1.16 | 0.00910 | 2.63 |
| Sphingosine-1-phosphate (d16:1) | up | 1.34 | 0.01151 | 2.55 |
| Ribose | down | 0.58 | 0.01196 | -2.54 |
| FFA_Eicosapentaenoic acid (C20:cis [5,8,11,14,17]5) | up | 1.95 | 0.01286 | 2.51 |
| Cholesterylester C16:2 | up | 1.25 | 0.01428 | 2.47 |
| Cholesterylester C16:0 | up | 1.21 | 0.01443 | 2.47 |
| TAG_Docosapentaenoic acid (C22:cis [7,10,13,16,19]5) | up | 1.86 | 0.01534 | 2.44 |
| LPC_Stearic acid (C18:0) | up | 1.27 | 0.01574 | 2.43 |
| Leucine | up | 1.18 | 0.01738 | 2.40 |
| Cholesterylester C22:5 | up | 1.21 | 0.01781 | 2.39 |
| PC_Myristic acid (C14:0) | up | 2.02 | 0.01880 | 2.37 |
| LPC_Palmitic acid (C16:0) | up | 1.33 | 0.01927 | 2.36 |
| Valine | up | 1.16 | 0.01964 | 2.35 |
| Kynurenine | down | 0.84 | 0.02218 | -2.30 |
| Myristic acid (C14:0) | up | 1.29 | 0.02234 | 2.30 |
| TAG_Myristic acid (C14:0) | up | 1.59 | 0.02424 | 2.27 |

(continued)

| Metabolite | Direction of change | ANOVA result of pancreatic cancer relative to liver cirrhosis (plasma combined with serum) | | |
| --- | --- | --- | --- | --- |
| | | Estimated fold change | p-value | t-value |
| Lysophosphatidylcholine (C18:2) | up | 1.23 | 0.02479 | 2.26 |
| TAG_Eicosanoic acid (C20:0) | up | 1.61 | 0.02915 | 2.20 |
| DAG_Oleic acid (C18:cis[9]1) | up | 1.91 | 0.02971 | 2.19 |
| Lysophosphatidylcholine (C17:0) | up | 1.22 | 0.03054 | 2.18 |
| Docosatetraenoic acid (C22:cis [7,10,13,16]4) | up | 1.19 | 0.03291 | 2.15 |
| Sphingomyelin (d16:1,C23:0) | up | 1.27 | 0.03500 | 2.12 |
| FFA_Docosapentaenoic acid (C22:cis [7,10,13,16,19]5) | up | 1.78 | 0.03767 | 2.09 |
| TAG_Docosahexaenoic acid (C22:cis [4,7,10,13,16,19]6) | up | 1.77 | 0.03880 | 2.08 |
| Taurochenodeoxycholic acid | down | 0.46 | 0.03929 | -2.07 |
| myo-Inositol, lipid fraction | up | 1.18 | 0.04068 | 2.06 |
| Pipecolic acid | down | 0.86 | 0.04451 | -2.02 |
| Erucic acid (C22:cis[13]1) | up | 1.07 | 0.05519 | 1.93 |
| Lysophosphatidylcholine (C18:1) | up | 1.12 | 0.05540 | 1.93 |
| Cholesterylester C15:0 | up | 1.17 | 0.05800 | 1.91 |
| Sphingomyelin (d18:0,C16:0) | up | 1.15 | 0.05841 | 1.90 |
| Cholesterylester C18:2 | up | 1.17 | 0.05915 | 1.90 |
| Lauric acid (C12:0) | up | 1.27 | 0.06037 | 1.89 |
| PC_Myristoleic acid (C14:cis[9]1) | up | 1.57 | 0.06602 | 1.85 |
| Eicosenoic acid (C20:cis[11]1) | up | 1.14 | 0.06673 | 1.84 |
| Sphinganine-1-phosphate (d18:0) | up | 1.21 | 0.06750 | 1.84 |
| PC_Eicosatrienoic acid (C20:[11,14,17]3) | up | 1.70 | 0.07040 | 1.82 |
| Phosphatidylcholine (C18:1,C18:2) | up | 1.05 | 0.07407 | 1.79 |
| 1-Methyladenosine | down | 0.91 | 0.07889 | -1.76 |
| Cholesterylester C18:4 | up | 1.30 | 0.08050 | 1.76 |
| Glutamine | down | 0.74 | 0.10018 | -1.65 |
| Proline betaine | down | 0.71 | 0.10394 | -1.63 |
| PI_Myristic acid (C14:0) | up | 1.74 | 0.10509 | 1.63 |
| Ketoleucine | up | 1.16 | 0.11319 | 1.59 |
| TAG_Palmitoleic acid (C16:cis[9]1) | up | 1.20 | 0.12119 | 1.56 |
| PC_Palmitoleic acid (C16:cis[9]1) | up | 1.22 | 0.12245 | 1.55 |
| Cholesterylester C20:1 | down | 0.86 | 0.13202 | -1.51 |
| PI_Myristoleic acid (C14:cis[9]1) | up | 1.54 | 0.13299 | 1.51 |
| Lysophosphatidylethanolamine (C22:5) | up | 1.10 | 0.13392 | 1.50 |

(continued)

| Metabolite | Direction of change | ANOVA result of pancreatic cancer relative to liver cirrhosis (plasma combined with serum) | | |
| --- | --- | --- | --- | --- |
| | | Estimated fold change | p-value | t-value |
| 5-Hydroxy-3-indoleacetic acid (5-HIAA) | down | 0.86 | 0.13656 | -1.49 |
| PE_Oleic acid (C18:cis[9]1) | up | 1.40 | 0.14003 | 1.48 |
| PC_Elaidic acid (C18:trans[9]1) | up | 1.29 | 0.14045 | 1.48 |
| Fumarate | down | 0.89 | 0.15659 | -1.42 |
| Biliverdin | down | 0.81 | 0.16094 | -1.41 |
| Glucosamine | up | 1.25 | 0.17602 | 1.36 |
| Glycolate | down | 0.93 | 0.17616 | -1.36 |
| Malate | down | 0.86 | 0.17952 | -1.35 |
| PC_Eicosenoic acid (C20:cis[11]1) | up | 1.24 | 0.18019 | 1.34 |
| PS_Myristoleic acid (C14:cis[9]1) | up | 1.81 | 0.18664 | 1.33 |
| Tryptophan | up | 1.10 | 0.18840 | 1.32 |
| DAG_Myristic acid (C14:0) | up | 1.57 | 0.19098 | 1.31 |
| Cholesterylester C20:4 | up | 1.18 | 0.19414 | 1.30 |

**Table 31:** List of identified up regulated biomarkers in plasma combined with serum for pancreatic cancer relative to liver cirrhosis

| Metabolite | Direction of change | ANOVA result of pancreatic cancer relative to liver cirrhosis (plasma combined with serum) | | |
| --- | --- | --- | --- | --- |
| | | Estimated fold change | p-value | t-value |
| Stearic acid (C18:0) | up | 1.40 | 0.00005 | 4.14 |
| Ceramide (d18:1,C24:0) | up | 1.48 | 0.00008 | 4.01 |
| Cholesterylester C20:5 | up | 1.65 | 0.00013 | 3.89 |
| PC_Eicosapentaenoic acid (C20:cis [5,8,11,14,17]5) | up | 1.76 | 0.00014 | 3.87 |
| Cholesterylester C20:3 | up | 1.46 | 0.00040 | 3.59 |
| Phosphatidylcholine (C16:0,C22:6) | up | 1.25 | 0.00041 | 3.58 |
| Sphingomyelin (d16:1,C24:0) | up | 1.48 | 0.00069 | 3.44 |
| Eicosapentaenoic acid (C20:cis [5,8,11,14,17]5) | up | 1.51 | 0.00085 | 3.38 |
| TAG_Linolenic acid (C18:cis[9,12,15]3) | up | 1.62 | 0.00099 | 3.34 |
| PC_Oleic acid (C18:cis[9]1) | up | 1.33 | 0.00119 | 3.28 |
| LPC_Linoleic acid (C18:cis[9,12]2) | up | 2.33 | 0.00128 | 3.26 |
| PC_gamma-Linolenic acid (C18:cis[6,9,12] 3) | up | 2.07 | 0.00134 | 3.25 |
| Ceramide (d16:1,C24:0) | up | 1.48 | 0.00138 | 3.24 |
| Cholesterylester C18:3 | up | 1.40 | 0.00139 | 3.24 |

(continued)

| Metabolite | Direction of change | ANOVA result of pancreatic cancer relative to liver cirrhosis (plasma combined with serum) | | |
| --- | --- | --- | --- | --- |
| | | Estimated fold change | p-value | t-value |
| Docosapentaenoic acid (C22:cis [7,10,13,16,19]5) | up | 1.35 | 0.00180 | 3.16 |
| Ceramide (d16:1,C23:0) | up | 1.52 | 0.00193 | 3.14 |
| PC_Docosapentaenoic acid (C22:cis [4,7,10,13,16]5) | up | 1.85 | 0.00220 | 3.10 |
| Carnosine | up | 1.74 | 0.00273 | 3.04 |
| FFA_Docosahexaenoic acid (C22:cis [4,7,10,13,16,19]6) | up | 1.89 | 0.00275 | 3.03 |
| TAG_Eicosadienoic acid (C20:cis[11,14]2) | up | 1.87 | 0.00290 | 3.01 |
| TAG_Eicosapentaenoic acid (C20:cis [5,8,11,14,17]5) | up | 2.19 | 0.00302 | 3.00 |
| Heptadecenoic acid (C17:cis[10]1) | up | 1.28 | 0.00325 | 2.97 |
| TAG_conjugated Linoleic acid (C18:cis[9] trans[11]2) | up | 1.77 | 0.00470 | 2.85 |
| 14-Methylhexadecanoic acid | up | 1.29 | 0.00545 | 2.81 |
| Cholesterylester C18:2 | up | 1.30 | 0.00572 | 2.79 |
| Lysophosphatidylcholine (C18:0) | up | 1.25 | 0.00580 | 2.78 |
| TAG_Docosapentaenoic acid (C22:cis [4,7,10,13,16]5) | up | 1.96 | 0.00704 | 2.72 |
| PC_Linolenic acid (C18:cis[9,12,15]3) | up | 1.77 | 0.00818 | 2.67 |
| Lysophosphatidylcholine (C20:4) | up | 1.19 | 0.00833 | 2.66 |
| Lysophosphatidylcholine (C16:0) | up | 1.13 | 0.00885 | 2.64 |
| Phosphatidylcholine (C16:0,C20:5) | up | 1.16 | 0.00910 | 2.63 |
| Sphingosine-1-phosphate (d16:1) | up | 1.34 | 0.01151 | 2.55 |
| FFA_Eicosapentaenoic acid (C20:cis [5,8,11,14,17]5) | up | 1.95 | 0.01286 | 2.51 |
| Cholesterylester C16:2 | up | 1.25 | 0.01428 | 2.47 |
| Cholesterylester C16:0 | up | 1.21 | 0.01443 | 2.47 |
| TAG_Docosapentaenoic acid (C22:cis [7,10,13,16,19]5) | up | 1.86 | 0.01534 | 2.44 |
| LPC_Stearic acid (C18:0) | up | 1.27 | 0.01574 | 2.43 |
| Leucine | up | 1.18 | 0.01738 | 2.40 |
| Cholesterylester C22:5 | up | 1.21 | 0.01781 | 2.39 |
| PC_Myristic acid (C14:0) | up | 2.02 | 0.01880 | 2.37 |
| LPC_Palmitic acid (C16:0) | up | 1.33 | 0.01927 | 2.36 |
| Valine | up | 1.16 | 0.01964 | 2.35 |
| Myristic acid (C14:0) | up | 1.29 | 0.02234 | 2.30 |
| TAG_Myristic acid (C14:0) | up | 1.59 | 0.02424 | 2.27 |

(continued)

| Metabolite | Direction of change | ANOVA result of pancreatic cancer relative to liver cirrhosis (plasma combined with serum) | | |
| --- | --- | --- | --- | --- |
| | | Estimated fold change | p-value | t-value |
| Lysophosphatidylcholine (C18:2) | up | 1.23 | 0.02479 | 2.26 |
| TAG_Eicosanoic acid (C20:0) | up | 1.61 | 0.02915 | 2.20 |
| DAG_Oleic acid (C18:cis[9]1) | up | 1.91 | 0.02971 | 2.19 |
| Lysophosphatidylcholine (C17:0) | up | 1.22 | 0.03054 | 2.18 |
| Docosatetraenoic acid (C22:cis[7,10,13,16]4) | up | 1.19 | 0.03291 | 2.15 |
| Sphingomyelin (d16:1,C23:0) | up | 1.27 | 0.03500 | 2.12 |
| FFA_Docosapentaenoic acid (C22:cis[7,10,13,16,19]5) | up | 1.78 | 0.03767 | 2.09 |
| TAG_Docosahexaenoic acid (C22:cis[4,7,10,13,16,19]6) | up | 1.77 | 0.03880 | 2.08 |
| myo-Inositol, lipid fraction | up | 1.18 | 0.04068 | 2.06 |
| Erucic acid (C22:cis[13]1) | up | 1.07 | 0.05519 | 1.93 |
| Lysophosphatidylcholine (C18:1) | up | 1.12 | 0.05540 | 1.93 |
| Cholesterylester C15:0 | up | 1.17 | 0.05800 | 1.91 |
| Sphingomyelin (d18:0,C16:0) | up | 1.15 | 0.05841 | 1.90 |
| Cholesterylester C18:2 | up | 1.17 | 0.05915 | 1.90 |
| Lauric acid (C12:0) | up | 1.27 | 0.06037 | 1.89 |
| PC_Myristoleic acid (C14:cis[9]1) | up | 1.57 | 0.06602 | 1.85 |
| Eicosenoic acid (C20:cis[11]1) | up | 1.14 | 0.06673 | 1.84 |
| Sphinganine-1-phosphate (d18:0) | up | 1.21 | 0.06750 | 1.84 |
| PC_Eicosatrienoic acid (C20:[11,14,17]3) | up | 1.70 | 0.07040 | 1.82 |
| Phosphatidylcholine (C18:1,C18:2) | up | 1.05 | 0.07407 | 1.79 |
| Cholesterylester C18:4 | up | 1.30 | 0.08050 | 1.76 |
| PI_Myristic acid (C14:0) | up | 1.74 | 0.10509 | 1.63 |
| Ketoleucine | up | 1.16 | 0.11319 | 1.59 |
| TAG_Palmitoleic acid (C16:cis[9]1) | up | 1.20 | 0.12119 | 1.56 |
| PC_Palmitoleic acid (C16:cis[9]1) | up | 1.22 | 0.12245 | 1.55 |
| PI_Myristoleic acid (C14:cis[9]1) | up | 1.54 | 0.13299 | 1.51 |
| Lysophosphatidylethanolamine (C22:5) | up | 1.10 | 0.13392 | 1.50 |
| PE_Oleic acid (C18:cis[9]1) | up | 1.40 | 0.14003 | 1.48 |
| PC_Elaidic acid (C18:trans[9]1) | up | 1.29 | 0.14045 | 1.48 |
| Glucosamine | up | 1.25 | 0.17602 | 1.36 |
| PC_Eicosenoic acid (C20:cis[11]1) | up | 1.24 | 0.18019 | 1.34 |
| PS_Myristoleic acid (C14:cis[9]1) | up | 1.81 | 0.18664 | 1.33 |
| Tryptophan | up | 1.10 | 0.18840 | 1.32 |

(continued)

| Metabolite | Direction of change | ANOVA result of pancreatic cancer relative to liver cirrhosis (plasma combined with serum) | | |
|---|---|---|---|---|
| | | Estimated fold change | p-value | t-value |
| DAG_Myristic acid (C14:0) | up | 1.57 | 0.19098 | 1.31 |
| Cholesterylester C20:4 | up | 1.18 | 0.19414 | 1.30 |

**Table 32:** List of identified down regulated biomarkers in plasma combined with serum for pancreatic cancer relative to liver cirrhosis

| Metabolite | Direction of change | ANOVA result of pancreatic cancer relative to liver cirrhosis (plasma combined with serum) | | |
|---|---|---|---|---|
| | | Estimated fold change | p-value | t-value |
| Ribose | down | 0.58 | 0.01196 | -2.54 |
| Kynurenine | down | 0.84 | 0.02218 | -2.30 |
| Taurochenodeoxycholic acid | down | 0.46 | 0.03929 | -2.07 |
| Pipecolic acid | down | 0.86 | 0.04451 | -2.02 |
| 1-Methyladenosine | down | 0.91 | 0.07889 | -1.76 |
| Glutamine | down | 0.74 | 0.10018 | -1.65 |
| Proline betaine | down | 0.71 | 0.10394 | -1.63 |
| Cholesterylester C20:1 | down | 0.86 | 0.13202 | -1.51 |
| 5-Hydroxy-3-indoleacetic acid (5-HIAA) | down | 0.86 | 0.13656 | -1.49 |
| Fumarate | down | 0.89 | 0.15659 | -1.42 |
| Biliverdin | down | 0.81 | 0.16094 | -1.41 |
| Glycolate | down | 0.93 | 0.17616 | -1.36 |
| Malate | down | 0.86 | 0.17952 | -1.35 |

**Claims**

1. A method for diagnosing pancreas cancer in a subject comprising the steps of:

(a) determining in a plasma, blood or serum sample of a subject suspected to suffer from pancreas cancer at least the amount of the biomarker Ceramide (d18:1,C24:0) and
(b) comparing the said amount of the at least one biomarker with a reference, whereby pancreas cancer is to be diagnosed.

2. The method of claim 1, wherein said reference is derived from a sample of a subject or group of subjects known not to suffer from pancreatic cancer or is a calculated reference.

3. The method of claim 1, wherein said reference is derived from a sample of a subject or group of subjects known to suffer from pancreatic cancer.

4. The method of any one of claims 1 to 3, wherein said subject exhibits pancreatitis as an underlying pancreatic disease.

**5.** The method of claim 1, wherein said reference is derived from a subject or group of subjects suffering from liver cirrhosis.

**6.** The method of any one of claims 1 to 5, wherein said subject is a human.

**7.** The method of any one of claims 1 to 7, wherein said pancreas cancer is pancreas adenocarcinoma.

**8.** A device for diagnosing pancreas cancer in a sample of a subject comprising:

a) an analyzing unit for a plasma, blood or serum sample of the subject comprising a detector for the biomarker Ceramide (d18:1,C24:0), said detector allowing for the determination of the amount of the said biomarker in the sample; and operatively linked thereto,
b) an evaluation unit comprising a data processing unit and a data base, said data base comprising a stored reference and said data processing unit having tangibly embedded an algorithm for carrying out a comparison of the amount of the biomarker determined by the analyzing unit and the stored reference and for generating an output information based on which the diagnosis can be established, wherein the output information is at least a classifier which indicates whether the subject suffers from pancreatic cancer or not

**9.** Use of the biomarker Ceramide (d18:1,C24:0) or a detection agent therefor in a plasma, blood or serum sample of a subject suspected to suffer from pancreatic cancer for diagnosing pancreatic cancer.

**Patentansprüche**

**1.** Verfahren zur Diagnose von Bauchspeicheldrüsenkrebs bei einem Individuum, umfassend die Schritte:

(a) Bestimmen von mindestens der Menge des Biomarkers Ceramid (d18:1, C24:0) in einer Plasma-, Blut- oder Serumprobe von einem Individuum, das vermutlich an Bauchspeicheldrüsenkrebs leidet, und
(b) Vergleichen der Menge des mindestens einen Biomarkers mit einer Referenz, wodurch Bauchspeicheldrüsenkrebs diagnostiziert werden soll.

**2.** Verfahren von Anspruch 1, wobei die Referenz von einer Probe von einem Individuum oder einer Gruppe von Individuen abgeleitet ist, von denen bekannt ist, dass sie nicht an Bauchspeicheldrüsenkrebs leiden, oder eine berechnete Referenz ist.

**3.** Verfahren von Anspruch 1, wobei die Referenz aus einer Probe von einem Individuum oder einer Gruppe von Individuen abgeleitet ist, von denen bekannt ist, dass sie an Bauchspeicheldrüsenkrebs leiden.

**4.** Verfahren von einem der Ansprüche 1 bis 3, wobei das Individuum Pankreatitis als eine zugrunde liegende Bauchspeicheldrüsenerkrankung zeigt.

**5.** Verfahren von Anspruch 1, wobei die Referenz aus einem Individuum oder einer Gruppe von Individuen abgeleitet ist, die an Leberzirrhose leiden.

**6.** Verfahren von einem der Ansprüche 1 bis 5, wobei das Individuum ein Mensch ist.

**7.** Verfahren von einem der Ansprüche 1 bis 7, wobei es sich bei dem Bauchspeicheldrüsenkrebs um ein Pankreasadenokarzinom handelt.

**8.** Gerät zur Diagnose von Bauchspeicheldrüsenkrebs in einer Probe eines Individuums, umfassend:

a) eine Analyseeinheit für eine Plasma-, Blut- oder Serumprobe des Individuums, umfassend einen Detektor für den Biomarker Ceramid (d18:1,C24:0), wobei der Detektor die Bestimmung der Menge des Biomarkers in der Probe ermöglicht; und operativ damit verbunden
b) eine Auswertungseinheit, die eine Datenverarbeitungseinheit und eine Datenbank umfasst, wobei die Datenbank eine gespeicherte Referenz umfasst und wobei bei der Datenverarbeitungseinheit ein Algorithmus zum Durchführen eines Vergleichs der durch die Analyseeinheit bestimmten Menge des Biomarkers und der gespeicherten Referenz und zum Erzeugen einer Ausgabeinformation, auf deren Grundlage die Diagnose erstellt werden kann, greifbar eingebettet ist, wobei die Ausgabeinformation mindestens ein Klassifizierer ist, der angibt, ob das Individuum an Bauchspeicheldrüsenkrebs leidet oder nicht.

9. Verwendung des Biomarkers Ceramid (d18:1,C24:0) oder eines Nachweismittels dafür in einer Plasma-, Blut- oder Serumprobe von einem Individuums, bei dem der Verdacht besteht, an Bauchspeicheldrüsenkrebs zu leiden, zur Diagnose von Bauchspeicheldrüsenkrebs.

**Revendications**

1. Méthode de diagnostic du cancer du pancréas chez un sujet comprenant les étapes consistant à :

   (a) déterminer dans un échantillon de plasma, de sang ou de sérum d'un sujet soupçonné de souffrir d'un cancer du pancréas au moins la quantité du biomarqueur Céramide (d18:1,C24:0) et
   (b) comparer ladite quantité de l'au moins un biomarqueur à une référence, par le biais de laquelle le cancer du pancréas doit être diagnostiqué.

2. Méthode selon la revendication 1, où ladite référence est dérivée d'un échantillon prélevé chez un sujet ou un groupe de sujets connus comme ne souffrant pas d'un cancer pancréatique ou est une référence calculée.

3. Méthode selon la revendication 1, où ladite référence est dérivée d'un échantillon prélevé chez un sujet ou un groupe de sujets connus comme souffrant d'un cancer pancréatique.

4. Méthode selon l'une quelconque des revendications 1 à 3, où ledit sujet souffre de pancréatite en tant que maladie pancréatique sous-jacente.

5. Méthode selon la revendication 1, où ladite référence est dérivée d'un sujet ou d'un groupe de sujets souffrant de cirrhose hépatique.

6. Méthode selon l'une quelconque des revendications 1 à 5, où ledit sujet est un humain.

7. Méthode selon l'une quelconque des revendications 1 à 7, où ledit cancer du pancréas est un adénocarcinome pancréatique.

8. Dispositif de diagnostic du cancer du pancréas dans un échantillon d'un sujet comprenant :

   a) une unité d'analyse d'un échantillon de plasma, de sang ou de sérum du sujet comprenant un détecteur pour le biomarqueur Céramide (d18:1, C24:0), ledit détecteur permettant de déterminer la quantité dudit biomarqueur dans l'échantillon, et, connectée de façon fonctionnelle à celle-ci,
   b) une unité d'évaluation comprenant une unité de traitement des données et une base de données, ladite base de données comprenant une référence stockée et ladite unité de traitement des données intégrant de façon tangible un algorithme pour mettre en oeuvre la comparaison de la quantité du biomarqueur déterminée par l'unité d'analyse et la référence stockée et pour générer des informations de sortie sur la base desquels le diagnostic peut être établi, où les informations de sortie sont au moins un classificateur qui indique que le sujet souffre ou non d'un cancer du pancréas.

9. Utilisation du biomarqueur Céramide (d18:1,C24:0) ou d'un agent de détection de celui-ci dans un échantillon de plasma, de sang ou de sérum d'un sujet soupçonné de souffrir d'un cancer du pancréas pour le diagnostic du cancer du pancréas.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011038509 A
- US 4540884 A
- US 5397894 A
- WO 03073464 A

### Non-patent literature cited in the description

- **EVERHART.** *Gastroenterology,* 2009, vol. 136, 1134-11449
- **DEWITT.** *Gastroenterol Hepatol,* 2006, vol. 4, 717-25
- **FRY.** *Langenbecks Arch Surg,* 2008, vol. 393, 883-90
- **HRUBAN.** *Am J Surg Path,* 2004, vol. 28, 977-987
- **LOWENFELS.** *N Engl J Med,* 1993, vol. 328, 1433-1437
- **HOWES.** *Clinical Gastroenterology and Hepatology,* 2004, vol. 2, 252-261
- **LOWENFELS.** *JAMA,* 2001, vol. 286, 169-170
- **LOWENFELS.** *J Natl Cancer Inst,* 1997, vol. 89, 442-44656
- **SCHRADER.** *Panceas,* 2009, vol. 38, 416-421
- **MODRAK.** *Mol Cancer Res,* 2009, vol. 7, 890-896
- **DOWDY ; WEARDEN.** Statistics for Research. John Wiley & Sons, 1983
- **BRAND RE et al.** *Gut,* 2007, vol. 56, 1460-9
- **NISSEN.** *Journal of Chromatography A,* 1995, vol. 703, 37-57
- **CHRISTIE.** *Journal of Lipid Research,* 1985, vol. 26, 507-512
- **SCHMIDT H.** *Prostaglandins & other Lipid Mediators,* 2006, vol. 81, 162-170